(19) **Europäisches Patentamt / European Patent Office / Office européen des brevets**

(11) **EP 4 621 062 A1**

(12) **EUROPEAN PATENT APPLICATION**
published in accordance with Art. 153(4) EPC

(43) Date of publication:
24.09.2025 Bulletin 2025/39

(21) Application number: 23891588.8

(22) Date of filing: 14.11.2023

(51) International Patent Classification (IPC):
$C12P\ 21/08^{(2006.01)}$    $B01D\ 61/14^{(2006.01)}$
$B01D\ 63/02^{(2006.01)}$    $B01D\ 69/00^{(2006.01)}$
$B01D\ 69/02^{(2006.01)}$    $B01D\ 71/06^{(2006.01)}$
$B01D\ 71/34^{(2006.01)}$    $B01D\ 71/68^{(2006.01)}$
$C12M\ 1/00^{(2006.01)}$    $C12M\ 1/12^{(2006.01)}$

(52) Cooperative Patent Classification (CPC):
B01D 61/14; B01D 63/02; B01D 69/00;
B01D 69/02; B01D 71/06; B01D 71/34;
B01D 71/68; C07K 16/00; C12M 1/00; C12M 1/12

(86) International application number:
PCT/JP2023/040994

(87) International publication number:
WO 2024/106441 (23.05.2024 Gazette 2024/21)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 15.11.2022 JP 2022182784

(71) Applicant: Asahi Kasei Life Science Corporation
Tokyo 100-0006 (JP)

(72) Inventors:
• MATSUDA, Shun
Tokyo 100-0006 (JP)

• SUGIYAMA, Yuya
Tokyo 100-0006 (JP)
• SAITO, Yuta
Tokyo 100-0006 (JP)
• NEMOTO, Honoka
Tokyo 100-0006 (JP)

(74) Representative: dompatent von Kreisler Selting
Werner -
Partnerschaft von Patent- und Rechtsanwälten
mbB
Deichmannhaus am Dom
Bahnhofsvorplatz 1
50667 Köln (DE)

(54) **METHOD FOR PRODUCING CELL PRODUCT, METHOD FOR PURIFYING CELL PRODUCT, METHOD FOR SUPPRESSING DECREASE IN MEMBRANE PERMEABILITY OF CELL PRODUCT, SYSTEM FOR PRODUCING CELL PRODUCT, AND SYSTEM FOR PURIFYING CELL PRODUCT**

(57) There is provided a method for producing a product by cells, the method including filtering a cell broth through a porous membrane to obtain a product by the cells, in which the porous membrane has a liquid contact surface having at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or more and 1.03 or less and a ratio of long pore diameter/short pore diameter of 1.8 or less, and a shear stress due to a flow of the cell broth on the liquid contact surface is set to 4.0 $N/m^2$ or less.

# Fig. 1

**Description**

Technical Field

The present invention relates to a filtration technique and specifically relates to a method for producing a product by cells, a method for purifying a product produced by cells, a method for suppressing a decrease in a membrane permeability of a product produced by cells, a system for producing a product by cells, and a system for purifying a product produced by cells.

Background Art

A cell culture technique is an essential technique in the production of various biopharmaceutical products such as an antibody, a growth hormone, and insulin, and it has significantly contributed to recent advancements in medicine. Among the biopharmaceutical products, in particular, an antibody pharmaceutical drug is attracting attention. Producing monoclonal antibodies efficiently and stably by culturing antibody-producing cells is one of the industrially important themes.

The industrial cell culture methods aimed at producing useful products by cells such as antibodies are broadly classified into two methods of an adherent culture method and a suspension culture method (floating culture method). In the adherent culture method, cells adhere to an inner surface of a culture vessel. In the suspension culture method, cells float in a cell broth. Among these, the suspension culture method has become mainstream due to the ease of scale-up and the ease of control in terms of large scale.

In a method for culturing cells by suspension culture, allowing cells to float by providing a stirring mechanism in a culture vessel, for example, a spinner flask, and using, as a stirring mechanism, a magnetic stirrer or a shaft-shaped impeller that is driven mechanically have been proposed. However, in the suspension culture method in the related art, cells may be cultured without the replenishment of nutrients, and growth-inhibiting substances such as lactic acid may accumulate. Therefore, the growth rate of the cells may stop in a state where the cell density is low.

On the other hand, a method has been proposed in the suspension culture method in order to culture cells in large quantities and at a high density and to continuously produce a product by cells with high efficiency, where in the method, cells are cultured while filtering out an old cell broth containing a product by cells while supplying a fresh culture medium into a culture vessel at a constant rate, thereby discharging the old cell broth out of the culture vessel at a constant rate. This method of culture is generally referred to as continuous culture or perfusion culture (see, for example, Patent Literatures 1 to 5). In continuous culture, an amount of a culture medium supplied to a culture vessel can be controlled to be equal to an amount of a cell broth discharged from the culture vessel. In continuous culture, it is important to efficiently separate cells in a cell broth from an old cell broth and a product by the cells over a long period, thereby taking out the old cell broth and the product by the cells from the culture vessel and continuously maintaining the cell growth environment within the culture vessel under optimal conditions for a long period of time.

Citation List

Patent Literatures

Patent Literature 1: Japanese Patent Application Laid-Open No. 2018-76291
Patent Literature 2: Japanese Patent Application Laid-Open No. 2009-45019
Patent Literature 3: Japanese Patent Application Laid-Open No. 2021-48776
Patent Literature 4: Japanese Patent No. 5696479
Patent Literature 5: PCT Japanese Translation Patent Publication No. 2022-516516

Summary of Invention

Technical Problem

In continuous culture, a porous membrane is used to separate cells in a cell broth of a culture vessel from an old cell broth and a product by the cells. However, fouling, which refers to the deposition or adhesion of substances on the surface and into micropores of the porous membrane, occurs as the time for filtration with the porous membrane accumulates, which causes a problem of a decrease in the permeation rate of the product by cells in the porous membrane. Therefore, an object of the present invention is to provide a method for producing a product by cells, a method for purifying a product produced by cells, a method for suppressing a decrease in a membrane permeability of a product produced by cells, a system for producing a product by cells, and a system for purifying a product produced by cells, all of which make it possible to suppress a decrease in a permeation rate of a product produced by cells in the porous membrane.

Solution to Problem

[1] A method for producing a product by cells, the method including: filtering a cell broth through a porous membrane to obtain a product by the cells, in which the porous membrane has a liquid contact surface having at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or more and 1.03 or less and a ratio of long pore diameter/short pore diameter of 1.8 or less, and a shear stress due to a flow of the cell broth on the liquid contact surface is set to 4.0 N/m$^2$ or less.

In the method for producing the product by cells, the liquid contact surface may have an opening ratio of 57.5% or less, and the liquid contact surface may also have a structural anisotropy of 0.97 or more and 1.03 or less.

In the method for producing the product by cells, the liquid contact surface may have a pore diameter having a coefficient of variation of 0.5 or less, and the liquid contact surface may also have a structural anisotropy of 0.97 or more and 1.03 or less.

In the method for producing the product by cells, the liquid contact surface may have a ratio of pore diameter/fiber diameter of 1.3 or less, and the liquid contact surface may also have a structural anisotropy of 0.97 or more and 1.03 or less.

In the method for producing the product by cells, the liquid contact surface may have an opening ratio of 57.5% or less and a pore diameter having a coefficient of variation of 0.5 or less, and the liquid contact surface may also have a structural anisotropy of 0.97 or more and 1.03 or less.

In the method for producing the product by cells, the liquid contact surface may have an opening ratio of 57.5% or less and a ratio of pore diameter/fiber diameter of 1.3 or less, and the liquid contact surface may also have a structural anisotropy of 0.97 or more and 1.03 or less.

In the method for producing the product by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or more and 1.03 or less.

In the method for producing the product by cells, the liquid contact surface may have an opening ratio of 57.5% or less, a pore diameter having a coefficient of variation of 0.5 or less and a ratio of pore diameter/fiber diameter of 1.3 or less, and the liquid contact surface may also have a structural anisotropy of 0.97 or more and 1.03 or less.

In the method for producing the product by cells, the liquid contact surface may have an opening ratio of 57.5% or less, and the liquid contact surface may also have a ratio of long pore diameter/short pore diameter of 1.8 or less.

In the method for producing the product by cells, the liquid contact surface may have a pore diameter having a coefficient of variation of 0.5 or less, and the liquid contact surface may also have a ratio of long pore diameter/short pore diameter of 1.8 or less.

In the method for producing the product by cells, the liquid contact surface may have a ratio of pore diameter/fiber diameter of 1.3 or less, and the liquid contact surface may also have a ratio of long pore diameter/short pore diameter of 1.8 or less.

In the method for producing the product by cells, the liquid contact surface may have an opening ratio of 57.5% or less and a pore diameter having a coefficient of variation of 0.5 or less, and the liquid contact surface may also have a ratio of long pore diameter/short pore diameter of 1.8 or less.

In the method for producing the product by cells, the liquid contact surface may have an opening ratio of 57.5% or less and a ratio of pore diameter/fiber diameter of 1.3 or less, and the liquid contact surface may also have a ratio of long pore diameter/short pore diameter of 1.8 or less.

In the method for producing the product by cells, the liquid contact surface may have a pore diameter having a coefficient of variation of 0.5 or less and a ratio of pore diameter/fiber diameter of 1.3 or less, and the liquid contact surface may also have a ratio of long pore diameter/short pore diameter of 1.8 or less.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a ratio of long pore diameter/short pore diameter of 1.8 or less.

In the method for producing the product by cells, the liquid contact surface may have an opening ratio of 57.5% or less, and the liquid contact surface may also have a structural anisotropy of 0.97 or more and 1.03 or less, and a ratio of long pore diameter/short pore diameter of 1.8 or less.

In the method for producing the product by cells, the liquid contact surface may have a pore diameter having a coefficient of variation of 0.5 or less, and the liquid contact surface may also have a structural anisotropy of 0.97 or more and 1.03 or less, and a ratio of long pore diameter/short pore diameter of 1.8 or less.

In the method for producing the product by cells, the liquid contact surface may have a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the method for producing the product by cells, the liquid contact surface may have an opening ratio of 57.5% or less and a pore diameter having a coefficient of variation of 0.5 or less, and the liquid contact surface may also have a structural anisotropy of 0.97 or more and 1.03 or less, and a ratio of long pore diameter/short pore diameter of 1.8 or less.

In the method for producing the product by cells, the liquid contact surface may have an opening ratio of 57.5% or less and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the method for producing the product by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

[2] The method for producing a product by cells according to [1], in which the porous membrane has at least any one selected from the group consisting of an average pore diameter of 1 $\mu$m or more on the liquid contact surface and a ratio of maximum value/minimum value of an average pore diameter in a membrane thickness direction of 3 or more.

In the method for producing the product by cells, the porous membrane may have an average pore diameter of 1 $\mu$m or more on the liquid contact surface.

In the method for producing the product by cells, the porous membrane may have a ratio of maximum value/minimum value of an average pore diameter in a membrane thickness direction of 3 or more.

In the method for producing the product by cells, the porous membrane may have an average pore diameter of 1 $\mu$m or more on the liquid contact surface, and a ratio of maximum value/minimum value of an average pore diameter in a membrane thickness direction of 3 or more.

[3] The method for producing a product by cells according to [1], in which the porous membrane has at least any one selected from the group consisting of an average pore diameter of 10 $\mu$m or less on the liquid contact surface and a ratio of maximum value/minimum value of an average pore diameter in a membrane thickness direction of 1 or more and 10 or less.

In the method for producing the product by cells, the porous membrane may have an average pore diameter of 10 $\mu$m or less on the liquid contact surface.

In the method for producing the product by cells, the porous membrane may have a ratio of maximum value/minimum value of an average pore diameter in a membrane thickness direction of 1 or more and 10 or less.

In the method for producing the product by cells, the porous membrane may have an average pore diameter of 10 $\mu$m or less on the liquid contact surface, and a ratio of maximum value/minimum value of an average pore diameter in a membrane thickness direction of 1 or more and 10 or less.

[4] The method for producing a product by cells according to [2], in which the porous membrane is a hollow fiber membrane.

[5] The method for producing a product by cells according to [4], in which the hollow fiber membrane has an inclined structure in which the average pore diameter decreases from a primary side toward a secondary side in the membrane thickness direction.

[6] The method for producing a product by cells according to [4] or [5], in which a pore of the hollow fiber membrane has a blocking pore diameter of 0.05 $\mu$m or more and 20 $\mu$m or less.

[7] The method for producing a product by cells according to any of [4] to [6], in which the hollow fiber membrane consists of a synthetic polymer membrane.

[8] The method for producing a product by cells according to [7], in which the synthetic polymer is polysulfone.

[9] The method for producing a product by cells according to any of [4] to [8], in which the hollow fiber membrane has a coarse layer and a dense layer.

[10] The method for producing a product by cells according to [2] or any of [4] to [9], in which a cell density contained in the cell broth to be filtered is $5 \times 10^7$ cells/mL or less.

[11] The method for producing a product by cells according to [3], in which the porous membrane is a hollow fiber membrane.

[12] The method for producing a product by cells according to [11], in which the hollow fiber membrane has a homogeneous structure that is substantially homogeneous from a primary side toward a secondary side in a membrane thickness direction.

[13] The method for producing a product by cells according to [11] or [12], in which a pore of the hollow fiber membrane has a blocking pore diameter of 0.05 $\mu$m or more and 10 $\mu$m or less.

[14] The method for producing a product by cells according to any of [11] to [13], in which the hollow fiber membrane consists of a synthetic polymer membrane.

[15] The method for producing a product by cells according to [14], in which the synthetic polymer is polyvinylidene fluoride.

[16] The method for producing a product by cells according to [3] or any of [11] to [15], in which a cell density contained in the cell broth to be filtered is $8 \times 10^7$ cells/mL or more.

[17] The method for producing a product by cells according to any of [1] to [16], further including: measuring the shear stress based on a viscosity of the cell broth, and controlling, in a direction parallel to a membrane surface, a flow speed of the cell broth to be filtered through the porous membrane such that the measured shear stress is 4.0 N/m$^2$ or less.

[18] The method for producing a product by cells according to any of [1] to [17], in which the cell broth that is not filtered through the hollow fiber membrane is returned to a culture vessel of the cells.

[19] The method for producing a product by cells according to any of [1] to [18], in which the product is an antibody.

[20] The method for producing a product by cells according to any of [1] to [19], in which the cells are subjected to perfusion culture.

[21] A method for purifying a product produced by cells, the method including: filtering a cell broth through a porous membrane to purify a product produced by the cells, in which the porous membrane has a liquid contact surface having at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or more and 1.03 or less and a ratio of long pore diameter/short pore diameter of 1.8 or less, and a shear stress due to a flow of the cell broth on the liquid contact surface is set to 4.0 N/m$^2$ or less.

In the method for purifying the product produced by cells, the liquid contact surface may have an opening ratio of 57.5% or less, and the liquid contact surface may also have a structural anisotropy of 0.97 or more and 1.03 or less.

In the method for purifying the product produced by cells, the liquid contact surface may have a pore diameter having a coefficient of variation of 0.5 or less, and the liquid contact surface may also have a structural anisotropy of 0.97 or more and 1.03 or less.

In the method for purifying the product produced by cells, the liquid contact surface may have a ratio of pore diameter/fiber diameter of 1.3 or less, and the liquid contact surface may also have a structural anisotropy of 0.97 or more and 1.03 or less.

In the method for purifying the product produced by cells, the liquid contact surface may have an opening ratio of 57.5% or less and a pore diameter having a coefficient of variation of 0.5 or less, and the liquid contact surface may also have a structural anisotropy of 0.97 or more and 1.03 or less.

In the method for purifying the product produced by cells, the liquid contact surface may have an opening ratio of 57.5% or less and a ratio of pore diameter/fiber diameter of 1.3 or less, and the liquid contact surface may also have a structural anisotropy of 0.97 or more and 1.03 or less.

In the method for purifying the product produced by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or more and 1.03 or less.

In the method for purifying the product produced by cells, the liquid contact surface may have an opening ratio of 57.5% or less, a pore diameter having a coefficient of variation of 0.5 or less and a ratio of pore diameter/fiber diameter of 1.3 or less, and the liquid contact surface may also have a structural anisotropy of 0.97 or more and 1.03 or less.

In the method for purifying the product produced by cells, the liquid contact surface may have an opening ratio of 57.5% or less, and the liquid contact surface may also have a ratio of long pore diameter/short pore diameter of 1.8 or less.

In the method for purifying the product produced by cells, the liquid contact surface may have a pore diameter having a coefficient of variation of 0.5 or less, and the liquid contact surface may also have a ratio of long pore diameter/short pore diameter of 1.8 or less.

In the method for purifying the product produced by cells, the liquid contact surface may have a ratio of pore diameter/fiber diameter of 1.3 or less, and the liquid contact surface may also have a ratio of long pore diameter/short pore diameter of 1.8 or less.

In the method for purifying the product produced by cells, the liquid contact surface may have an opening ratio of 57.5% or less and a pore diameter having a coefficient of variation of 0.5 or less, and the liquid contact surface may also have a ratio of long pore diameter/short pore diameter of 1.8 or less.

In the method for purifying the product produced by cells, the liquid contact surface may have an opening ratio of 57.5% or less and a ratio of pore diameter/fiber diameter of 1.3 or less, and the liquid contact surface may also have a ratio of long pore diameter/short pore diameter of 1.8 or less.

In the method for purifying the product produced by cells, the liquid contact surface may have a pore diameter having a coefficient of variation of 0.5 or less and a ratio of pore diameter/fiber diameter of 1.3 or less, and the liquid contact surface may also have a ratio of long pore diameter/short pore diameter of 1.8 or less.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a ratio of long pore diameter/short pore diameter of 1.8 or less.

In the method for purifying the product produced by cells, the liquid contact surface may have an opening ratio of 57.5% or less, and the liquid contact surface may also have a structural anisotropy of 0.97 or more and 1.03 or less, and a ratio of long pore diameter/short pore diameter of 1.8 or less.

In the method for purifying the product produced by cells, the liquid contact surface may have a pore diameter having a coefficient of variation of 0.5 or less, and the liquid contact surface may also have a structural anisotropy of 0.97 or more and 1.03 or less, and a ratio of long pore diameter/short pore diameter of 1.8 or less.

In the method for purifying the product produced by cells, the liquid contact surface may have a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the method for purifying the product produced by cells, the liquid contact surface may have an opening ratio of 57.5% or less and a pore diameter having a coefficient of variation of 0.5 or less, and the liquid contact surface may also have a structural anisotropy of 0.97 or more and 1.03 or less, and a ratio of long pore diameter/short pore diameter of 1.8 or less.

In the method for purifying the product produced by cells, the liquid contact surface may have an opening ratio of 57.5% or less and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the method for purifying the product produced by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

[22] The method for purifying a product produced by cells according to [21], in which the porous membrane has at least any one selected from the group consisting of an average pore diameter of 1 $\mu$m or more on the liquid contact surface and a ratio of maximum value/minimum value of an average pore diameter in a membrane thickness direction of 3 or more.

In the method for purifying the product produced by cells, the porous membrane may have an average pore diameter of 1 $\mu$m or more on the liquid contact surface.

In the method for purifying the product produced by cells, the porous membrane may have a ratio of maximum value/minimum value of an average pore diameter in a membrane thickness direction of 3 or more.

In the method for purifying the product produced by cells, the porous membrane may have an average pore diameter of 1 $\mu$m or more on the liquid contact surface, and a ratio of maximum value/minimum value of an average pore diameter in a membrane thickness direction of 3 or more.

[23] The method for purifying a product produced by cells according to [21], in which the porous membrane has at least any one selected from the group consisting of an average pore diameter of 10 $\mu$m or less on the liquid contact surface and a ratio of maximum value/minimum value of an average pore diameter in a membrane thickness direction of 1 or more and 10 or less.

In the method for purifying the product produced by cells, the porous membrane may have an average pore diameter of 10 $\mu$m or less on the liquid contact surface.

In the method for purifying the product produced by cells, the porous membrane may have a ratio of maximum value/minimum value of an average pore diameter in a membrane thickness direction of 1 or more and 10 or less.

In the method for purifying the product produced by cells, the porous membrane may have an average pore diameter of 10 $\mu$m or less on the liquid contact surface, and a ratio of maximum value/minimum value of an average pore diameter in a membrane thickness direction of 1 or more and 10 or less.

[24] The method for purifying a product produced by cells according to [22], in which the porous membrane is a hollow fiber membrane.

[25] The method for purifying a product produced by cells according to [24], in which the hollow fiber membrane has an inclined structure in which the average pore diameter decreases from a primary side toward a secondary side in the membrane thickness direction.

[26] The method for purifying a product produced by cells according to [24] or [25], in which a pore of the hollow fiber membrane has a blocking pore diameter of 0.05 μm or more and 20 μm or less.

[27] The method for purifying a product produced by cells according to any of [24] to [26], in which the hollow fiber membrane consists of a synthetic polymer membrane.

[28] The method for purifying a product produced by cells according to [27], in which the synthetic polymer is polysulfone.

[29] The method for purifying a product produced by cells according to any of [24] to [28], in which the hollow fiber membrane has a coarse layer and a dense layer.

[30] The method for purifying a product produced by cells according to [22] or any of [24] to [29], in which a cell density contained in the cell broth to be filtered is $5 \times 10^7$ cells/mL or less.

[31] The method for purifying a product produced by cells according to [23], in which the porous membrane is a hollow fiber membrane.

[32] The method for purifying a product produced by cells according to [31], in which the hollow fiber membrane has a homogeneous structure that is substantially homogeneous from a primary side toward a secondary side in the membrane thickness direction.

[33] The method for purifying a product produced by cells according to [31] or [32], in which a pore of the hollow fiber membrane has a blocking pore diameter of 0.05 μm or more and 10 μm or less.

[34] The method for purifying a product produced by cells according to any of [31] to [33], in which the hollow fiber membrane consists of a synthetic polymer membrane.

[35] The method for purifying a product produced by cells according to [34], in which the synthetic polymer is polyvinylidene fluoride.

[36] The method for purifying a product produced by cells according to [23] or any of [31] to [35], in which a cell density contained in the cell broth to be filtered is $8 \times 10^7$ cells/mL or more.

[37] The method for purifying a product produced by cells according to any of [21] to [36], further including: measuring the shear stress based on a viscosity of the cell broth, and controlling, in a direction parallel to a membrane surface, a flow speed of the cell broth to be filtered through the porous membrane such that the measured shear stress is 4.0 N/m$^2$ or less.

[38] The method for purifying a product produced by cells according to any of [21] to [37], in which the cell broth that is not filtered through the hollow fiber membrane is returned to a culture vessel of the cells.

[39] The method for purifying a product produced by cells according to any of [21] to [38], in which the product is an antibody.

[40] The method for purifying a product produced by cells according to any of [21] to [39], in which the cells are subjected to perfusion culture.

[41] A method for producing a product by cells, the method including: filtering a cell broth through a porous membrane to obtain a product by the cells, in which the porous membrane has a liquid contact surface having at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or less and a ratio of long pore diameter/short pore diameter of 1.8 or more, and a shear stress due to a flow of the cell broth on the liquid contact surface is set to 4.0 N/m$^2$ or more.

In the method for producing the product by cells, the liquid contact surface may have an opening ratio of 57.5% or less, and the liquid contact surface may also have a structural anisotropy of 0.97 or less.

In the method for producing the product by cells, the liquid contact surface may have a pore diameter having a coefficient of variation of 0.5 or less, and the liquid contact surface may also have a structural anisotropy of 0.97 or less.

In the method for producing the product by cells, the liquid contact surface may have a ratio of pore diameter/fiber diameter of 1.3 or less, and the liquid contact surface may also have a structural anisotropy of 0.97 or less.

In the method for producing the product by cells, the liquid contact surface may have an opening ratio of 57.5% or less and a pore diameter having a coefficient of variation of 0.5 or less, and the liquid contact surface may also have a structural anisotropy of 0.97 or less.

In the method for producing the product by cells, the liquid contact surface may have an opening ratio of 57.5% or less and a ratio of pore diameter/fiber diameter of 1.3 or less, and the liquid contact surface may also have a structural anisotropy of 0.97 or less.

In the method for producing the product by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or less.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or less.

In the method for producing the product by cells, the liquid contact surface may have an opening ratio of 57.5% or less, and the liquid contact surface may also have a ratio of long pore diameter/short pore diameter of 1.8 or more.

In the method for producing the product by cells, the liquid contact surface may have a pore diameter having a coefficient of variation of 0.5 or less, and the liquid contact surface may also have a ratio of long pore diameter/short pore diameter of 1.8 or more.

In the method for producing the product by cells, the liquid contact surface may have a ratio of pore diameter/fiber diameter of 1.3 or less, and the liquid contact surface may also have a ratio of long pore diameter/short pore diameter of 1.8 or more.

In the method for producing the product by cells, the liquid contact surface may have an opening ratio of 57.5% or less and a pore diameter having a coefficient of variation of 0.5 or less, and the liquid contact surface may also have a ratio of long pore diameter/short pore diameter of 1.8 or more.

In the method for producing the product by cells, the liquid contact surface may have an opening ratio of 57.5% or less and a ratio of pore diameter/fiber diameter of 1.3 or less, and the liquid contact surface may also have a ratio of long pore diameter/short pore diameter of 1.8 or more.

In the method for producing the product by cells, the liquid contact surface may have a pore diameter having a coefficient of variation of 0.5 or less and a ratio of pore diameter/fiber diameter of 1.3 or less, and the liquid contact surface may also have a ratio of long pore diameter/short pore diameter of 1.8 or more.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a ratio of long pore diameter/short pore diameter of 1.8 or more.

In the method for producing the product by cells, the liquid contact surface may have an opening ratio of 57.5% or less, and the liquid contact surface may also have a structural anisotropy of 0.97 or less, and a ratio of long pore diameter/short pore diameter of 1.8 or more.

In the method for producing the product by cells, the liquid contact surface may have a pore diameter having a coefficient of variation of 0.5 or less, and the liquid contact surface may also have a structural anisotropy of 0.97 or less, and a ratio of long pore diameter/short pore diameter of 1.8 or more.

In the method for producing the product by cells, the liquid contact surface may have a ratio of pore diameter/fiber diameter of 1.3 or less, and the liquid contact surface may also have a structural anisotropy of 0.97 or less, and a ratio of long pore diameter/short pore diameter of 1.8 or more.

In the method for producing the product by cells, the liquid contact surface may have an opening ratio of 57.5% or less and a pore diameter having a coefficient of variation of 0.5 or less, and the liquid contact surface may also have a structural anisotropy of 0.97 or less, and a ratio of long pore diameter/short pore diameter of 1.8 or more.

In the method for producing the product by cells, the liquid contact surface may have an opening ratio of 57.5% or less and a ratio of pore diameter/fiber diameter of 1.3 or less, and the liquid contact surface may also have a structural anisotropy of 0.97 or less, and a ratio of long pore diameter/short pore diameter of 1.8 or more.

In the method for producing the product by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or less, and a ratio of long pore diameter/short pore diameter of 1.8 or more.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or less, and a ratio of long pore diameter/short pore diameter of 1.8 or more.

[42] The method for producing a product by cells according to [41], in which the porous membrane is a hollow fiber membrane.

[43] The method for producing a product by cells according to [42], in which the hollow fiber membrane has a homogeneous structure that is substantially homogeneous from a primary side toward a secondary side in a membrane thickness direction.

[44] The method for producing a product by cells according to [42] or [43], in which a pore of the hollow fiber membrane has a blocking pore diameter of 0.05 $\mu$m or more and 10 $\mu$m or less.

[45] The method for producing a product by cells according to any of [42] to [44], in which the hollow fiber membrane consists of a synthetic polymer membrane.

[46] The method for producing a product by cells according to [45], in which the synthetic polymer is polyvinylidene fluoride.

[47] The method for producing a product by cells according to any of [41] to [46], further including: measuring the shear stress based on a viscosity of the cell broth, and controlling, in a direction parallel to a membrane surface, a flow speed of the cell broth to be filtered through the porous membrane such that the measured shear stress is 4.0 N/m$^2$ or more.

[48] The method for producing a product by cells according to any of [41] to [47], in which the cell broth that is not filtered through the hollow fiber membrane is returned to a culture vessel of the cells.

[49] The method for producing a product by cells according to any of [41] to [48], in which the product is an antibody.

[50] The method for producing a product by cells according to any of [41] to [49], in which the cells are subjected to perfusion culture.

[51] A method for purifying a product produced by cells, the method including: filtering a cell broth through a porous membrane to purify a product produced by the cells, in which the porous membrane has a liquid contact surface having at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or less and a ratio of long pore diameter/short pore diameter of 1.8 or more, and a shear stress due to a flow of the cell broth on the liquid contact surface is set to 4.0 N/m$^2$ or more.

In the method for purifying the product produced by cells, the liquid contact surface may have an opening ratio of 57.5% or less, and the liquid contact surface may also have a structural anisotropy of 0.97 or less.

In the method for purifying the product produced by cells, the liquid contact surface may have a pore diameter having a coefficient of variation of 0.5 or less, and the liquid contact surface may also have a structural anisotropy of 0.97 or less.

In the method for purifying the product produced by cells, the liquid contact surface may have a ratio of pore diameter/fiber diameter of 1.3 or less, and the liquid contact surface may also have a structural anisotropy of 0.97 or less.

In the method for purifying the product produced by cells, the liquid contact surface may have an opening ratio of 57.5% or less and a pore diameter having a coefficient of variation of 0.5 or less, and the liquid contact surface may also have a structural anisotropy of 0.97 or less.

In the method for purifying the product produced by cells, the liquid contact surface may have an opening ratio of 57.5% or less and a ratio of pore diameter/fiber diameter of 1.3 or less, and the liquid contact surface may also have a structural anisotropy of 0.97 or less.

In the method for purifying the product produced by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or less.

In the method for purifying the product produced by cells, the liquid contact surface may have an opening ratio of 57.5% or less, a pore diameter having a coefficient of variation of 0.5 or less and a ratio of pore diameter/fiber diameter of 1.3 or less, and the liquid contact surface may also have a structural anisotropy of 0.97 or less.

In the method for purifying the product produced by cells, the liquid contact surface may have an opening ratio of 57.5% or less, and the liquid contact surface may also have a ratio of long pore diameter/short pore diameter of 1.8 or more.

In the method for purifying the product produced by cells, the liquid contact surface may have a pore diameter having a coefficient of variation of 0.5 or less, and the liquid contact surface may also have a ratio of long pore diameter/short pore diameter of 1.8 or more.

In the method for purifying the product produced by cells, the liquid contact surface may have a ratio of pore diameter/fiber diameter of 1.3 or less, and the liquid contact surface may also have a ratio of long pore diameter/short pore diameter of 1.8 or more.

In the method for purifying the product produced by cells, the liquid contact surface may have an opening ratio of 57.5% or less and a pore diameter having a coefficient of variation of 0.5 or less, and the liquid contact surface may also have a ratio of long pore diameter/short pore diameter of 1.8 or more.

In the method for purifying the product produced by cells, the liquid contact surface may have an opening ratio of 57.5% or less and a ratio of pore diameter/fiber diameter of 1.3 or less, and the liquid contact surface may also have a ratio of long pore diameter/short pore diameter of 1.8 or more.

In the method for purifying the product produced by cells, the liquid contact surface may have a pore diameter having a coefficient of variation of 0.5 or less and a ratio of pore diameter/fiber diameter of 1.3 or less, and the liquid contact surface may also have a ratio of long pore diameter/short pore diameter of 1.8 or more.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a ratio of long pore diameter/short pore diameter of 1.8 or more.

In the method for purifying the product produced by cells, the liquid contact surface may have an opening ratio of 57.5% or less, and the liquid contact surface may also have a structural anisotropy of 0.97 or less, and a ratio of long pore diameter/short pore diameter of 1.8 or more.

In the method for purifying the product produced by cells, the liquid contact surface may have a pore diameter having a coefficient of variation of 0.5 or less, and the liquid contact surface may also have a structural anisotropy of 0.97 or less, and a ratio of long pore diameter/short pore diameter of 1.8 or more.

In the method for purifying the product produced by cells, the liquid contact surface may have a ratio of pore diameter/fiber diameter of 1.3 or less, and the liquid contact surface may also have a structural anisotropy of 0.97 or less, and a ratio of long pore diameter/short pore diameter of 1.8 or more.

In the method for purifying the product produced by cells, the liquid contact surface may have an opening ratio of 57.5% or less and a pore diameter having a coefficient of variation of 0.5 or less, and the liquid contact surface may also have a structural anisotropy of 0.97 or less, and a ratio of long pore diameter/short pore diameter of 1.8 or more.

In the method for purifying the product produced by cells, the liquid contact surface may have an opening ratio of 57.5% or less and a ratio of pore diameter/fiber diameter of 1.3 or less, and the liquid contact surface may also have a structural anisotropy of 0.97 or less, and a ratio of long pore diameter/short pore diameter of 1.8 or more.

In the method for purifying the product produced by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or less, and a ratio of long pore diameter/short pore diameter of 1.8 or more.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or less, and a ratio of long pore diameter/short pore diameter of 1.8 or more.

[52] The method for purifying a product produced by cells according to [51], in which the porous membrane is a hollow fiber membrane.

[53] The method for purifying a product produced by cells according to [52], in which the hollow fiber membrane has a homogeneous structure that is substantially homogeneous from a primary side toward a secondary side in a membrane thickness direction.

[54] The method for purifying a product produced by cells according to [52] or [53], in which a pore of the hollow fiber membrane has a blocking pore diameter of 0.05 $\mu$m or more and 10 $\mu$m or less.

[55] The method for purifying a product produced by cells according to any of [52] to [54], in which the hollow fiber membrane consists of a synthetic polymer membrane.

[56] The method for purifying a product produced by cells according to [55], in which the synthetic polymer is polyvinylidene fluoride.

[57] The method for purifying a product produced by cells according to any of [51] to [56], further including: measuring the shear stress based on a viscosity of the cell broth, and controlling, in a direction parallel to a membrane surface, a flow speed of the cell broth to be filtered through the porous membrane such that the measured shear stress is 4.0 N/m$^2$ or more.

[58] The method for purifying a product produced by cells according to any of [51] to [57], in which the cell broth that is not filtered through the hollow fiber membrane is returned to a culture vessel of the cells.

[59] The method for purifying a product produced by cells according to any of [51] to [58], in which the product is an antibody.

[60] The method for purifying a product produced by cells according to any of [51] to [59], in which the cells are subjected to perfusion culture.

[61] A method for suppressing a decrease in a membrane permeability of a product produced by cells, the method including: filtering a cell broth through a porous membrane to obtain a product by the cells, in which the decrease in the membrane permeability of the product by cells is suppressed by; the porous membrane having a liquid contact surface having at least any one selected from the group consisting of an opening ratio of 57.5% or less, a pore diameter having a coefficient of variation of 0.5 or less, and a ratio of pore diameter/fiber diameter of 1.3 or less, and the liquid contact surface having at least any one selected from the group consisting of a structural anisotropy of 0.97 or more and 1.03 or less and a ratio of long pore diameter/short pore diameter of 1.8 or less, and a shear stress due to a flow of the cell broth on the liquid contact surface being set to 4.0 N/m$^2$ or less.

In the method for suppressing the decrease in the membrane permeability of the product produced by cells, the liquid contact surface may have an opening ratio of 57.5% or less, and the liquid contact surface may also have a structural anisotropy of 0.97 or more and 1.03 or less.

In the method for suppressing the decrease in the membrane permeability of the product produced by cells, the liquid contact surface may have a pore diameter having a coefficient of variation of 0.5 or less, and the liquid contact surface may also have a structural anisotropy of 0.97 or more and 1.03 or less.

In the method for suppressing the decrease in the membrane permeability of the product produced by cells, the liquid contact surface may have a ratio of pore diameter/fiber diameter of 1.3 or less, and the liquid contact surface may also have a structural anisotropy of 0.97 or more and 1.03 or less.

In the method for suppressing the decrease in the membrane permeability of the product produced by cells, the liquid contact surface may have an opening ratio of 57.5% or less and a pore diameter having a coefficient of variation of 0.5 or less, and the liquid contact surface may also have a structural anisotropy of 0.97 or more and 1.03 or less.

In the method for suppressing the decrease in the membrane permeability of the product produced by cells, the liquid contact surface may have an opening ratio of 57.5% or less and a ratio of pore diameter/fiber diameter of 1.3 or less, and the liquid contact surface may also have a structural anisotropy of 0.97 or more and 1.03 or less.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or more and 1.03 or less.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or more and 1.03 or less.

In the method for suppressing the decrease in the membrane permeability of the product produced by cells, the liquid contact surface may have an opening ratio of 57.5% or less, and the liquid contact surface may also have a ratio of long pore diameter/short pore diameter of 1.8 or less.

In the method for suppressing the decrease in the membrane permeability of the product produced by cells, the liquid contact surface may have a pore diameter having a coefficient of variation of 0.5 or less, and the liquid contact surface may also have a ratio of long pore diameter/short pore diameter of 1.8 or less.

In the method for suppressing the decrease in the membrane permeability of the product produced by cells, the liquid contact surface may have a ratio of pore diameter/fiber diameter of 1.3 or less, and the liquid contact surface may also have a ratio of long pore diameter/short pore diameter of 1.8 or less.

In the method for suppressing the decrease in the membrane permeability of the product produced by cells, the liquid contact surface may have an opening ratio of 57.5% or less and a pore diameter having a coefficient of variation of 0.5 or less, and the liquid contact surface may also have a ratio of long pore diameter/short pore diameter of 1.8 or less.

In the method for suppressing the decrease in the membrane permeability of the product produced by cells, the liquid contact surface may have an opening ratio of 57.5% or less and a ratio of pore diameter/fiber diameter of 1.3 or less, and the liquid contact surface may also have a ratio of long pore diameter/short pore diameter of 1.8 or less.

In the method for suppressing the decrease in the membrane permeability of the product produced by cells, the liquid contact surface may have a pore diameter having a coefficient of variation of 0.5 or less, and a ratio of pore diameter/fiber diameter of 1.3 or less, and the liquid contact surface may also have a ratio of long pore diameter/short pore diameter of 1.8 or less.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a ratio of long pore diameter/short pore diameter of 1.8 or less.

In the method for suppressing the decrease in the membrane permeability of the product produced by cells, the liquid contact surface may have an opening ratio of 57.5% or less, and the liquid contact surface may also have a structural anisotropy of 0.97 or more and 1.03 or less, and a ratio of long pore diameter/short pore diameter of 1.8 or less.

In the method for suppressing the decrease in the membrane permeability of the product produced by cells, the liquid contact surface may have a pore diameter having a coefficient of variation of 0.5 or less, and the liquid contact surface may also have a structural anisotropy of 0.97 or more and 1.03 or less, and a ratio of long pore diameter/short pore diameter of 1.8 or less.

In the method for suppressing the decrease in the membrane permeability of the product produced by cells, the liquid contact surface may have a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the method for suppressing the decrease in the membrane permeability of the product produced by cells, the liquid contact surface may have an opening ratio of 57.5% or less and a pore diameter having a coefficient of variation of 0.5 or less, and the liquid contact surface may also have a structural anisotropy of 0.97 or more and 1.03 or less, and a ratio of long pore diameter/short pore diameter of 1.8 or less.

In the method for suppressing the decrease in the membrane permeability of the product produced by cells, the liquid contact surface may have an opening ratio of 57.5% or less and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

[62] The method for suppressing a decrease in a membrane permeability of a product produced by cells according to [61], in which the porous membrane has at least any one selected from the group consisting of an average pore diameter of 1 μm or more on the liquid contact surface and a ratio of maximum value/minimum value of an average pore diameter in a membrane thickness direction of 3 or more.

In the method for suppressing the decrease in the membrane permeability of the product produced by cells, the porous membrane may have an average pore diameter of 1 μm or more on the liquid contact surface.

In the method for suppressing the decrease in the membrane permeability of the product produced by cells, the porous membrane may have a ratio of maximum value/minimum value of an average pore diameter in a membrane thickness direction of 3 or more.

In the method for suppressing the decrease in the membrane permeability of the product produced by cells, the porous membrane may have an average pore diameter of 1 μm or more on the liquid contact surface, and a ratio of maximum value/minimum value of an average pore diameter in a membrane thickness direction of 3 or more.

[63] The method for suppressing a decrease in a membrane permeability of a product produced by cells according to [61], in which the porous membrane has at least any one selected from the group consisting of an average pore diameter of 10 μm or less on the liquid contact surface and a ratio of maximum value/minimum value of an average pore diameter in a membrane thickness direction of 1 or more and 10 or less.

In the method for suppressing the decrease in the membrane permeability of the product produced by cells, the porous membrane may have an average pore diameter of 10 μm or less on the liquid contact surface.

In the method for suppressing the decrease in the membrane permeability of the product produced by cells, the porous membrane may have a ratio of maximum value/minimum value of an average pore diameter in a membrane thickness direction of 1 or more and 10 or less.

In the method for suppressing the decrease in the membrane permeability of the product produced by cells, the porous membrane may have an average pore diameter of 10 μm or less on the liquid contact surface, and a ratio of maximum value/minimum value of an average pore diameter in a membrane thickness direction of 1 or more and 10 or less.

[64] The method for suppressing a decrease in a membrane permeability of a product produced by cells according to [62], in which the porous membrane is a hollow fiber membrane.

[65] The method for suppressing a decrease in a membrane permeability of a product produced by cells according to [64], in which the hollow fiber membrane has an inclined structure in which the average pore diameter decreases from a primary side toward a secondary side in the membrane thickness direction.

[66] The method for suppressing a decrease in a membrane permeability of a product produced by cells according to [64] or [65], in which a pore of the hollow fiber membrane has a blocking pore diameter of 0.05 μm or more and 10 μm or less.

[67] The method for suppressing a decrease in a membrane permeability of a product produced by cells according to any of [64] to [66], in which the hollow fiber membrane consists of a synthetic polymer membrane.

[68] The method for suppressing a decrease in a membrane permeability of a product produced by cells according to [67], in which the synthetic polymer is polysulfone.

[69] The method for suppressing a decrease in a membrane permeability of a product produced by cells according to any of [64] to [68], in which the hollow fiber membrane has a coarse layer and a dense layer.

[70] The method for suppressing a decrease in a membrane permeability of a product produced by cells according to [62] or any of [64] to [69], in which a cell density contained in the cell broth to be filtered is $5 \times 10^7$ cells/mL or less.

[71] The method for suppressing a decrease in a membrane permeability of a product produced by cells according to [63], in which the porous membrane is a hollow fiber membrane.

[72] The method for suppressing a decrease in a membrane permeability of a product produced by cells according to [71], in which the hollow fiber membrane has a homogeneous structure that is substantially homogeneous from a primary side toward a secondary side in the membrane thickness direction.

[73] The method for suppressing a decrease in a membrane permeability of a product produced by cells according to [71] or [72], in which a pore of the hollow fiber membrane has a blocking pore diameter of 0.05 $\mu$m or more and 10 $\mu$m or less.

[74] The method for suppressing a decrease in a membrane permeability of a product produced by cells according to any of [71] to [73], in which the hollow fiber membrane consists of a synthetic polymer membrane.

[75] The method for suppressing a decrease in a membrane permeability of a product produced by cells according to [74], in which the synthetic polymer is polyvinylidene fluoride.

[76] The method for suppressing a decrease in a membrane permeability of a product produced by cells according to [63] or any of [71] to [75], in which a cell density contained in the cell broth to be filtered is $8 \times 10^7$ cells/mL or more.

[77] The method for suppressing a decrease in a membrane permeability of a product produced by cells according to any of [61] to [76], further including: measuring the shear stress based on a viscosity of the cell broth, and controlling, in a direction parallel to a membrane surface, a flow speed of the cell broth to be filtered through the porous membrane such that the measured shear stress is 4.0 N/m$^2$ or less.

[78] The method for suppressing a decrease in a membrane permeability of a product produced by cells according to any of [61] to [77], in which the cell broth that is not filtered through the hollow fiber membrane is returned to a culture vessel of the cells.

[79] The method for suppressing a decrease in a membrane permeability of a product produced by cells according to any of [61] to [78], in which the product is an antibody.

[80] The method for suppressing a decrease in a membrane permeability of a product produced by cells according to any of [61] to [79], in which the cells are subjected to perfusion culture.

[81] A method for suppressing a decrease in a membrane permeability of a product produced by cells, the method including: filtering a cell broth through a porous membrane to obtain a product by the cells, in which the decrease in the membrane permeability of the product produced by cells is suppressed by: the porous membrane having a liquid contact surface having at least any one selected from the group consisting of an opening ratio of 57.5% or less, a pore diameter having a coefficient of variation of 0.5 or less, and a ratio of pore diameter/fiber diameter of 1.3 or less, and the liquid contact surface having at least any one selected from the group consisting of a structural anisotropy of 0.97 or less and a ratio of long pore diameter/short pore diameter of 1.8 or more, and a shear stress due to a flow of the cell broth on the liquid contact surface being set to 4.0 N/m$^2$ or more.

In the method for suppressing the decrease in the membrane permeability of the product produced by cells, the liquid contact surface may have an opening ratio of 57.5% or less, and the liquid contact surface may also have a structural anisotropy of 0.97 or less.

In the method for suppressing the decrease in the membrane permeability of the product produced by cells, the liquid contact surface may have a pore diameter having a coefficient of variation of 0.5 or less, and the liquid contact surface may also have a structural anisotropy of 0.97 or less.

In the method for suppressing the decrease in the membrane permeability of the product produced by cells, the liquid contact surface may have a ratio of pore diameter/fiber diameter of 1.3 or less, and the liquid contact surface may also have a structural anisotropy of 0.97 or less.

In the method for suppressing the decrease in the membrane permeability of the product produced by cells, the liquid contact surface may have an opening ratio of 57.5% or less and a pore diameter having a coefficient of variation of 0.5 or less, and the liquid contact surface may also have a structural anisotropy of 0.97 or less.

In the method for suppressing the decrease in the membrane permeability of the product produced by cells, the liquid contact surface may have an opening ratio of 57.5% or less and a ratio of pore diameter/fiber diameter of 1.3 or less, and the liquid contact surface may also have a structural anisotropy of 0.97 or less.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or less.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or less.

In the method for suppressing the decrease in the membrane permeability of the product produced by cells, the liquid contact surface may have an opening ratio of 57.5% or less, and the liquid contact surface may also have a ratio of long pore diameter/short pore diameter of 1.8 or more.

In the method for suppressing the decrease in the membrane permeability of the product produced by cells, the liquid contact surface may have a pore diameter having a coefficient of variation of 0.5 or less, and the liquid contact surface may also have a ratio of long pore diameter/short pore diameter of 1.8 or more.

In the method for suppressing the decrease in the membrane permeability of the product produced by cells, the liquid contact surface may have a ratio of pore diameter/fiber diameter of 1.3 or less, and the liquid contact surface may also have a ratio of long pore diameter/short pore diameter of 1.8 or more.

In the method for suppressing the decrease in the membrane permeability of the product produced by cells, the liquid contact surface may have an opening ratio of 57.5% or less and a pore diameter having a coefficient of variation of 0.5 or less, and the liquid contact surface may also have a ratio of long pore diameter/short pore diameter of 1.8 or more.

In the method for suppressing the decrease in the membrane permeability of the product produced by cells, the liquid contact surface may have an opening ratio of 57.5% or less and a ratio of pore diameter/fiber diameter of 1.3 or less, and the liquid contact surface may also have a ratio of long pore diameter/short pore diameter of 1.8 or more.

In the method for suppressing the decrease in the membrane permeability of the product produced by cells, the liquid contact surface may have a pore diameter having a coefficient of variation of 0.5 or less, and a ratio of pore diameter/fiber diameter of 1.3 or less, and the liquid contact surface may also have a ratio of long pore diameter/short pore diameter of 1.8 or more.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a ratio of long pore diameter/short pore diameter of 1.8 or more.

In the method for suppressing the decrease in the membrane permeability of the product produced by cells, the liquid contact surface may have an opening ratio of 57.5% or less, and the liquid contact surface may also have a structural anisotropy of 0.97 or less, and a ratio of long pore diameter/short pore diameter of 1.8 or more.

In the method for suppressing the decrease in the membrane permeability of the product produced by cells, the liquid contact surface may have a pore diameter having a coefficient of variation of 0.5 or less, and the liquid contact surface may also have a structural anisotropy of 0.97 or less, and a ratio of long pore diameter/short pore diameter of 1.8 or more.

In the method for suppressing the decrease in the membrane permeability of the product produced by cells, the liquid contact surface may have a ratio of pore diameter/fiber diameter of 1.3 or less, and the liquid contact surface may also have a structural anisotropy of 0.97 or less, and a ratio of long pore diameter/short pore diameter of 1.8 or more.

In the method for suppressing the decrease in the membrane permeability of the product produced by cells, the liquid contact surface may have an opening ratio of 57.5% or less and a pore diameter having a coefficient of variation of 0.5 or less, and the liquid contact surface may also have a structural anisotropy of 0.97 or less, and a ratio of long pore diameter/short pore diameter of 1.8 or more.

In the method for suppressing the decrease in the membrane permeability of the product produced by cells, the liquid contact surface may have an opening ratio of 57.5% or less and a ratio of pore diameter/fiber diameter of 1.3 or less, and the liquid contact surface may also have a structural anisotropy of 0.97 or less, and a ratio of long pore diameter/short pore diameter of 1.8 or more.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or less, and a ratio of long pore diameter/short pore diameter of 1.8 or more.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or less, and a ratio of long pore diameter/short pore diameter of 1.8 or more.

[82] The method for suppressing a decrease in a membrane permeability of a product produced by cells according to [81], in which the porous membrane is a hollow fiber membrane.

[83] The method for suppressing a decrease in a membrane permeability of a product produced by cells according to [82], in which the hollow fiber membrane has a homogeneous structure that is substantially homogeneous from a primary side toward a secondary side in the membrane thickness direction.

[84] The method for suppressing a decrease in a membrane permeability of a product produced by cells according to [82] or [83], in which a pore of the hollow fiber membrane has a blocking pore diameter of 0.05 $\mu$m or more and 10 $\mu$m or less.

[85] The method for suppressing a decrease in a membrane permeability of a product produced by cells according to any of [82] to [84], in which the hollow fiber membrane consists of a synthetic polymer membrane.

[86] The method for suppressing a decrease in a membrane permeability of a product produced by cells according to [85], in which the synthetic polymer is polyvinylidene fluoride.

[87] The method for suppressing a decrease in a membrane permeability of a product produced by cells according to any of [81] to [86], further including: measuring the shear stress based on a viscosity of the cell broth, and controlling, in a direction parallel to a membrane surface, a flow speed of the cell broth to be filtered through the porous membrane such that the measured shear stress is 4.0 N/m² or more.

[88] The method for suppressing a decrease in a membrane permeability of a product produced by cells according to any of [81] to [87], in which the cell broth that is not filtered through the hollow fiber membrane is returned to a culture vessel of the cells.

[89] The method for suppressing a decrease in a membrane permeability of a product produced by cells according to any of [81] to [88], in which the product is an antibody.

[90] The method for suppressing a decrease in a membrane permeability of a product produced by cells according to any of [81] to [89], in which the cells are subjected to perfusion culture.

[91] A system for producing a product by cells, the system including: a culture vessel for culturing cells; a porous membrane for filtering a cell broth to obtain a product by the cells; a pump for feeding the cell broth from the culture vessel to the porous membrane; and a controller configured to control the pump such that a shear stress due to a flow of the cell broth on a liquid contact surface of the porous membrane is set to 4.0 N/m² or less, in which the liquid contact surface has at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or more and 1.03 or less and a ratio of long pore diameter/short pore diameter of 1.8 or less.

In the system for producing the product by cells, the liquid contact surface may have an opening ratio of 57.5% or less, and the liquid contact surface may also have a structural anisotropy of 0.97 or more and 1.03 or less.

In the system for producing the product by cells, the liquid contact surface may have a pore diameter having a coefficient of variation of 0.5 or less, and the liquid contact surface may also have a structural anisotropy of 0.97 or more and 1.03 or less.

In the system for producing the product by cells, the liquid contact surface may have a ratio of pore diameter/fiber diameter of 1.3 or less, and the liquid contact surface may also have a structural anisotropy of 0.97 or more and 1.03 or less.

In the system for producing the product by cells, the liquid contact surface may have an opening ratio of 57.5% or less and a pore diameter having a coefficient of variation of 0.5 or less, and the liquid contact surface may also have a structural anisotropy of 0.97 or more and 1.03 or less.

In the system for producing the product by cells, the liquid contact surface may have an opening ratio of 57.5% or less and a ratio of pore diameter/fiber diameter of 1.3 or less, and the liquid contact surface may also have a structural anisotropy of 0.97 or more and 1.03 or less.

In the system for producing the product by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or more and 1.03 or less.

In the system for producing the product by cells, the liquid contact surface may have an opening ratio of 57.5% or less, a pore diameter having a coefficient of variation of 0.5 or less and a ratio of pore diameter/fiber diameter of 1.3 or less, and the liquid contact surface may also have a structural anisotropy of 0.97 or more and 1.03 or less.

In the system for producing the product by cells, the liquid contact surface may have an opening ratio of 57.5% or less, and the liquid contact surface may also have a ratio of long pore diameter/short pore diameter of 1.8 or less.

In the system for producing the product by cells, the liquid contact surface may have a pore diameter having a coefficient of variation of 0.5 or less, and the liquid contact surface may also have a ratio of long pore diameter/short pore diameter of 1.8 or less.

In the system for producing the product by cells, the liquid contact surface may have a ratio of pore diameter/fiber diameter of 1.3 or less, and the liquid contact surface may also have a ratio of long pore diameter/short pore diameter of 1.8 or less.

In the system for producing the product by cells, the liquid contact surface may have an opening ratio of 57.5% or less and a pore diameter having a coefficient of variation of 0.5 or less, and the liquid contact surface may also have a ratio of long pore diameter/short pore diameter of 1.8 or less.

In the system for producing the product by cells, the liquid contact surface may have an opening ratio of 57.5% or less and a ratio of pore diameter/fiber diameter of 1.3 or less, and the liquid contact surface may also have a ratio of long pore diameter/short pore diameter of 1.8 or less.

In the system for producing the product by cells, the liquid contact surface may have a pore diameter having a coefficient of variation of 0.5 or less and a ratio of pore diameter/fiber diameter of 1.3 or less, and the liquid contact surface may also have a ratio of long pore diameter/short pore diameter of 1.8 or less.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a ratio of long pore diameter/short pore diameter of 1.8 or less.

In the system for producing the product by cells, the liquid contact surface may have an opening ratio of 57.5% or less, and the liquid contact surface may also have a structural anisotropy of 0.97 or more and 1.03 or less, and a ratio of long pore diameter/short pore diameter of 1.8 or less.

In the system for producing the product by cells, the liquid contact surface may have a pore diameter having a coefficient of variation of 0.5 or less, and the liquid contact surface may also have a structural anisotropy of 0.97 or more and 1.03 or less, and a ratio of long pore diameter/short pore diameter of 1.8 or less.

In the system for producing the product by cells, the liquid contact surface may have a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the system for producing the product by cells, the liquid contact surface may have an opening ratio of 57.5% or less and a pore diameter having a coefficient of variation of 0.5 or less, and the liquid contact surface may also have a structural anisotropy of 0.97 or more and 1.03 or less, and a ratio of long pore diameter/short pore diameter of 1.8 or less.

In the system for producing the product by cells, the liquid contact surface may have an opening ratio of 57.5% or less and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the system for producing the product by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the system for producing the product by cells, the liquid contact surface may have an opening ratio of 57.5% or less, a pore diameter having a coefficient of variation of 0.5 or less and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

[92] The system for producing a product by cells according to [91], in which the porous membrane has at least any one selected from the group consisting of an average pore diameter of 1 $\mu$m or more on the liquid contact surface and a ratio of maximum value/minimum value of an average pore diameter in a membrane thickness direction of 3 or more.

In the system for producing the product by cells, the porous membrane may have an average pore diameter of 1 $\mu$m or more on the liquid contact surface.

In the system for producing the product by cells, the porous membrane may have a ratio of maximum value/minimum value of an average pore diameter in a membrane thickness direction of 3 or more.

In the system for producing the product by cells, the porous membrane may have an average pore diameter of 1 $\mu$m or more on the liquid contact surface, and a ratio of maximum value/minimum value of an average pore diameter in a membrane thickness direction of 3 or more.

[93] The system for producing a product by cells according to [91], in which the porous membrane has at least any one selected from the group consisting of an average pore diameter of 10 $\mu$m or less on the liquid contact surface and a ratio of maximum value/minimum value of an average pore diameter in a membrane thickness direction of 1 or more and 10 or less.

In the system for producing the product by cells, the porous membrane may have an average pore diameter of 10 $\mu$m or less on the liquid contact surface.

In the system for producing the product by cells, the porous membrane may have a ratio of maximum value/minimum value of an average pore diameter in a membrane thickness direction of 1 or more and 10 or less.

In the system for producing the product by cells, the porous membrane may have an average pore diameter of 10 $\mu$m or less on the liquid contact surface, and a ratio of maximum value/minimum value of an average pore diameter in a membrane thickness direction of 1 or more and 10 or less.

[94] The system for producing a product by cells according to [92], in which the porous membrane is a hollow fiber membrane.

[95] The system for producing a product by cells according to [94], in which the hollow fiber membrane has an inclined structure in which the average pore diameter decreases from a primary side toward a secondary side in the membrane thickness direction.

[96] The system for producing a product by cells according to [94] or [95], in which a pore of the hollow fiber membrane has a blocking pore diameter of 0.05 $\mu$m or more and 20 $\mu$m or less.

[97] The system for producing a product by cells according to any of [94] to [96], in which the hollow fiber membrane consists of a synthetic polymer membrane.

[98] The system for producing a product by cells according to [97], in which the synthetic polymer is polysulfone.

[99] The system for producing a product by cells according to any of [94] to [98], in which the hollow fiber membrane has a coarse layer and a dense layer.

[100] The system for producing a product by cells according to [92] or any of [94] to [99], in which a cell density contained in the cell broth to be filtered is $5 \times 10^7$ cells/mL or less.

[101] The system for producing a product by cells according to [93], in which the porous membrane is a hollow fiber membrane.

[102] The system for producing a product by cells according to [101], in which the hollow fiber membrane has a homogeneous structure that is substantially homogeneous from a primary side toward a secondary side in the membrane thickness direction.

[103] The system for producing a product by cells according to [101] or [102], in which a pore of the hollow fiber membrane has a blocking pore diameter of 0.05 μm or more and 10 μm or less.

[104] The system for producing a product by cells according to any of [101] to [103], in which the hollow fiber membrane consists of a synthetic polymer membrane.

[105] The system for producing a product by cells according to [104], in which the synthetic polymer is polyvinylidene fluoride.

[106] The system for producing a product by cells according to [93] or any of [101] to [105], in which a cell density contained in the cell broth to be filtered is $8 \times 10^7$ cells/mL or more.

[107] The system for producing a product by cells according to any of [91] to [106], in which the controller measures the shear stress based on a viscosity of the cell broth, and controls, in a direction parallel to a membrane surface, a flow speed of the cell broth to be filtered through the porous membrane such that the measured shear stress is 4.0 N/m$^2$ or less.

[108] The system for producing a product by cells according to any of [91] to [107], in which the controller receives an input of data of a viscosity of the cell broth.

[109] The system for producing a product by cells according to any of [91] to [108], further including: a flow channel for returning the cell broth that is not filtered through the hollow fiber membrane, to the culture vessel.

[110] The system for producing a product by cells according to any of [91] to [109], in which the product is an antibody.

[111] The system for producing a product by cells according to any of [91] to [110], in which the cells are subjected to perfusion culture in the culture vessel.

[112] A system for producing a product by cells, the system including: a culture vessel for culturing cells; a porous membrane for filtering a cell broth to obtain a product by the cells; a pump for feeding the cell broth from the culture vessel to the porous membrane; and a controller configured to control the pump such that a shear stress due to a flow of the cell broth on a liquid contact surface of the porous membrane is set to 4.0 N/m$^2$ or more, in which the liquid contact surface has at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or less and a ratio of long pore diameter/short pore diameter of 1.8 or more.

In the system for producing the product by cells, the liquid contact surface may have an opening ratio of 57.5% or less, and the liquid contact surface may also have a structural anisotropy of 0.97 or less.

In the system for producing the product by cells, the liquid contact surface may have a pore diameter having a coefficient of variation of 0.5 or less, and the liquid contact surface may also have a structural anisotropy of 0.97 or less.

In the system for producing the product by cells, the liquid contact surface may have a ratio of pore diameter/fiber diameter of 1.3 or less, and the liquid contact surface may also have a structural anisotropy of 0.97 or less.

In the system for producing the product by cells, the liquid contact surface may have an opening ratio of 57.5% or less, a pore diameter having a coefficient of variation of 0.5 or less, and the liquid contact surface may also have a structural anisotropy of 0.97 or less.

In the system for producing the product by cells, the liquid contact surface may have an opening ratio of 57.5% or less and a ratio of pore diameter/fiber diameter of 1.3 or less, and the liquid contact surface may also have a structural anisotropy of 0.97 or less.

In the system for producing the product by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or less.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or less.

In the system for producing the product by cells, the liquid contact surface may have an opening ratio of 57.5% or less, and the liquid contact surface may also have a ratio of long pore diameter/short pore diameter of 1.8 or more.

In the system for producing the product by cells, the liquid contact surface may have a pore diameter having a coefficient of variation of 0.5 or less, and the liquid contact surface may also have a ratio of long pore diameter/short pore diameter of 1.8 or more.

In the system for producing the product by cells, the liquid contact surface may have a ratio of pore diameter/fiber diameter of 1.3 or less, and the liquid contact surface may also have a ratio of long pore diameter/short pore diameter of 1.8 or more.

In the system for producing the product by cells, the liquid contact surface may have an opening ratio of 57.5% or less and a pore diameter having a coefficient of variation of 0.5 or less, and the liquid contact surface may also have a ratio of long pore diameter/short pore diameter of 1.8 or more.

In the system for producing the product by cells, the liquid contact surface may have an opening ratio of 57.5% or less and a ratio of pore diameter/fiber diameter of 1.3 or less, and the liquid contact surface may also have a ratio of long pore diameter/short pore diameter of 1.8 or more.

In the system for producing the product by cells, the liquid contact surface may have a pore diameter having a coefficient of variation of 0.5 or less and a ratio of pore diameter/fiber diameter of 1.3 or less, and the liquid contact surface may also have a ratio of long pore diameter/short pore diameter of 1.8 or more.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a ratio of long pore diameter/short pore diameter of 1.8 or more.

In the system for producing the product by cells, the liquid contact surface may have an opening ratio of 57.5% or less, and the liquid contact surface may also have a structural anisotropy of 0.97 or less, and a ratio of long pore diameter/short pore diameter of 1.8 or more.

In the system for producing the product by cells, the liquid contact surface may have a pore diameter having a coefficient of variation of 0.5 or less, and the liquid contact surface may also have a structural anisotropy of 0.97 or less, and a ratio of long pore diameter/short pore diameter of 1.8 or more.

In the system for producing the product by cells, the liquid contact surface may have a ratio of pore diameter/fiber diameter of 1.3 or less, and the liquid contact surface may also have a structural anisotropy of 0.97 or less, and a ratio of long pore diameter/short pore diameter of 1.8 or more.

In the system for producing the product by cells, the liquid contact surface may have an opening ratio of 57.5% or less and a coefficient of variation of pore diameter of 0.5 or less, and the liquid contact surface may also have a structural anisotropy of 0.97 or less, and a ratio of long pore diameter/short pore diameter of 1.8 or more.

In the system for producing the product by cells, the liquid contact surface may have an opening ratio of 57.5% or less and a ratio of pore diameter/fiber diameter of 1.3 or less, and the liquid contact surface may also have a structural anisotropy of 0.97 or less, and a ratio of long pore diameter/short pore diameter of 1.8 or more.

In the system for producing the product by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or less, and a ratio of long pore diameter/short pore diameter of 1.8 or more.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or less, and a ratio of long pore diameter/short pore diameter of 1.8 or more.

[113] The system for producing a product by cells according to [112], in which the porous membrane is a hollow fiber membrane.

[114] The system for producing a product by cells according to [113], in which the hollow fiber membrane has a homogeneous structure that is substantially homogeneous from a primary side toward a secondary side in the membrane thickness direction.

[115] The system for producing a product by cells according to [113] or [114], in which a pore of the hollow fiber membrane has a blocking pore diameter of 0.05 $\mu$m or more and 10 $\mu$m or less.

[116] The system for producing a product by cells according to any of [113] to [115], in which the hollow fiber membrane consists of a synthetic polymer membrane.

[117] The system for producing a product by cells according to [116], in which the synthetic polymer is polyvinylidene fluoride.

[118] The system for producing a product by cells according to any of [112] to [117], in which the controller measures the shear stress based on a viscosity of the cell broth, and controls, in a direction parallel to a membrane surface, a flow speed of the cell broth to be filtered through the porous membrane such that the measured shear stress is 4.0 N/m$^2$ or more.

[119] The system for producing a product by cells according to any of [112] to [118], in which the controller receives an input of data of a viscosity of the cell broth.

[120] The system for producing a product by cells according to any of [112] to [119], further including: a flow channel for returning the cell broth that is not filtered through the hollow fiber membrane, to the culture vessel.

[121] The system for producing a product by cells according to any of [112] to [120], in which the product is an antibody.

[122] The system for producing a product by cells according to any of [112] to [121], in which the cells are subjected to perfusion culture in the culture vessel.

[123] A system for purifying a product produced by cells, the system including: a culture vessel for culturing cells; a porous membrane for filtering a cell broth of the cells to purify a product produced by the cells; a pump for feeding the cell broth from the culture vessel to the porous membrane; and a controller configured to control the pump such that a shear stress due to a flow of the cell broth on a liquid contact surface of the porous membrane is set to 4.0 N/m$^2$ or less, in which the liquid contact surface has at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or more and 1.03 or less and a ratio of long pore diameter/short pore diameter of 1.8 or less.

In the system for purifying the product produced by cells, the liquid contact surface may have an opening ratio of 57.5% or less, and the liquid contact surface may also have a structural anisotropy of 0.97 or more and 1.03 or less.

In the system for purifying the product produced by cells, the liquid contact surface may have a pore diameter having a coefficient of variation of 0.5 or less, and the liquid contact surface may also have a structural anisotropy of 0.97 or more and 1.03 or less.

In the system for purifying the product produced by cells, the liquid contact surface may have a ratio of pore diameter/fiber diameter of 1.3 or less, and the liquid contact surface may also have a structural anisotropy of 0.97 or more and 1.03 or less.

In the system for purifying the product produced by cells, the liquid contact surface may have an opening ratio of 57.5% or less and a pore diameter having a coefficient of variation of 0.5 or less, and the liquid contact surface may also have a structural anisotropy of 0.97 or more and 1.03 or less.

In the system for purifying the product produced by cells, the liquid contact surface may have an opening ratio of 57.5% or less and a ratio of pore diameter/fiber diameter of 1.3 or less, and the liquid contact surface may also have a structural anisotropy of 0.97 or more and 1.03 or less.

In the system for purifying the product produced by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or more and 1.03 or less.

In the system for purifying the product produced by cells, the liquid contact surface may have an opening ratio of 57.5% or less, a pore diameter having a coefficient of variation of 0.5 or less and a ratio of pore diameter/fiber diameter of 1.3 or less, and the liquid contact surface may also have a structural anisotropy of 0.97 or more and 1.03 or less.

In the system for purifying the product produced by cells, the liquid contact surface may have an opening ratio of 57.5% or less, and the liquid contact surface may also have a ratio of long pore diameter/short pore diameter of 1.8 or less.

In the system for purifying the product produced by cells, the liquid contact surface may have a pore diameter having a coefficient of variation of 0.5 or less, and the liquid contact surface may also have a ratio of long pore diameter/short pore diameter of 1.8 or less.

In the system for purifying the product produced by cells, the liquid contact surface may have a ratio of pore diameter/fiber diameter of 1.3 or less, and the liquid contact surface may also have a ratio of long pore diameter/short pore diameter of 1.8 or less.

In the system for purifying the product produced by cells, the liquid contact surface may have an opening ratio of 57.5% or less and a pore diameter having a coefficient of variation of 0.5 or less, and the liquid contact surface may also have a ratio of long pore diameter/short pore diameter of 1.8 or less.

In the system for purifying the product produced by cells, the liquid contact surface may have an opening ratio of 57.5% or less and a ratio of pore diameter/fiber diameter of 1.3 or less, and the liquid contact surface may also have a ratio of long pore diameter/short pore diameter of 1.8 or less.

In the system for purifying the product produced by cells, the liquid contact surface may have a pore diameter having a coefficient of variation of 0.5 or less and a ratio of pore diameter/fiber diameter of 1.3 or less, and the liquid contact surface may also have a ratio of long pore diameter/short pore diameter of 1.8 or less.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a ratio of long pore diameter/short pore diameter of 1.8 or less.

In the system for purifying the product produced by cells, the liquid contact surface may have an opening ratio of 57.5% or less, and the liquid contact surface may also have a structural anisotropy of 0.97 or more and 1.03 or less, and a ratio of long pore diameter/short pore diameter of 1.8 or less.

In the system for purifying the product produced by cells, the liquid contact surface may have a pore diameter having a coefficient of variation of 0.5 or less, and the liquid contact surface may also have a structural anisotropy of 0.97 or more and 1.03 or less, and a ratio of long pore diameter/short pore diameter of 1.8 or less.

In the system for purifying the product produced by cells, the liquid contact surface may have a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the system for purifying the product produced by cells, the liquid contact surface may have an opening ratio of 57.5% or less and a pore diameter having a coefficient of variation of 0.5 or less, and the liquid contact surface may also have a structural anisotropy of 0.97 or more and 1.03 or less, and a ratio of long pore diameter/short pore diameter of 1.8 or less.

In the system for purifying the product produced by cells, the liquid contact surface may have an opening ratio of 57.5% or less and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the system for purifying the product produced by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or more and 1.03 or less, and a ratio of long pore diameter/short pore diameter of 1.8 or less.

[124] The system for purifying a product produced by cells according to [123], in which the porous membrane has at least any one selected from the group consisting of an average pore diameter of 1 $\mu$m or more on the liquid contact surface and a ratio of maximum value/minimum value of an average pore diameter in a membrane thickness direction of 3 or more.

In the system for purifying the product produced by cells, the porous membrane may have an average pore diameter of 1 $\mu$m or more on the liquid contact surface.

In the system for purifying the product produced by cells, the porous membrane may have a ratio of maximum value/minimum value of an average pore diameter in a membrane thickness direction of 3 or more.

In the system for purifying the product produced by cells, the porous membrane may have an average pore diameter of 1 $\mu$m or more on the liquid contact surface, and a ratio of maximum value/minimum value of an average pore diameter in a membrane thickness direction of 3 or more.

[125] The system for purifying a product produced by cells according to [123], in which the porous membrane has at least any one selected from the group consisting of an average pore diameter of 10 $\mu$m or less on the liquid contact surface and a ratio of maximum value/minimum value of an average pore diameter in a membrane thickness direction of 1 or more and 10 or less.

In the system for purifying the product produced by cells, the porous membrane may have an average pore diameter of 10 $\mu$m or less on the liquid contact surface.

In the system for purifying the product produced by cells, the porous membrane may have a ratio of maximum value/minimum value of an average pore diameter in a membrane thickness direction of 1 or more and 10 or less.

In the system for purifying the product produced by cells, the porous membrane may have an average pore diameter of 10 $\mu$m or less on the liquid contact surface, and a ratio of maximum value/minimum value of an average pore diameter in a membrane thickness direction of 1 or more and 10 or less.

[126] The system for purifying a product produced by cells according to [124], in which the porous membrane is a hollow fiber membrane.

[127] The system for purifying a product produced by cells according to [126], in which the hollow fiber membrane has an inclined structure in which the average pore diameter decreases from a primary side toward a secondary side in the membrane thickness direction.

[128] The system for purifying a product produced by cells according to [126] or [127], in which a pore of the hollow fiber membrane has a blocking pore diameter of 0.05 $\mu$m or more and 20 $\mu$m or less.

[129] The system for purifying a product produced by cells according to any of [126] to [128], in which the hollow fiber membrane consists of a synthetic polymer membrane.

[130] The system for purifying a product produced by cells according to [129], in which the synthetic polymer is polysulfone.

[131] The system for purifying a product produced by cells according to any of [126] to [130], in which the hollow fiber membrane has a coarse layer and a dense layer.

[132] The system for purifying a product produced by cells according to [124] or any of [126] to [131], in which a cell density contained in the cell broth to be filtered is $5 \times 10^7$ cells/mL or less.

[133] The system for purifying a product produced by cells according to [125], in which the porous membrane is a hollow fiber membrane.

[134] The system for purifying a product produced by cells according to [133], in which the hollow fiber membrane has a homogeneous structure that is substantially homogeneous from a primary side toward a secondary side in the membrane thickness direction.

[135] The system for purifying a product produced by cells according to [133] or [134], in which a pore of the hollow fiber membrane has a blocking pore diameter of 0.05 $\mu$m or more and 10 $\mu$m or less.

[136] The system for purifying a product produced by cells according to any of [133] to [135], in which the hollow fiber membrane consists of a synthetic polymer membrane.

[137] The system for purifying a product produced by cells according to [136], in which the synthetic polymer is polyvinylidene fluoride.

[138] The system for purifying a product produced by cells according to [125] or any of [133] to [137], in which a cell density contained in the cell broth to be filtered is $8 \times 10^7$ cells/mL or more.

[139] The system for purifying a product produced by cells according to any of [123] to [138], in which the controller measures the shear stress based on a viscosity of the cell broth, and controls, in a direction parallel to a membrane surface, a flow speed of the cell broth to be filtered through the porous membrane such that the measured shear stress is 4.0 N/m$^2$ or less.

[140] The system for purifying a product produced by cells according to any of [123] to [139], in which the controller receives an input of data of a viscosity of the cell broth.

[141] The system for purifying a product produced by cells according to any of [123] to [140], further including: a flow channel for returning the cell broth that is not filtered through the hollow fiber membrane, to the culture vessel.

[142] The system for purifying a product produced by cells according to any of [123] to [141], in which the product is an antibody.

[143] The system for purifying a product produced by cells according to any of [123] to [142], in which the cells are subjected to perfusion culture in the culture vessel.

[144] A system for purifying a product produced by cells, the system including: a culture vessel for culturing cells; a porous membrane for filtering a cell broth to purify a product produced by the cells; a pump for feeding the cell broth from the culture vessel to the porous membrane; and a controller configured to control the pump such that a shear stress due to a flow of the cell broth on a liquid contact surface of the porous membrane is set to 4.0 N/m$^2$ or more, in which the liquid contact surface has at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or less and a ratio of long pore diameter/short pore diameter of 1.8 or more.

In the system for purifying the product produced by cells, the liquid contact surface may have an opening ratio of 57.5% or less, and the liquid contact surface may also have a structural anisotropy of 0.97 or less.

In the system for purifying the product produced by cells, the liquid contact surface may have a pore diameter having a coefficient of variation of 0.5 or less, and the liquid contact surface may also have a structural anisotropy of 0.97 or less.

In the system for purifying the product produced by cells, the liquid contact surface may have a ratio of pore diameter/fiber diameter of 1.3 or less, and the liquid contact surface may also have a structural anisotropy of 0.97 or less.

In the system for purifying the product produced by cells, the liquid contact surface may have an opening ratio of 57.5% or less and a pore diameter having a coefficient of variation of 0.5 or less, and the liquid contact surface may also have a structural anisotropy of 0.97 or less.

In the system for purifying the product produced by cells, the liquid contact surface may have an opening ratio of 57.5% or less and a ratio of pore diameter/fiber diameter of 1.3 or less, and the liquid contact surface may also have a structural anisotropy of 0.97 or less.

In the system for purifying the product produced by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or less.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or less.

In the system for purifying the product produced by cells, the liquid contact surface may have an opening ratio of 57.5% or less, and the liquid contact surface may also have a ratio of long pore diameter/short pore diameter of 1.8 or more.

In the system for purifying the product produced by cells, the liquid contact surface may have a pore diameter having a coefficient of variation of 0.5 or less, and the liquid contact surface may also have a ratio of long pore diameter/short pore diameter of 1.8 or more.

In the system for purifying the product produced by cells, the liquid contact surface may have a ratio of pore diameter/fiber diameter of 1.3 or less, and the liquid contact surface may also have a ratio of long pore diameter/short pore diameter of 1.8 or more.

In the system for purifying the product produced by cells, the liquid contact surface may have an opening ratio of 57.5% or less and a pore diameter having a coefficient of variation of 0.5 or less, and the liquid contact surface may also have a ratio of long pore diameter/short pore diameter of 1.8 or more.

In the system for purifying the product produced by cells, the liquid contact surface may have an opening ratio of 57.5% or less and a ratio of pore diameter/fiber diameter of 1.3 or less, and the liquid contact surface may also have a ratio of long pore diameter/short pore diameter of 1.8 or more.

In the system for purifying the product produced by cells, the liquid contact surface may have a pore diameter having a coefficient of variation of 0.5 or less and a ratio of pore diameter/fiber diameter of 1.3 or less, and the liquid contact surface may also have a ratio of long pore diameter/short pore diameter of 1.8 or more.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a ratio of long pore diameter/short pore diameter of 1.8 or more.

In the system for purifying the product produced by cells, the liquid contact surface may have an opening ratio of 57.5% or less, and the liquid contact surface may also have a structural anisotropy of 0.97 or less, and a ratio of long pore diameter/short pore diameter of 1.8 or more.

In the system for purifying the product produced by cells, the liquid contact surface may have a pore diameter having a coefficient of variation of 0.5 or less, and the liquid contact surface may also have a structural anisotropy of 0.97 or less, and a ratio of long pore diameter/short pore diameter of 1.8 or more.

In the system for purifying the product produced by cells, the liquid contact surface may have a ratio of pore diameter/fiber diameter of 1.3 or less, and the liquid contact surface may also have a structural anisotropy of 0.97 or less, and a ratio of long pore diameter/short pore diameter of 1.8 or more.

In the system for purifying the product produced by cells, the liquid contact surface may have an opening ratio of 57.5% or less and a coefficient of variation of pore diameter of 0.5 or less, and the liquid contact surface may also have a structural anisotropy of 0.97 or less, and a ratio of long pore diameter/short pore diameter of 1.8 or more.

In the system for purifying the product produced by cells, the liquid contact surface may have an opening ratio of 57.5% or less and a ratio of pore diameter/fiber diameter of 1.3 or less, and the liquid contact surface may also have a structural anisotropy of 0.97 or less, and a ratio of long pore diameter/short pore diameter of 1.8 or more.

In the system for purifying the product produced by cells, the liquid contact surface may have a pore diameter having a coefficient of variation of 0.5 or less and a ratio of pore diameter/fiber diameter of 1.3 or less, and the liquid contact surface may also have a structural anisotropy of 0.97 or less, and a ratio of long pore diameter/short pore diameter of 1.8 or more.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, and the liquid contact surface may also have a structural anisotropy of 0.97 or less, and a ratio of long pore diameter/short pore diameter of 1.8 or more.

[145] The system for purifying a product produced by cells according to [144], in which the porous membrane is a hollow fiber membrane.

[146] The system for purifying a product produced by cells according to [145], in which the hollow fiber membrane has a homogeneous structure that is substantially homogeneous from a primary side toward a secondary side in the membrane thickness direction.

[147] The system for purifying a product produced by cells according to [145] or [146], in which a pore of the hollow fiber membrane has a blocking pore diameter of 0.05 $\mu$m or more and 10 $\mu$m or less.

[148] The system for purifying a product produced by cells according to any of [145] to [147], in which the hollow fiber membrane consists of a synthetic polymer membrane.

[149] The system for purifying a product produced by cells according to [148], in which the synthetic polymer is polyvinylidene fluoride.

[150] The system for purifying a product produced by cells according to any of [144] to [149], in which the controller measures the shear stress based on a viscosity of the cell broth, and controls, in a direction parallel to a membrane surface, a flow speed of the cell broth to be filtered through the porous membrane such that the measured shear stress is 4.0 N/m$^2$ or more.

[151] The system for purifying a product produced by cells according to any of [144] to [150], in which the controller receives an input of data of a viscosity of the cell broth.

[152] The system for purifying a product produced by cells according to any of [144] to [151], further including: a flow channel for returning the cell broth that is not filtered through the hollow fiber membrane, to the culture vessel.

[153] The system for purifying a product produced by cells according to any of [144] to [152], in which the product is an antibody.

[154] The system for purifying a product produced by cells according to any of [144] to [153], in which the cells are subjected to perfusion culture in the culture vessel.

[155] A method for producing a product by cells, the method including: filtering a cell broth through a porous membrane to obtain a product by the cells, in which the porous membrane has a liquid contact surface having at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or more and 1.03 or less and a ratio of long pore diameter/short pore diameter of 1.8 or less, and the porous membrane has at least any one selected from the group consisting of an average pore diameter of 10 $\mu$m or less on the liquid contact surface and a ratio of maximum value/minimum value of an average pore diameter in a membrane thickness direction of 1 or more and 10 or less, and a cell density contained in the cell broth to be filtered is $8 \times 10^7$ cells/mL or more.

In the method for producing the product by cells, the liquid contact surface may have an opening ratio of 57.5% or less, the liquid contact surface may also have a structural anisotropy of 0.97 or more and 1.03 or less, and the porous membrane may also have an average pore diameter of 10 $\mu$m or less on the liquid contact surface.

In the method for producing the product by cells, the liquid contact surface may have a pore diameter having a coefficient of variation of 0.5 or less, the liquid contact surface may also have a structural anisotropy of 0.97 or more and 1.03 or less, and the porous membrane may also have an average pore diameter of 10 $\mu$m or less on the liquid contact surface.

In the method for producing the product by cells, the liquid contact surface may have a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface.

In the method for producing the product by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have an average pore diameter of 10 $\mu$m or less on the liquid contact surface.

In the method for producing the product by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface.

In the method for producing the product by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface.

In the method for producing the product by cells, the liquid contact surface may have an opening ratio of 57.5% or less, the liquid contact surface may also have a ratio of long pore diameter/short pore diameter of 1.8 or less, and the porous membrane may also have an average pore diameter of 10 $\mu$m or less on the liquid contact surface.

In the method for producing the product by cells, the liquid contact surface may have a pore diameter having a coefficient of variation of 0.5 or less, the liquid contact surface may also have a ratio of long pore diameter/short pore diameter of 1.8 or less, and the porous membrane may also have an average pore diameter of 10 $\mu$m or less on the liquid contact surface.

In the method for producing the product by cells, the liquid contact surface may have a ratio of pore diameter/fiber diameter of 1.3 or less, the liquid contact surface may also have a ratio of long pore diameter/short pore diameter of 1.8 or less, and the porous membrane may also have an average pore diameter of 10 $\mu$m or less on the liquid contact surface.

In the method for producing the product by cells, the liquid contact surface may have an opening ratio of 57.5% or less and a pore diameter having a coefficient of variation of 0.5 or less, the liquid contact surface may also have a ratio of long pore diameter/short pore diameter of 1.8 or less, and the porous membrane may also have an average pore diameter of 10 $\mu$m or less on the liquid contact surface.

In the method for producing the product by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have an average pore diameter of 10 $\mu$m or less on the liquid contact surface.

In the method for producing the product by cells, the liquid contact surface may have a pore diameter having a coefficient of variation of 0.5 or less and a ratio of pore diameter/fiber diameter of 1.3 or less, the liquid contact surface may also have a ratio of long pore diameter/short pore diameter of 1.8 or less, and the porous membrane may also have an average pore diameter of 10 $\mu$m or less on the liquid contact surface.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have an average pore diameter of 10 $\mu$m or less on the liquid contact surface.

In the method for producing the product by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the method for producing the product by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the method for producing the product by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the method for producing the product by cells, the liquid contact surface may have an opening ratio of 57.5% or less and a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the method for producing the product by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the method for producing the product by cells, the liquid contact surface may have a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the method for producing the product by cells, the liquid contact surface may have an opening ratio of 57.5% or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for producing the product by cells, the liquid contact surface may have a pore diameter having a coefficient of variation of 0.5 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for producing the product by cells, the liquid contact surface may have a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for producing the product by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for producing the product by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for producing the product by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may have a structural anisotropy of 0.97 or more and 1.03 or less, and the porous membrane may also have a ratio of maximum value/minimum value of an average pore diameter in a membrane thickness direction of 1 or more and 10 or less.

In the method for producing the product by cells, the liquid contact surface may have an opening ratio of 57.5% or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have a pore diameter having a coefficient of variation of 0.5 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have an opening ratio of 57.5% or less and a coefficient of variation of pore diameter of 0.5 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have an opening ratio of 57.5% or less and a coefficient of variation of pore diameter of 0.5 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may also have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for producing the product by cells, the liquid contact surface may have an opening ratio of 57.5% or less, the liquid contact surface may have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for producing the product by cells, the liquid contact surface may have a pore diameter having a coefficient of variation of 0.5 or less, the liquid contact surface may also have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for producing the product by cells, the liquid contact surface may have a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for producing the product by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for producing the product by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for producing the product by cells, the liquid contact surface may have a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for producing the product by cells, the liquid contact surface may have an opening ratio of 57.5% or less, the liquid contact surface may also have a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for producing the product by cells, the liquid contact surface may have a pore diameter having a coefficient of variation of 0.5 or less, the liquid contact surface may also have a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for producing the product by cells, the liquid contact surface may have a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for producing the product by cells, the liquid contact surface may have an opening ratio of 57.5% or less and a coefficient of variation of pore diameter of 0.5 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for producing the product by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for producing the product by cells, the liquid contact surface may have a pore diameter having a coefficient of variation of 0.5 or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for producing the product by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for producing the product by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for producing the product by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for producing the product by cells, the liquid contact surface may have an opening ratio of 57.5% or less and a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for producing the product by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for producing the product by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

[156] The method for producing a product by cells according to [155], in which the porous membrane is a hollow fiber membrane.

[157] The method for producing a product by cells according to [156], in which the hollow fiber membrane has a homogeneous structure that is substantially homogeneous from a primary side toward a secondary side in the membrane thickness direction.

[158] The method for producing a product by cells according to [156] or [157], in which a pore of the hollow fiber membrane has a blocking pore diameter of 0.05 μm or more and 10 μm or less.

[159] The method for producing a product by cells according to any of [156] to [158], in which the hollow fiber membrane consists of a synthetic polymer membrane.

[160] The method for producing a product by cells according to [159], in which the synthetic polymer is polyvinylidene fluoride.

[161] The method for producing a product by cells according to any of [155] to [160], in which the cell broth that is not filtered through the hollow fiber membrane is returned to a culture vessel of the cells.

[162] The method for producing a product by cells according to any of [155] to [161], in which the product is an antibody.

[163] The method for producing a product by cells according to any of [155] to [162], in which the cells are subjected to perfusion culture.

[164] A method for purifying a product produced by cells, the method including: filtering a cell broth through a porous membrane to purify a product produced by the cells, in which the porous membrane has a liquid contact surface having at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or more and 1.03 or less and a ratio of long pore diameter/short pore diameter of 1.8 or less, and the porous membrane has at least any one selected from the group consisting of an average pore diameter of 10 μm or less on the liquid contact surface and a ratio of maximum value/minimum value of an average pore diameter in a membrane thickness direction of 1 or more and 10 or less, and a cell density contained in the cell broth to be filtered is $8 \times 10^7$ cells/mL or more.

In the method for purifying the product produced by cells, the liquid contact surface may have an opening ratio of 57.5% or less, the liquid contact surface may also have a structural anisotropy of 0.97 or more and 1.03 or less, and the porous membrane may also have an average pore diameter of 10 μm or less on the liquid contact surface.

In the method for purifying the product produced by cells, the liquid contact surface may have a pore diameter having a coefficient of variation of 0.5 or less, the liquid contact surface may also have a structural anisotropy of 0.97 or more and 1.03 or less, and the porous membrane may also have an average pore diameter of 10 μm or less on the liquid contact surface.

In the method for purifying the product produced by cells, the liquid contact surface may have a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface.

In the method for purifying the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have an average pore diameter of 10 μm or less on the liquid contact surface.

In the method for purifying the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface.

In the method for purifying the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface.

In the method for purifying the product produced by cells, the liquid contact surface may have an opening ratio of 57.5% or less, the liquid contact surface may also have a ratio of long pore diameter/short pore diameter of 1.8 or less, and the porous membrane may also have an average pore diameter of 10 μm or less on the liquid contact surface.

In the method for purifying the product produced by cells, the liquid contact surface may have a pore diameter having a coefficient of variation of 0.5 or less, the liquid contact surface may also have a ratio of long pore diameter/short pore diameter of 1.8 or less, and the porous membrane may also have an average pore diameter of 10 μm or less on the liquid contact surface.

In the method for purifying the product produced by cells, the liquid contact surface may have a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface.

In the method for purifying the product produced by cells, the liquid contact surface may have an opening ratio of 57.5% or less and a pore diameter having a coefficient of variation of 0.5 or less, the liquid contact surface may also have a ratio of long pore diameter/short pore diameter of 1.8 or less, and the porous membrane may also have an average pore diameter of 10 μm or less on the liquid contact surface.

In the method for purifying the product produced by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface.

In the method for purifying the product produced by cells, the liquid contact surface may have a pore diameter having a coefficient of variation of 0.5 or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface.

In the method for purifying the product produced by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for purifying the product produced by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for purifying the product produced by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for purifying the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for purifying the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for purifying the product produced by cells, the liquid contact surface may have a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for purifying the product produced by cells, the liquid contact surface may have an opening ratio of 57.5% or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for purifying the product produced by cells, the liquid contact surface may have a pore diameter having a coefficient of variation of 0.5 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for purifying the product produced by cells, the liquid contact surface may have a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for purifying the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for purifying the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for purifying the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may have a structural anisotropy of 0.97 or more and 1.03 or less, and the porous membrane may also have a ratio of maximum value/minimum value of an average pore diameter in a membrane thickness direction of 1 or more and 10 or less.

In the method for purifying the product produced by cells, the liquid contact surface may have an opening ratio of 57.5% or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have a pore diameter having a coefficient of variation of 0.5 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have an opening ratio of 57.5% or less and a coefficient of variation of pore diameter of 0.5 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have an opening ratio of 57.5% or less and a coefficient of variation of pore diameter of 0.5 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for purifying the product produced by cells, the liquid contact surface may have an opening ratio of 57.5% or less, the liquid contact surface may have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have a pore diameter having a coefficient of variation of 0.5 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have an opening ratio of 57.5% or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have a pore diameter having a coefficient of variation of 0.5 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have an opening ratio of 57.5% or less and a coefficient of variation of pore diameter of 0.5 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have a pore diameter having a coefficient of variation of 0.5 or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for purifying the product produced by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for purifying the product produced by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for purifying the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for purifying the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for purifying the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

[165] The method for purifying a product produced by cells according to [164], in which the porous membrane is a hollow fiber membrane.

[166] The method for purifying a product produced by cells according to [165], in which the hollow fiber membrane has a homogeneous structure that is substantially homogeneous from a primary side toward a secondary side in the membrane thickness direction.

[167] The method for purifying a product produced by cells according to [165] or [166], in which a pore of the hollow fiber membrane has a blocking pore diameter of 0.05 $\mu$m or more and 10 $\mu$m or less.

[168] The method for purifying a product produced by cells according to any of [165] to [167], in which the hollow fiber membrane consists of a synthetic polymer membrane.

[169] The method for purifying a product produced by cells according to [168], in which the synthetic polymer is polyvinylidene fluoride.

[170] The method for purifying a product produced by cells according to any of [165] to [169], in which the cell broth that is not filtered through the hollow fiber membrane is returned to a culture vessel of the cells.

[171] The method for purifying a product produced by cells according to any of [164] to [170], in which the product is an antibody.

[172] The method for purifying a product produced by cells according to any of [164] to [171], in which the cells are subjected to perfusion culture.

[173] A method for suppressing a decrease in a membrane permeability of a product produced by cells, the method including: filtering a cell broth through a porous membrane to obtain a product by the cells, in which the method is carried out such that; the porous membrane has a liquid contact surface having at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or more and 1.03 or less and a ratio of long pore diameter/short pore diameter of 1.8 or less, and the porous membrane has at least any one selected from the group consisting of an average pore diameter of 10 $\mu$m or less on the liquid contact surface and a ratio of maximum value/minimum value of an average pore diameter in a membrane thickness direction of 1 or more and 10 or less, and a cell density contained in the cell broth to be filtered is $8 \times 10^7$ cells/mL or more.

In the method for suppressing the decrease in the membrane permeability of the product produced by cells, the liquid contact surface may have an opening ratio of 57.5% or less, the liquid contact surface may also have a structural anisotropy of 0.97 or more and 1.03 or less, and the porous membrane may also have an average pore diameter of 10 $\mu$m or less on the liquid contact surface.

In the method for suppressing the decrease in the membrane permeability of the product produced by cells, the liquid contact surface may have a pore diameter having a coefficient of variation of 0.5 or less, the liquid contact surface may also have a structural anisotropy of 0.97 or more and 1.03 or less, and the porous membrane may also have an average pore diameter of 10 $\mu$m or less on the liquid contact surface.

In the method for suppressing the decrease in the membrane permeability of the product produced by cells, the liquid contact surface may have a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface.

In the method for suppressing the decrease in the membrane permeability of the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have an average pore diameter of 10 $\mu$m or less on the liquid contact surface.

In the method for suppressing the decrease in the membrane permeability of the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface.

In the method for suppressing the decrease in the membrane permeability of the product produced by cells, the liquid contact surface may have an opening ratio of 57.5% or less, the liquid contact surface may also have a ratio of long pore diameter/short pore diameter of 1.8 or less, and the porous membrane may also have an average pore diameter of 10 $\mu$m or less on the liquid contact surface.

In the method for suppressing the decrease in the membrane permeability of the product produced by cells, the liquid contact surface may have a pore diameter having a coefficient of variation of 0.5 or less, the liquid contact surface may also have a ratio of long pore diameter/short pore diameter of 1.8 or less, and the porous membrane may also have an average pore diameter of 10 $\mu$m or less on the liquid contact surface.

In the method for suppressing the decrease in the membrane permeability of the product produced by cells, the liquid contact surface may have a ratio of pore diameter/fiber diameter of 1.3 or less, the liquid contact surface may also have a ratio of long pore diameter/short pore diameter of 1.8 or less, and the porous membrane may also have an average pore diameter of 10 $\mu$m or less on the liquid contact surface.

In the method for suppressing the decrease in the membrane permeability of the product produced by cells, the liquid contact surface may have an opening ratio of 57.5% or less and a pore diameter having a coefficient of variation of 0.5 or less, the liquid contact surface may also have a ratio of long pore diameter/short pore diameter of 1.8 or less, and the porous membrane may also have an average pore diameter of 10 $\mu$m or less on the liquid contact surface.

In the method for suppressing the decrease in the membrane permeability of the product produced by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have an average pore diameter of 10 $\mu$m or less on the liquid contact surface.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface.

In the method for suppressing the decrease in the membrane permeability of the product produced by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for suppressing the decrease in the membrane permeability of the product produced by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for suppressing the decrease in the membrane permeability of the product produced by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for suppressing the decrease in the membrane permeability of the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for suppressing the decrease in the membrane permeability of the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface.

In the method for suppressing the decrease in the membrane permeability of the product produced by cells, the liquid contact surface may have an opening ratio of 57.5% or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have a pore diameter having a coefficient of variation of 0.5 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may have a structural anisotropy of 0.97 or more and 1.03 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have a pore diameter having a coefficient of variation of 0.5 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less, and a coefficient of variation of pore diameter of 0.5 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less and a ratio of pore diameter/fiber diameter of 1.3 or less, and the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less, the liquid contact surface may have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have a pore diameter having a coefficient of variation of 0.5 or less, the liquid contact surface may also have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less, the liquid contact surface may also have a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have a pore diameter having a coefficient of variation of 0.5 or less, the liquid contact surface may also have a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have a ratio of pore diameter/fiber diameter of 1.3 or less, the liquid contact surface may also have a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less and a pore diameter having a coefficient of variation of 0.5 or less, the liquid contact surface may also have a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, the liquid contact surface may also have a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the method for suppressing the decrease in the membrane permeability of the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

[174] The method for suppressing a decrease in a membrane permeability of a product produced by cells according to [173], in which the porous membrane is a hollow fiber membrane.

[175] The method for suppressing a decrease in a membrane permeability of a product produced by cells according to [174], in which the hollow fiber membrane has a homogeneous structure that is substantially homogeneous from a primary side toward a secondary side in the membrane thickness direction.

[176] The method for suppressing a decrease in a membrane permeability of a product produced by cells according to [174] or [175], in which a pore of the hollow fiber membrane has a blocking pore diameter of 0.05 μm or more and 10 μm or less.

[177] The method for suppressing a decrease in a membrane permeability of a product produced by cells according to any of [174] to [176], in which the hollow fiber membrane consists of a synthetic polymer membrane.

[178] The method for suppressing a decrease in a membrane permeability of a product produced by cells according to [177], in which the synthetic polymer is polyvinylidene fluoride.

[179] The method for suppressing a decrease in a membrane permeability of a product produced by cells according to any of [174] to [178], in which the cell broth that is not filtered through the hollow fiber membrane is returned to a culture vessel of the cells.

[180] The method for suppressing a decrease in a membrane permeability of a product produced by cells according to any of [173] to [179], in which the product is an antibody.

[181] The method for suppressing a decrease in a membrane permeability of a product produced by cells according to any of [173] to [180], in which the cells are subjected to perfusion culture.

[182] A system for producing a product by cells, the system including: a culture vessel for culturing cells; a porous membrane for filtering a cell broth to obtain a product by the cells; and a pump for feeding the cell broth from the culture vessel to the porous membrane, in which a liquid contact surface of the porous membrane has at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or more and 1.03 or less and a ratio of long pore diameter/short pore diameter of 1.8 or less, and the porous membrane has at least any one selected from the group consisting of an average pore diameter of 10 μm or less on the liquid contact surface and a ratio of maximum value/minimum value of an average pore diameter in a membrane thickness direction of 1 or more and 10 or less, and a cell density contained in the cell broth to be filtered is $8 \times 10^7$ cells/mL or more.

In the system for producing the product by cells, the liquid contact surface may have an opening ratio of 57.5% or less, the liquid contact surface may also have a structural anisotropy of 0.97 or more and 1.03 or less, and the porous membrane may also have an average pore diameter of 10 μm or less on the liquid contact surface.

In the system for producing the product by cells, the liquid contact surface may have a pore diameter having a coefficient of variation of 0.5 or less, the liquid contact surface may also have a structural anisotropy of 0.97 or more and 1.03 or less, and the porous membrane may also have an average pore diameter of 10 μm or less on the liquid contact surface.

In the system for producing the product by cells, the liquid contact surface may have a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface.

In the system for producing the product by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have an average pore diameter of 10 μm or less on the liquid contact surface.

In the system for producing the product by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface.

In the system for producing the product by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface.

In the system for producing the product by cells, the liquid contact surface may have an opening ratio of 57.5% or less, the liquid contact surface may also have a ratio of long pore diameter/short pore diameter of 1.8 or less, and the porous membrane may also have an average pore diameter of 10 μm or less on the liquid contact surface.

In the system for producing the product by cells, the liquid contact surface may have a pore diameter having a coefficient of variation of 0.5 or less, the liquid contact surface may also have a ratio of long pore diameter/short pore diameter of 1.8 or less, and the porous membrane may also have an average pore diameter of 10 μm or less on the liquid contact surface.

In the system for producing the product by cells, the liquid contact surface may have a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface.

In the system for producing the product by cells, the liquid contact surface may have an opening ratio of 57.5% or less and a pore diameter having a coefficient of variation of 0.5 or less, the liquid contact surface may also have a ratio of long pore diameter/short pore diameter of 1.8 or less, and the porous membrane may also have an average pore diameter of 10 μm or less on the liquid contact surface.

In the system for producing the product by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface.

In the system for producing the product by cells, the liquid contact surface may have a pore diameter having a coefficient of variation of 0.5 or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface.

In the system for producing the product by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the system for producing the product by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the system for producing the product by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the system for producing the product by cells, the liquid contact surface may have an opening ratio of 57.5% or less and a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the system for producing the product by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the system for producing the product by cells, the liquid contact surface may have a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 $\mu$m or less on the liquid contact surface.

In the system for producing the product by cells, the liquid contact surface may have an opening ratio of 57.5% or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the system for producing the product by cells, the liquid contact surface may have a pore diameter having a coefficient of variation of 0.5 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the system for producing the product by cells, the liquid contact surface may have a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the system for producing the product by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the system for producing the product by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the system for producing the product by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may have a structural anisotropy of 0.97 or more and 1.03 or less, and the porous membrane may also have a ratio of maximum value/minimum value of an average pore diameter in a membrane thickness direction of 1 or more and 10 or less.

In the system for producing the product by cells, the liquid contact surface may have an opening ratio of 57.5% or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have a pore diameter having a coefficient of variation of 0.5 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have an opening ratio of 57.5% or less and a coefficient of variation of pore diameter of 0.5 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have an opening ratio of 57.5% or less and a coefficient of variation of pore diameter of 0.5 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for producing the product by cells, the liquid contact surface may have an opening ratio of 57.5% or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have a pore diameter having a coefficient of variation of 0.5 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have an opening ratio of 57.5% or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have a pore diameter having a coefficient of variation of 0.5 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have an opening ratio of 57.5% or less and a coefficient of variation of pore diameter of 0.5 or less, the liquid contact surface may also have a ratio of long pore diameter/short pore diameter of 1.8 or less, and the porous membrane may also have an average pore diameter of 10 μm

or less on the liquid contact surface, and a ratio of maximum value/minimum value of an average pore diameter in a membrane thickness direction of 1 or more and 10 or less.

In the system for producing the product by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have a pore diameter having a coefficient of variation of 0.5 or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have an opening ratio of 57.5% or less and a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for producing the product by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

[183] The system for producing a product by cells according to [182], in which the porous membrane is a hollow fiber membrane.

[184] The system for producing a product by cells according to [183], in which the hollow fiber membrane has a homogeneous structure that is substantially homogeneous from a primary side toward a secondary side in the membrane thickness direction.

[185] The system for producing a product by cells according to [183] or [184], in which a pore of the hollow fiber membrane has a blocking pore diameter of 0.05 μm or more and 10 μm or less.

[186] The system for producing a product by cells according to any of [183] to [185], in which the hollow fiber membrane consists of a synthetic polymer membrane.

[187] The system for producing a product by cells according to [186], in which the synthetic polymer is polyvinylidene fluoride.

[188] The system for producing a product by cells according to any of [182] to [187], further including: a controller that receives an input of data of a viscosity of the cell broth.

[189] The system for producing a product by cells according to any of [182] to [188], further including: a flow channel for returning the cell broth that is not filtered through the hollow fiber membrane, to the culture vessel.

[190] The system for producing a product by cells according to any of [182] to [189], in which the product is an antibody.

[191] The system for producing a product by cells according to any of [182] to [190], in which the cells are subjected to perfusion culture in the culture vessel.

[192] A system for purifying a product produced by cells, the system including: a culture vessel for culturing cells; a porous membrane for filtering a cell broth to purify a product produced by the cells; and a pump for feeding the cell broth from the culture vessel to the porous membrane, in which a liquid contact surface of the porous membrane has at least any one selected from the group consisting of an opening ratio of 57.5% or less, a pore diameter having a coefficient of variation of 0.5 or less, and a ratio of pore diameter/fiber diameter of 1.3 or less, and the liquid contact surface has at least any one selected from the group consisting of a structural anisotropy of 0.97 or more and 1.03 or less and a ratio of long pore diameter/short pore diameter of 1.8 or less, and the porous membrane has at least any one selected from the group consisting of an average pore diameter of 10 μm or less on the liquid contact surface and a ratio of maximum value/minimum value of an average pore diameter in a membrane thickness direction of 1 or more and 10 or less, and a cell density contained in the cell broth to be filtered is $8 \times 10^7$ cells/mL or more.

In the system for purifying the product produced by cells, the liquid contact surface may have an opening ratio of 57.5% or less, the liquid contact surface may also have a structural anisotropy of 0.97 or more and 1.03 or less, and the porous membrane may also have an average pore diameter of 10 μm or less on the liquid contact surface.

In the system for purifying the product produced by cells, the liquid contact surface may have a pore diameter having a coefficient of variation of 0.5 or less, the liquid contact surface may also have a structural anisotropy of 0.97 or more and 1.03 or less, and the porous membrane may also have an average pore diameter of 10 μm or less on the liquid contact surface.

In the system for purifying the product produced by cells, the liquid contact surface may have a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface.

In the system for purifying the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have an average pore diameter of 10 μm or less on the liquid contact surface.

In the system for purifying the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface.

In the system for purifying the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface.

In the system for purifying the product produced by cells, the liquid contact surface may have an opening ratio of 57.5% or less, the liquid contact surface may also have a ratio of long pore diameter/short pore diameter of 1.8 or less, and the porous membrane may also have an average pore diameter of 10 µm or less on the liquid contact surface.

In the system for purifying the product produced by cells, the liquid contact surface may have a pore diameter having a coefficient of variation of 0.5 or less, the liquid contact surface may also have a ratio of long pore diameter/short pore diameter of 1.8 or less, and the porous membrane may also have an average pore diameter of 10 µm or less on the liquid contact surface.

In the system for purifying the product produced by cells, the liquid contact surface may have a ratio of pore diameter/fiber diameter of 1.3 or less, the liquid contact surface may also have a ratio of long pore diameter/short pore diameter of 1.8 or less, and the porous membrane may also have an average pore diameter of 10 µm or less on the liquid contact surface.

In the system for purifying the product produced by cells, the liquid contact surface may have an opening ratio of 57.5% or less and a pore diameter having a coefficient of variation of 0.5 or less, the liquid contact surface may also have a ratio of long pore diameter/short pore diameter of 1.8 or less, and the porous membrane may also have an average pore diameter of 10 µm or less on the liquid contact surface.

In the system for purifying the product produced by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have an average pore diameter of 10 µm or less on the liquid contact surface.

In the system for purifying the product produced by cells, the liquid contact surface may have a pore diameter having a coefficient of variation of 0.5 or less and a ratio of pore diameter/fiber diameter of 1.3 or less, the liquid contact surface may also have a ratio of long pore diameter/short pore diameter of 1.8 or less, and the porous membrane may also have an average pore diameter of 10 µm or less on the liquid contact surface.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have an average pore diameter of 10 µm or less on the liquid contact surface.

In the system for purifying the product produced by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have an average pore diameter of 10 µm or less on the liquid contact surface.

In the system for purifying the product produced by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 µm or less on the liquid contact surface.

In the system for purifying the product produced by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 µm or less on the liquid contact surface.

In the system for purifying the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 µm or less on the liquid contact surface.

In the system for purifying the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 µm or less on the liquid contact surface.

In the system for purifying the product produced by cells, the liquid contact surface may have a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 µm or less on the liquid contact surface.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 µm or less on the liquid contact surface.

In the system for purifying the product produced by cells, the liquid contact surface may have an opening ratio of 57.5% or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the system for purifying the product produced by cells, the liquid contact surface may have a pore diameter having a coefficient of variation of 0.5 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the system for purifying the product produced by cells, the liquid contact surface may have a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the system for purifying the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the system for purifying the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the system for purifying the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less, and the porous membrane may also have a ratio of maximum value/minimum value of an average pore diameter in a membrane thickness direction of 1 or more and 10 or less.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may have a structural anisotropy of 0.97 or more and 1.03 or less, and the porous membrane may also have a ratio of maximum value/minimum value of an average pore diameter in a membrane thickness direction of 1 or more and 10 or less.

In the system for purifying the product produced by cells, the liquid contact surface may have an opening ratio of 57.5% or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have a pore diameter having a coefficient of variation of 0.5 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have an opening ratio of 57.5% or less and a coefficient of variation of pore diameter of 0.5 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have an opening ratio of 57.5% or less and a coefficient of variation of pore diameter of 0.5 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have a ratio of maximum value/minimum value of an average pore diameter in a membrane thickness direction of 1 or more and 10 or less.

In the system for purifying the product produced by cells, the liquid contact surface may have an opening ratio of 57.5% or less, the liquid contact surface may have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have a pore diameter having a coefficient of variation of 0.5 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have an opening ratio of 57.5% or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have a pore diameter having a coefficient of variation of 0.5 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have an opening ratio of 57.5% or less and a coefficient of variation of pore diameter of 0.5 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have an opening ratio of 57.5% or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have a pore diameter having a coefficient of variation of 0.5 or less and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have an opening ratio of 57.5% or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have an opening ratio of 57.5% or less and a pore diameter having a coefficient of variation of 0.5 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have an opening ratio of 57.5% or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have a pore diameter having a coefficient of variation of 0.5 or less and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

In the system for purifying the product produced by cells, the liquid contact surface may have an opening ratio of 57.5% or less, a pore diameter having a coefficient of variation of 0.5 or less, and a ratio of pore diameter/fiber diameter of 1.3 or less, the liquid contact surface may also have a structural anisotropy of 0.97 or more and 1.03 or less and a ratio of long pore diameter/short pore diameter of 1.8 or less, and the porous membrane may also have an average pore diameter of 10 μm or less on the liquid contact surface, and a ratio of maximum value/minimum value of an average pore diameter in a membrane thickness direction of 1 or more and 10 or less.

[193] The system for purifying a product produced by cells according to [192], in which the porous membrane is a hollow fiber membrane.

[194] The system for purifying a product produced by cells according to [193], in which the hollow fiber membrane has a homogeneous structure that is substantially homogeneous from a primary side toward a secondary side in the membrane thickness direction.

[195] The system for purifying a product produced by cells according to [193] or [194], in which a pore of the hollow fiber membrane has a blocking pore diameter of 0.05 μm or more and 10 μm or less.

[196] The system for purifying a product produced by cells according to any of [193] to [195], in which the hollow fiber membrane consists of a synthetic polymer membrane.

[197] The system for purifying a product produced by cells according to [196], in which the synthetic polymer is polyvinylidene fluoride.

[198] The system for purifying a product produced by cells according to any of [192] to [197], further including: a controller that receives an input of data of a viscosity of the cell broth.

[199] The system for purifying a product produced by cells according to any of [192] to [198], further including: a flow channel for returning the cell broth that is not filtered through the hollow fiber membrane, to the culture vessel.

[200] The system for purifying a product produced by cells according to any of [192] to [199], in which the product is an antibody.

[201] The system for purifying a product produced by cells according to any of [192] to [200], in which the cells are subjected to perfusion culture in the culture vessel.

Advantageous Effect of Invention

According to the present invention, it is possible to provide a method for producing a product by cells, a method for purifying a product produced by cells, a method for suppressing a decrease in a membrane permeability of a product produced by cells, a system for producing a product by cells, and a system for purifying a product produced by cells, all of which make it possible to suppress a decrease in a permeation rate of a product produced by cells in the porous membrane.

EP 4 621 062 A1

Brief Description of Drawings

[Figure 1] Figure 1 is a view schematically illustrating a system for producing a product by cells according to an embodiment.
[Figure 2] Figure 2 is a view illustrating an example of image processing in a Local thickness method according to an embodiment.
[Figure 3] Figure 3 is a view illustrating an example of a maximum circle illustrated in the Local thickness method according to the embodiment.
[Figure 4] Figure 4 is a view illustrating an example of a maximum circle, which is illustrated in the Local thickness method according to the embodiment.
[Figure 5] Figure 5 is a view illustrating an example of image processing according to an embodiment.
[Figure 6] Figure 6 is a view illustrating an example of image processing in a case of calculating a structural anisotropy Z' of a surface of a porous membrane according to an embodiment.
[Figure 7] Figure 7 is a view schematically illustrating a distribution of a flow speed of a fluid flowing through a hollow portion and a distribution of a shear stress in a case where the porous membrane according to the embodiment is a hollow fiber membrane.
[Figure 8] Figure 8 is a view schematically illustrating a cake layer and clogging in a cross section in a case where the porous membrane according to the embodiment is a hollow fiber membrane.
[Figure 9] Figure 9 is a graph showing a relationship between a shear stress on a liquid contact surface of a hollow fiber membrane according to Examples and a permeation rate of an antibody at a filtration amount of 300 L/m$^2$.
[Figure 10] Figure 10 is a graph showing a relationship between a shear stress on a liquid contact surface of a hollow fiber membrane according to Examples and a transmembrane pressure at a filtration amount of 300 L/m$^2$.
[Figure 11] Figure 11 is a graph showing a relationship between a shear stress on a liquid contact surface of a hollow fiber membrane according to Examples and a permeation rate of an antibody at a filtration amount of 300 L/m$^2$.
[Figure 12] Figure 12 is a graph showing a relationship between a shear stress on a liquid contact surface of a hollow fiber membrane according to Examples and a transmembrane pressure at a filtration amount of 300 L/m$^2$.
[Figure 13] Figure 13 is a graph showing a relationship between a shear stress on a liquid contact surface of a hollow fiber membrane according to Examples and a permeation rate of an antibody at a filtration amount of 300 L/m$^2$.
[Figure 14] Figure 14 is a graph showing a relationship between a shear stress on a liquid contact surface of a hollow fiber membrane according to Examples and a transmembrane pressure at a filtration amount of 300 L/m$^2$.

Description of Embodiments

Hereinafter, embodiments (hereinafter, referred to as "the present embodiments") for carrying out the present invention will be described in detail. It is noted that the present embodiment is provided to facilitate understanding of the present invention and thus is not intended to limit the present invention. The present invention is not limited to the present embodiment and thus can be implemented with various modifications within the scope of the present invention.

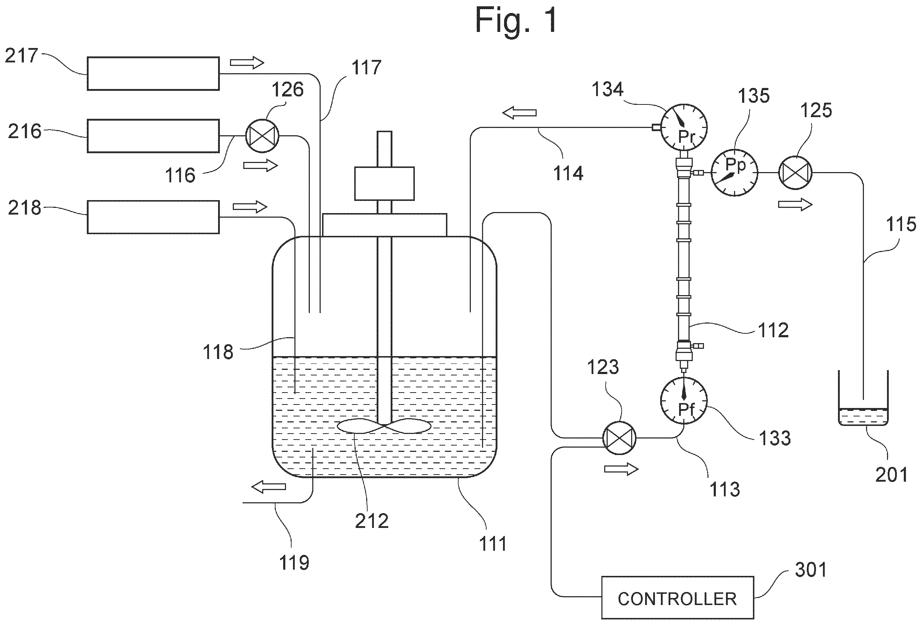

With reference to Figure 1, a method for producing a product by cells according to the present embodiment includes filtering a cell broth through a porous membrane 112 to obtain a product by the cells. For example, the cell broth is fed from a culture vessel 111 to the porous membrane 112. The culture vessel 111 may have a ventilation port or filter having a pore diameter of 0.2 μm or less in order to prevent external bacteria from entering the inside of the culture vessel 111. The culture vessel 111 may be equipped with a stirring device 212 for stirring the cell broth in the culture vessel 111. The cells are subjected to floating culture in the cell broth in the culture vessel 111.

The cells to be cultured in the culture vessel 111 are not particularly limited. The cell may be derived from an animal including a human or may be derived from a microorganism. The cell may be a eukaryotic cell or may be a prokaryotic cell. Examples of the animal include mammals, reptiles, birds, amphibians, fish, and insects. The cell may be a genetically recombinant cell. Examples of the cells include a Chinese hamster ovary (CHO) cell, an HEK cell, a BHK-21 cell, an Sp2/0 cell, an SP2/0-Ag14 cell, an NS0 cell, a Vero cell, a PER.C6 cell, a yeast, Bacillus subtilis, and Escherichia coli.

The cells produce, for example, a product that can be used as a pharmaceutical drug and release the product into the cell broth. Examples of the product that can be used as a pharmaceutical drug include a peptide, a protein, and a virus (including a virus-like particle). Examples of the protein include an antibody, a hormone, a cytokine, a growth factor, an enzyme, and a blood plasma protein. The protein may be a recombinant protein.

The antibody may be a monoclonal antibody or may be a polyclonal antibody. The antibody may be a human antibody or may be an antibody protein derived from mammals other than a human, such as a bovine and mouse. Alternatively, the antibody may be a chimeric antibody protein with human IgG or a humanized antibody. The chimeric

52

antibody with human IgG is an antibody in which although variable regions are derived from an organism other than a human, such as a mouse, constant regions other than the variable regions are substituted with constant regions of human-derived immunoglobulin. In addition, the humanized antibody is an antibody in which although a complementarity-determining region (CDR) in the variable region is derived from an organism other than a human, a framework region (FR) other than the complementarity-determining region (CDR) is derived from a human. The humanized antibody has reduced immunogenicity as compared with the chimeric antibody.

By filtering the cell broth containing cells using the porous membrane 112, the cells in the cell broth are removed, and the product by the cells in the cell broth is permeated through the porous membrane 112 and purified. In the porous membrane 112, the surface that comes into contact with the cell broth to be filtered is referred to as a liquid contact surface. The shape of the porous membrane 112 is not particularly limited. Examples of the porous membrane include a hollow fiber membrane, a flat membrane, and a tubular membrane. In the following description, an example in which the porous membrane 112 is a hollow fiber membrane will be described. In the hollow fiber membrane, a liquid contact surface to which a cell broth is supplied is referred to as a primary side of the hollow fiber membrane. In addition, a surface on which a permeated solution permeated through the hollow fiber membrane flows out is referred to as a secondary side of the hollow fiber membrane. In an embodiment in which a cell broth to be filtered is supplied to an inner peripheral surface, the inner peripheral surface of the hollow fiber membrane serves as the primary side, and the outer peripheral surface of the hollow fiber membrane serves as the secondary side. In an embodiment in which a cell broth to be filtered is supplied to an outer peripheral surface, the outer peripheral surface of the hollow fiber membrane serves as the primary side, and the inner peripheral surface of the hollow fiber membrane serves as the secondary side.

The filtration method in the porous membrane 112 may be a tangential flow filtration (TFF) method. The tangential flow filtration method is a filtration method in which a cell broth is allowed to flow in a direction parallel to the primary side surface of the hollow fiber membrane on the primary side surface of the hollow fiber membrane. The tangential flow filtration method includes an alternating tangential flow filtration (ATF) method. In the present embodiment, in a case of simply being referred to as a tangential flow filtration (TFF) method, it may refer to a filtration method in which a cell broth is allowed to flow in one direction on the primary side surface of the hollow fiber membrane. The alternating tangential flow filtration (ATF) method refers to a filtration method in which a cell broth is allowed to flow so that it flows back and forth on the primary side surface of the hollow fiber membrane.

A flow channel 113 for feeding the cell broth in the culture vessel 111 to the porous membrane 112 and a flow channel 114 for returning, to the culture vessel 111, a cell broth that has passed through the porous membrane 112 without being filtered through the porous membrane 112 by passing through the hollow portion without passing through the micropore portion of the porous membrane 112 are disposed between the culture vessel 111 and the porous membrane 112. The cell broth flowing through the flow channel 113 may contain the cells and the product by the cells. The cell broth flowing through the flow channel 114, which has passed through the porous membrane 112 without being filtered therethrough, may contain the cells and the product by the cells. In addition, a flow channel 115 for recovering a cell broth filtered by passing through the micropore portions of the porous membrane 112 is connected to the porous membrane 112. The cell broth flowing through the flow channel 115, which has been filtered through the porous membrane 112, may contain the product by cells. The cell broth filtered through the porous membrane 112 is recovered in, for example, a container 201. The container 201 may be aseptically connected to the flow channel 115. The flow channel 115 through which the cell broth filtered through the porous membrane 112 flows may be directly connected to, for example, a column that is used for the next purification step.

For example, the flow channel 113 may be equipped with a pump 123 for feeding the cell broth in the culture vessel 111 to the porous membrane 112. Examples of the pump include a diaphragm pump, a tube pump, a centrifugal pump, and a rotary pump; however, examples thereof are not limited thereto. The flow channel 113 may be equipped with a pressure gauge 133 that measures the pressure of the cell broth that is supplied to the porous membrane 112. The flow channel 113 may be equipped with a flowmeter that measures at least one of a flow speed or a flow rate of the cell broth that flows in the flow channel 113. The flow channel 113 may be equipped with a thermometer that measures the temperature of the cell broth that flows in the flow channel 113. The flow channel 113 may be equipped with a sampling unit for sampling the cell broth that flows in the flow channel 113. The sampling unit is closed except for the time of sampling.

The flow channel 114 may be equipped with a pump for feeding, to the culture vessel 111, a cell broth that has passed through the hollow portion without passing through the micropore portion of the porous membrane 112 and has not been filtered through the porous membrane 112. It is noted that the pump may be provided in both the flow channel 113 and the flow channel 114 or may be provided in any one of the flow channel 113 or the flow channel 114. The flow channel 114 may be equipped with a pressure gauge 134 that measures the pressure of the cell broth that has passed through the porous membrane 112. The flow channel 114 may be equipped with a flowmeter that measures at least one of a flow speed or a flow rate of the cell broth that flows in the flow channel 114. The flow channel 114 may be equipped with a thermometer that measures the temperature of the cell broth that flows in the flow channel 114. The flow channel 114 may be equipped with a sampling unit for sampling the cell broth that flows in the flow channel 114. The sampling unit is closed except for the time of sampling.

The flow channel 115 is equipped with, for example, a pump 125 for feeding the cell broth that has passed through the micropore portion of the porous membrane 112 and then has been filtered through the porous membrane 112. The flow channel 115 may be equipped with a pressure gauge 135 that measures the pressure of the cell broth that has been filtered through the porous membrane 112. The flow channel 115 may be equipped with a flowmeter that measures at least one of a flow speed or a flow rate of the cell broth that flows in the flow channel 115. The flow channel 115 may be equipped with a sampling unit for sampling the cell broth that flows in the flow channel 115. The sampling unit is closed except for the time of sampling.

The culture vessel 111 and the porous membrane 112 form at least a part of a path through which the cell broth circulates. In addition, the flow channels 113 and 114 form at least a part of a path through which the cell broth circulates.

A flow channel 116 for supplying a culture medium to the culture vessel 111 may be connected to the culture vessel 111. The flow channel 116 is connected to, for example, a culture medium tank 216 that contains a culture medium. For example, the flow channel 116 is equipped with a pump 126 for feeding the culture medium to the culture vessel 111. For example, the pump 125 and the pump 126 are controlled such that the amount of the cell broth that is filtered through the porous membrane 112 but is not returned to the culture vessel 111 and the amount of the culture medium that is supplied to the culture vessel 111 are the same. This control may be performed by observing the liquid level height using a liquid level sensor (level sensor) installed in the culture vessel 111 so that the liquid level height is constant, or it may be performed by measuring the weight of the entire culture vessel 111 containing the cell broth so that the weight is constant.

A flow channel 117 for supplying air containing carbon dioxide to the culture vessel 111 may be connected to the culture vessel 111. The flow channel 117 is connected to, for example, a container 217 that contains air containing carbon dioxide. In addition, a flow channel 118 for supplying oxygen to the culture vessel 111 may be connected to the culture vessel 111. The flow channel 118 is connected to, for example, a container 218 that contains oxygen.

A flow channel 119 for discharging at least a part of the cells in the culture vessel 111 may be connected to the culture vessel 111. For example, by discharging at least a part of the cells in the culture vessel 111 using the flow channel 119, the cell density in the cell broth in the culture vessel 111 is kept constant, which suppresses a shortage of oxygen and cell broth components in the cell broth due to an increase in the cell density, or an increase in the concentration of impurities. Discharging at least a part of the cells in the culture vessel 111 is referred to as bleeding. It is noted that although sampling for analyzing the cell broth in the culture vessel 111 may be carried out using the flow channel 119, the sampling is distinguished from bleeding since the sampling is not intended to adjust the cell density.

The culture vessel 111 may be equipped with at least one of a thermometer for measuring a temperature of a cell broth in the culture vessel 111, a dissolved oxygen (DO) meter for measuring DO, or a pH meter for measuring **pH.**

[Method for specifying structure of porous membrane]

The feature quantity of pores, the opening ratio, the fiber diameter, and the anisotropy of the structure in the structure of the liquid contact surface of the porous membrane can be specified according to the following methods.

[Method for acquiring image of liquid contact surface of porous membrane]

In a case where the porous membrane is a hollow fiber membrane, the central portion of the hollow fiber membrane is cut with a razor blade in parallel with the fiber length direction to expose the inner surface, and it is fixed to an observation specimen support of a scanning electron microscope (SEM) with a carbon paste. Using an osmium coater (HPC-30W, manufactured by Vacuum Device Inc.), the prepared specimen is subjected to osmium coating under conditions of an applied voltage adjustment knob setting of 4.5 and a discharge time of 3 seconds, whereby an observation specimen is obtained. The observation specimen is irradiated with an electron beam under the following conditions, and the angle of field of view is adjusted such that the vertical direction of the image is parallel to the fiber length direction of the hollow fiber membrane. In a field of view consisting of only a skeleton resin and pores, the contrast is adjusted by an auto function. The imaging magnification is set to a magnification at which such an inner surface pore diameter distribution that includes 5 or more and 60 or less of pores largest in the vertical direction of the image and includes 7 or more and 60 or less of pores largest in the lateral direction of the image can be described. For example, the inner surface imaging magnification of the hollow fiber membrane used in the first example of the porous membrane described later is set to 150 times, the inner surface imaging magnification of the hollow fiber membrane used in the second example of the porous membrane is set to 5,000 times, and the inner surface imaging magnification of the hollow fiber membrane used in the third example of the porous membrane is set to 3,000 times. A backscattered electron image is obtained, where the backscattered electron image is in a state where there is no distortion of the structure due to charge-up and no abnormal contrast, and the backscattered electron image is composed of an inner surface resin skeleton and inner surface pores but does not include a specimen support, a carbon paste, and a cross section cut with a razor blade. It is noted that even in a case where the porous membrane is a membrane other than a hollow fiber membrane, the liquid contact surface needs only to be imaged in the same manner.

(Conditions for SEM device)

SEM: scanning electron microscope SU7000 manufactured by Hitachi High-Tech Corporation
Probe current: Normal
Acceleration voltage: 1 kV
Operating distance: 6 mm
Detector: MD detector (for backscattered electron image)
Image size: 960 pixels in length $\times$ 1280 pixels in width

Using three lots of hollow fiber membranes for one kind of hollow fiber membrane, SEM images (a total of 9 or more images) from three or more fields of view without overlapping with each other are acquired for each lot.

[Method for calculating opening ratio $C_0$' of liquid contact surface of porous membrane]

A label (text or scale bar portion) of the electron microscope image is removed by trimming. This image is subjected to filtering processing by a median filter using a $5 \times 5$ square kernel, and then two threshold values which are not zero are determined according to the multi-Otsu method. Among the threshold values, the larger value is used as the threshold value, and the binarization of the image, in which pixels equal to or smaller than the threshold value are set to black and pixels other than these pixels are set to white, is carried out. It is noted that the brightness of white is 255, and the brightness of black is 0. The binarized image is subjected to one time of closing processing using a $2 \times 2$ square kernel to remove noise. An opening ratio $C_0$ (%) of one SEM image is calculated from the following Expression (1), where the total number of pixels of the entire image is denoted as $A_0$, and the number of pixels which are black is denoted as $B_0$ with respect to the obtained first analysis target image.

$$C_0 = B_0 \times 100/A_0 \qquad (1)$$

The opening ratio $C_0$ is calculated using SEM images from three or more fields of view (a total of 9 images) for each of three lots of one kind of hollow fiber membrane. The number average value of these opening ratios $C_0$ is defined as an opening ratio $C_0$' of the inner surface of the hollow fiber membrane.
In the related art, it has been known that the deterioration of water permeation performance due to clogging is suppressed by using a membrane having a high opening ratio for filtration (Pamphlet of International Publication No. 2001/053213). However, as a result of intensive studies, the present inventors have confirmed that a porous membrane having a lower opening ratio exhibits excellent filtration performance in a porous membrane that is used for tangential flow filtration in perfusion culture. While not being bound by theory, this is conceived to be because in tangential flow filtration in the perfusion culture, the lower the opening ratio, the membrane clogging-causing substance is less likely to enter the inside of the membrane from the primary side, and the membrane clogging-causing substance is retained on the primary side. Therefore, there is a tendency that the smaller the opening ratio of the liquid contact surface is, the less likely to occur the clogging of the membrane pores due to the deposition of the removed substances on the membrane surface.

[Method for calculating fiber diameter F' of liquid contact surface of porous membrane]

Using a filters.local_thickness function of a library porespy (2.0.2) of python (3.7.10), which is described in Reference Literature 1 (Gostick et al., (2019). PoreSpy: A Python Toolkit for Quantitative Analysis of Porous Media Images. Journal of Open Source Software, 4(37), 1296.), the first analysis target image is analyzed, where the first argument is set to the first analysis target image, and the second argument is set to mode = 'dt'. Since the filter.local_thickness function is a function that carries out analysis on white pixels in the first analysis target image, a fiber diameter (unit: pixel), which is the diameter of the resin skeleton, is calculated in a case where the first analysis target image has been analyzed. The diameter (unit: pixel) is multiplied by the pixel resolution (unit: m/pixel) to obtain a diameter (unit: m). A fiber diameter F in one SEM image can be indicated by Expression (2) by using the total number $D_0$ of white pixels and the total $E_0$ of all the obtained diameters.

$$F = E_0/D_0 \qquad (2)$$

Since each pixel is given a diameter, F is a weighted average of the fiber diameters in the SEM image. The fiber diameter F is calculated using SEM images from three or more fields of view (a total of 9 images) for each of three lots of one kind of hollow fiber membrane. The number average value of the fiber diameters F of all the SEM images is defined as the fiber diameter F' of the inner surface of the hollow fiber membrane.

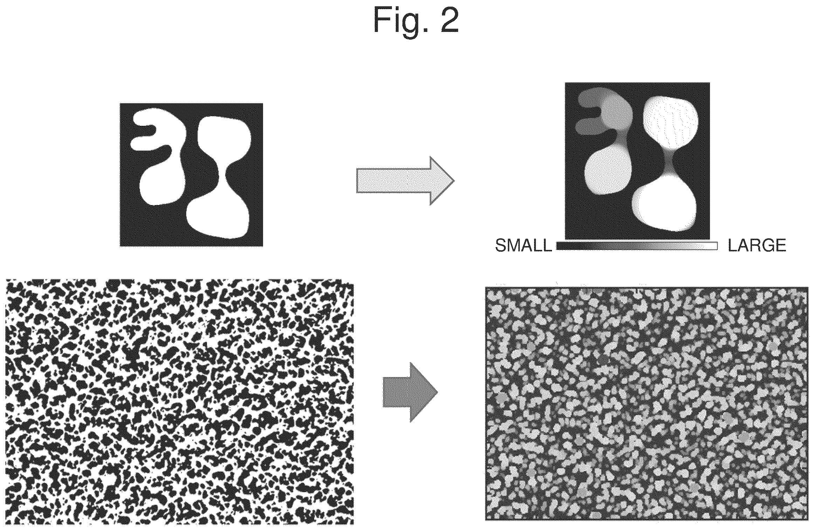

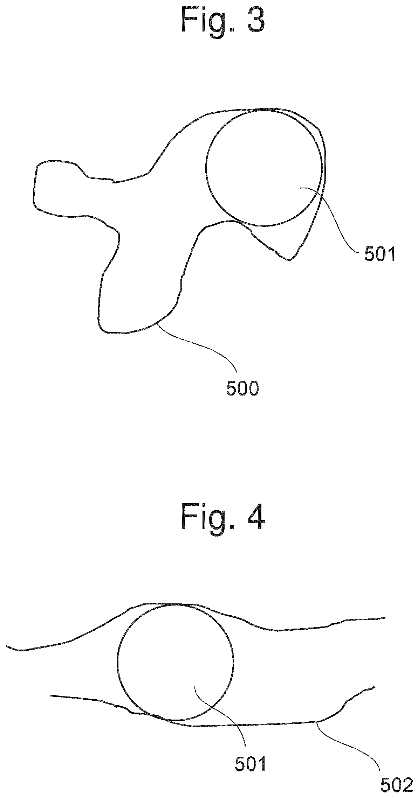

Here, in the local thickness method that is used in the filters.local_thickness function, as illustrated in Figure 2, the diameter is determined by filling a region surrounded by a contour with circles that enter the region, in descending order in terms of circle size. Therefore, as illustrated in Figure 3, a region surrounded by a contour 500 into which a circle 501 is placed does not need to be a circle. In addition, as illustrated in Figure 4, a region surrounded by a contour 502 does not need to be a closed region that is completely surrounded by line segments. It is also possible to calculate an effective diameter to be used in filtration, the effective diameter for fibers or membrane pores having various shapes on the surface of the porous membrane. The same applies to a method using a filters.local_thickness function in the following description.

[Method for calculating pore diameter I' of liquid contact surface of porous membrane]

A second analysis target image that is an image, in which black and white in the first analysis target image are reversed such that pixels that are white in the first analysis target image are black and pixels that are black in the first analysis target image are white, is obtained. Using a filters.local_thickness function of a library porespy (2.0.2) of python (3.7.10), which is described in Reference Literature 1, analysis is carried out, where the first argument is set to the second analysis target image, and the second argument is set to mode = 'dt'. Since the filter.local_thickness function is a function that carries out analysis on white pixels in the second analysis target image, a pore diameter (unit: pixel) is calculated in a case where the second analysis target image has been analyzed. The diameter (unit: pixel) is multiplied by the pixel resolution (unit: m/pixel) to obtain a diameter (unit: m). A pore diameter I in one SEM image can be indicated by Expression (3) by using the total number G of white pixels and the total H of all the obtained diameters. Since each pixel is given a diameter, the pore diameter I is a weighted average of the pore diameters in the SEM image.

$$I = H/G \quad (3)$$

A pore diameter I is calculated using SEM images from three or more fields of view (a total of 9 images) for each of three lots of one kind of hollow fiber membrane. The number average value of the pore diameters I of all the SEM images is defined as the pore diameter I' of the inner surface of the hollow fiber membrane.

[Method for calculating coefficient of variation V of pore diameter of liquid contact surface of porous membrane]

A coefficient of variation V of the pore diameter I is given according to Expression (4), where a standard deviation of the pore diameters I of all the SEM images is denoted as **S.**

$$V = S/I' \quad (4)$$

A large coefficient of variation of the pore diameter means a large variation of the pore diameter. In a case where the variation of the pore diameter is large, it is considered that, in a cell broth in which impurities of various sizes and various states are present, impurities are likely to be caught on the surface of the porous membrane and are likely to be a scaffold in the deposition of impurities onto the surface of the porous membrane. Therefore, there is a tendency that the smaller the coefficient of variation of the pore diameter of the liquid contact surface is, the less likely to occur the clogging of the membrane pores due to the deposition of the removed substances on the membrane surface.

[Calculation of ratio J' of pore diameter/fiber diameter of liquid contact surface of porous membrane]

A ratio J of pore diameter/fiber diameter is given according to the following Expression (5) by using the pore diameter I and the fiber diameter F in each SEM image.

$$J = I/F \quad (5)$$

The ratio J of pore diameter/fiber diameter is calculated using SEM images from three or more fields of view (a total of 9 images) for each of three lots of one kind of hollow fiber membrane. The number average value of the ratio J of pore diameter/fiber diameter in all of the SEM images is defined as a ratio J' of pore diameter/fiber diameter of the inner surface of the hollow fiber membrane. The ratio of the pore diameter to the fiber diameter (ratio of pore diameter/fiber diameter) on the liquid contact surface of the porous membrane is a value obtained by normalizing the effective pore diameter for filtration using the fiber diameter.

It is considered that the smaller the pore diameter normalized by the fiber diameter is, the membrane clogging-causing substance is less likely to enter the inside of the membrane from the primary side, and the membrane clogging-causing substance is retained on the primary side. Therefore, there is a tendency that the smaller the ratio of pore diameter/fiber diameter of the liquid contact surface is, the less likely to occur the clogging of the membrane pores due to the deposition of the removed substances on the membrane surface.

[Calculation of ratio **M''** of long pore diameter/short pore diameter of pore present on liquid contact surface of porous membrane]

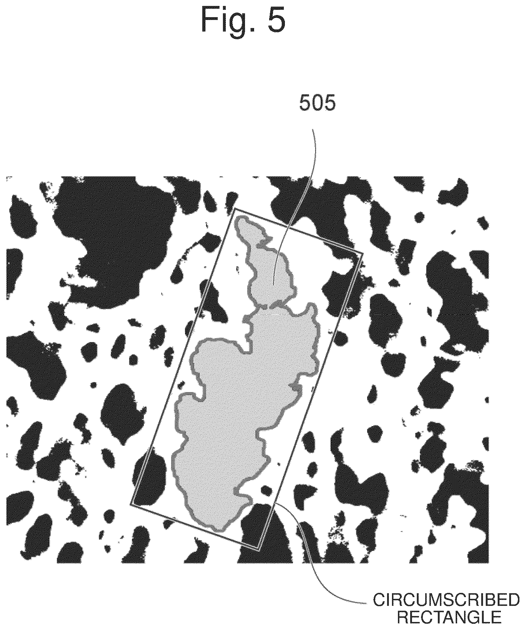

Independent white regions are extracted in the second analysis target image. Contour detection of each region is carried out for each of the extracted white regions, by extracting white pixels of which adjacent pixels in any of the up, down, left, and right directions are black. As illustrated in Figure 5, among the circumscribed rectangles that are drawn even in consideration of rotation with respect to the contour, a rectangle having the smallest area is set as a circumscribed rectangle. M indicating the ratio of long diameter/short diameter in a certain contour is given according to the following Expression (6), where a length of a short side of four sides of the circumscribed rectangle is denoted as a short diameter K, and a length of a long side thereof is denoted as a long diameter L.

$$M = L/K \quad (6)$$

M (long diameter/short diameter) is calculated for all the contours included in one SEM image, and the number average value thereof is defined as M'. M' is calculated using SEM images from three or more fields of view (a total of 9 images) for each of three lots of one kind of hollow fiber membrane. The number average value of M' in all the SEM images is defined as an average value M'' of the ratio of long pore diameter/short pore diameter of pores present on the inner surface of the hollow fiber membrane. The ratio (long diameter/short diameter) of the long diameter to the short diameter of the pores on the liquid contact surface of the porous membrane is an indicator of anisotropy of the pores on the liquid contact surface.

[Measurement of structural anisotropy Z' of liquid contact surface of porous membrane]

An analysis target image $O_I$ having a size of $512 \times 512$ pixels and having a left upper end vertex as one end, an analysis target image $P_I$ having the same size as $O_I$ and having a left lower end vertex as one end, an analysis target image $Q_I$ having the same size as $O_I$ and having a right upper end vertex as one end, and an analysis target image $R_I$ having the same size as $O_I$ and having a right lower end vertex as one end are extracted in the first analysis target image. The analysis target image $O_I$ is subjected to masking processing in which an outside of a circle having a diameter of 512 pixels is set to black, where the circle has the same center as $O_I$, whereby a third analysis target image for FFT analysis is obtained. After converting the third analysis target image into a brightness matrix, a two-dimensional fast Fourier transform is carried out using an fft.fft2 function of a Numpy (1.20.3) library of python (3.7.10), whereby a matrix $T_s$ is obtained. However, the first argument of the fft.fft2 function is set to the third analysis target image, and the default settings are used for the other arguments. Thereafter, a matrix T, in which the matrix $T_s$ has been rearranged so that the low frequency components are at the center using the fft.fft shift function of Numpy (1.20.3), is obtained. However, the first argument of the fft.fft shift function is set to the matrix $T_s$, and the default settings are used for the other arguments. Thereafter, a power spectrum U is obtained according to the following Expression (7).

$$U = 20 \times \log(T) \quad (7)$$

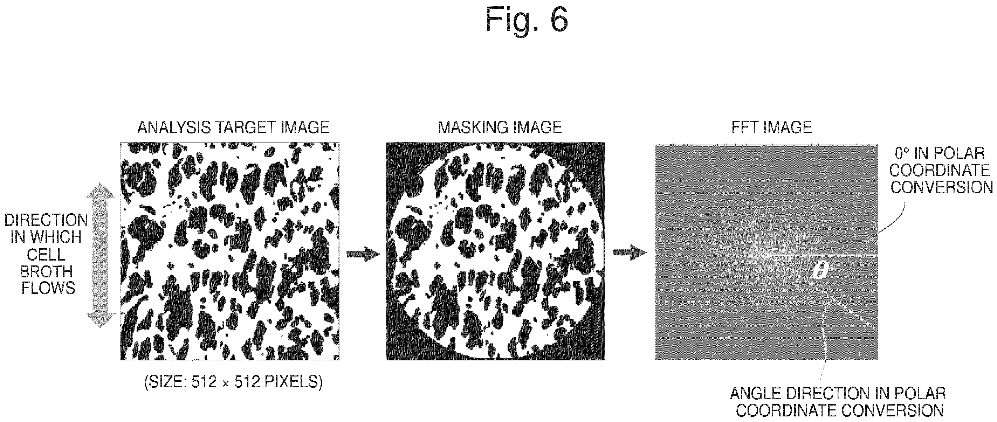

The power spectrum U to be obtained is subjected to polar coordinate conversion using a warpPolar function of a library OpenCV (4.5.2) of python (3.7.10) to obtain a $360 \times 360$ matrix $T_V$ in which the center of the power spectrum is the origin. However, the lateral direction of the image illustrated in Figure 6 is set to 0° in the polar coordinate, and polar coordinate conversion is carried out in the $\theta$ direction. Thereafter, a matrix $T_W$, which indicates the intensity average for each row of the matrix $T_V$, is obtained. The matrix $T_W$ is a matrix of one column and corresponds to the intensity average for each angle. In addition, the intensity average is a number average value. All the elements of the matrix $T_W$ are added up, and the sum thereof is divided by the number of elements to obtain an element average of the matrix $T_W$. The matrix $T_W$ is divided by the element average to obtain a matrix $T_Y$.

In a case where an element in an n-th row of the matrix $T_Y$ is denoted as $Y_n$, where n is any natural number, an intensity average $X_{180}$ in the vicinity of 180° in the power spectrum is given according to the following Expression (8).

$$X_{180} = (Y_{178} + Y_{179} + Y_{180} + Y_{181} + Y_{182})/5 \quad (8)$$

In addition, an intensity average $X_{90}$ in the vicinity of 90° in the power spectrum is given according to the following Expression (9).

$$X_{90} = (Y_{88} + Y_{89} + Y_{90} + Y_{91} + Y_{92})/5 \quad (9)$$

Using these, a structural anisotropy $Z_O$ of the analysis target image $O_I$ is given according to the following Expression (10) .

$$Z_O = X_{90}/X_{180} \quad (10)$$

In a case where the structural anisotropies obtained by analyzing the analysis target images $P_I$, $Q_I$, and $R_I$ by the same method as described above are denoted as $Z_P$, $Z_Q$, and $Z_R$, respectively, a structural anisotropy Z in one SEM image is given according to Expression (11).

$$Z = (Z_O + Z_P + Z_Q + Z_R)/4 \quad (11)$$

The structural anisotropy Z is calculated using SEM images from three or more fields of view (a total of 9 images) for each of three lots of one kind of hollow fiber membrane. The number average value of the structural anisotropies Z in all of these SEM images is defined as the structural anisotropy Z' of the liquid contact surface of the hollow fiber membrane. In a case where the structural anisotropy Z' of the surface of the porous membrane is 1, it indicates that the pores are uniformly aligned in the vertical axis direction and the horizontal axis direction of the image, and the anisotropy of the membrane structure is small. In a case where the structural anisotropy Z' is more than 1, it indicates that the membrane structure is aligned in the horizontal axis direction of the image. In a case where the structural anisotropy Z' is less than 1, it indicates that the membrane structure is aligned in the vertical axis direction of the image.

[Method for calculating ratio of maximum value/minimum value of average pore diameter of porous membrane in membrane thickness direction]

(Preparation of cross section)

A hollow fiber membrane is fixed to a specimen support with a carbon paste, and then a cross section in the membrane thickness direction is prepared under the following conditions for preparing cross section with an ion milling device E-3500 (manufactured by Hitachi High-Tech Corporation). The cross section is a smooth cross section such that an Ar ion beam penetrates from the outer surface to the inner surface of the hollow fiber membrane, there is no deformation of the resin skeleton due to heat, and there is no adhered specimen piece that has been sputtered. It is noted that even in a case where the porous membrane is a membrane other than the hollow fiber membrane, a cross section in the membrane thickness direction needs only to be prepared in the same manner.

(Conditions for preparing cross section)

Acceleration voltage: 3 kV
Irradiation current: 20 $\mu$A

(Cross-sectional SEM observation)

Using an osmium coater (HPC-30W, manufactured by Vacuum Device Inc.), the prepared specimen is subjected to osmium coating under conditions of an applied voltage adjustment knob setting of 4.5 and a discharge time of 3 seconds, whereby an observation specimen is obtained. The observation specimen is irradiated with an electron beam under the following observation conditions, and the contrast is adjusted by an auto function in a field of view consisting of only a skeleton resin and pores. In addition, a magnification at which the average pore diameter is 10 to 20 pixels at a position where the average pore diameter is the smallest in the cross section is defined as the imaging magnification. For example, the cross-sectional imaging magnification of the first example and the second example of the porous membrane described later is set to 5,000 times, and the cross-sectional imaging magnification of the hollow fiber membrane used in the third example of the porous membrane is set to 3,000 times. Under the conditions described above, the outer surface is allowed to coincide with a right side of the SEM image to obtain a first backscattered electron image in which the membrane thickness direction of the hollow fiber membrane and the lateral direction of the SEM image are parallel. It is noted that the backscattered electron image is an image in which a smooth cross section is focused and is an image in which there is no distortion of the structure or abnormal contrast due to charge-up. It is noted that even in a case where the porous membrane is a membrane other than the hollow fiber membrane, a cross section in the membrane thickness direction needs only to be imaged in the same manner.

[Observation conditions]

SEM: scanning electron microscope SU7000 manufactured by Hitachi High-Tech Corporation
Probe current: Normal
Acceleration voltage: 1 kV
Operating distance: 3 mm
Detector: MD detector (for backscattered electron image)
Image size: 960 pixels in length $\times$ 1280 pixels in width

Next, the field of view of the first backscattered electron image is shifted to the inner surface side in parallel with the membrane thickness direction to obtain a second backscattered electron image. However, the fields of view are slightly overlapped so that the pore structure in the second backscattered electron image and the pore structure in the first backscattered electron image are smoothly connected. A step of acquiring the backscattered electron image is continued by slightly overlapping the previous field of view while shifting the field of view in the membrane thickness direction, and the imaging is terminated at a time point at which the inner surface is included in the backscattered electron image.

A label (text or scale bar portion) part of the obtained backscattered electron image is removed by trimming, and all the images are combined such that the pore structures are smoothly connected to each other, thereby obtaining a single cross-sectional SEM image. However, the cross-sectional image is rectangular, and in a case where there is a deviation of the SEM image in the vertical direction during the connection, the deviation is removed by trimming after the combination. The purpose is to acquire a high-resolution cross section in a region that can be sufficiently approximated to a rectangle, since the cross section of the hollow fiber membrane is intrinsically circular.

The cross-sectional SEM image is trimmed such that the left end is an inner surface and the right end is an outer surface, thereby obtaining a first cross-sectional SEM image. The first cross-sectional SEM image has the same pixel resolution as the first backscattered electron image. In a case where a resin skeleton present in the pore in the depth direction is observed to have a brightness brighter than the brightness of the cross-sectional resin, a resin skeleton in the depth direction may be also subjected to hand painting with a dark color in the binarization step so that the resin skeleton is also recognized as a part of the pore.

(Analysis of cross-sectional pore diameter)

The pore diameter of the cross section is analyzed by the following method. In the first cross-sectional SEM image, a first region having a width of 100 pixels including one side of the left end is extracted. Hereinafter, the height of all the regions to be extracted is assumed to be equal to the height of the first cross-sectional SEM image. A maximum diameter of a plurality of pores included in the first region, a coefficient of variation of pore diameters, an average pore diameter, and an opening ratio are calculated. The first region is subjected to filtering processing with a median filter using a $5 \times 5$ square kernel, and then two threshold values which are not zero are determined according to the multi-Otsu method. Among the threshold values, the larger value is used as the threshold value, and the binarization of the image, in which pixels equal to or smaller than the threshold value are set to white and pixels other than these pixels are set to black, is carried out. However, the brightness of white is 255, and the brightness of black is 0. The binarized image is subjected to one time of closing processing using a $2 \times 2$ square kernel to remove noise. Using a filters.local_thickness function of a library porespy (2.0.2) of python (3.7.10), the obtained analysis target image is analyzed, where the first argument is set to the analysis target image, and the second argument is set to mode = 'dt'. Since the filters.local_thickness function is a function that carries out analysis on white pixels in the analysis target image, a pore diameter (unit: pixel) is calculated. The pore diameter (unit: pixel) is multiplied by the pixel resolution (unit: m/pixel) to obtain a pore diameter (unit: m). Although the pore diameter is given to all pixels, the maximum pore diameter among the pore diameters is defined as the maximum pore diameter $I_{max\,(1)}$ of the first region.

The coefficient of variation of the pore diameter in the pore diameter distribution in the analysis target image is defined as a coefficient of variation $V_1$ of the first region. Further, an average pore diameter $I_1$ of the extracted region is given according to the following Expression (12) by using the total number $D_1$ of white pixels and the total $E_1$ of pore diameters given to all the pixels.

$$I_1 = E_1/D_1 \quad (12)$$

In addition, the opening ratio $C_1$ (%) is given according to the following Expression (13) by using the total number of pixels $A_1$ in the first region.

$$C_1 = D_1 \times 100/A_1 \quad (13)$$

In a case where the coefficient of variation $V_1$ of the pore diameter in the first region is within a predetermined range of being more than 0.3 and less than 0.5, the center of the first region having a width of 100 pixels in the vertical direction with respect to one side of the left end is set as the center of the first region. In a case where the coefficient of variation $V_1$ of the pore diameter is larger than a value in a predetermined range, the width $W_1$ of the first region is narrowed according to the following Expression (14) until the coefficient of variation $V_1$ of the pore diameter is within a predetermined range, and then the calculations of the maximum pore diameter $I_{max\,(1)}$, the coefficient of variation $V_1$, the average pore diameter $I_1$, and the opening ratio $C_1$ are carried out again. It is noted that the center of the width $W_1$ of the first region is set as the center of the first region. In Expression (14), $N_1$ indicates the number of times of the operation in which a region is re-taken until the region is within a predetermined range.

$$W_1 = (10 - 0.1 \times N_1) \times I_{max\,(1)} \quad (14)$$

In a case where the coefficient of variation $V_1$ of the pore diameter is smaller than a value in a predetermined range, the width $W_1$ of the first region is widened according to Expression (15) until the coefficient of variation $V_1$ of the pore diameter is within a predetermined range, and then the calculations of the maximum pore diameter $I_{max(1)}$, the coefficient of variation $V_1$, the average pore diameter $I_1$, and the opening ratio $C_1$ are carried out again. It is noted that the center of the width $W_1$ of the first region is set as the center of the first region. In Expression (15), $N_1$ indicates the number of times of the operation in which a region is re-taken until the region is within a predetermined range.

$$W_1 = (10 + 0.1 \times N_1) \times I_{max\,(1)} \quad (15)$$

The coefficient of variation $V_1$ is allowed to satisfy a predetermined range, and the finally determined average pore diameter $I_1$ and the opening ratio $C_1$ are recorded in association with the center coordinates of the first region.

Thereafter, in the vertical direction with respect to one side of the left end of the first cross-sectional SEM image, the center of the (n + 2)-th region separated by 100 pixels from the center of the (n + 1)-th region is set, where n is an integer, which has an initial value of 0. The width $W_{n+2}$ of the (n + 2)-th region is determined according to Expression (16) by using the maximum value $I_{max\,(n+1)}$ of the pore diameter included in the (n + 1)-th region.

$$W_{n+2} = 10 \times I_{max\,(n+1)} \quad (16)$$

A maximum diameter and a coefficient of variation of pore diameters of a plurality of contours included in the (n + 2)-th region are calculated. The (n + 2)-th region is subjected to filtering processing with a median filter using a $5 \times 5$ square kernel, and then two threshold values which are not zero are determined according to the multi-Otsu method. Among the threshold values, the larger value is used as the threshold value, and the binarization of the image, in which pixels equal to or smaller than the threshold value are set to white and pixels other than these pixels are set to black, is carried out.

However, the brightness of white is 255, and the brightness of black is 0. The binarized image is subjected to one time of closing processing using a $2 \times 2$ square kernel to remove noise. Using a filters.local_thickness function of a library porespy (2.0.2) of python (3.7.10), the obtained analysis target image is analyzed, where the first argument is set to the analysis target image, and the second argument is set to mode = 'dt'. Since the filters.local_thickness function is a function that carries out analysis on white pixels in the analysis target image, a pore diameter (unit: pixel) is calculated. The pore diameter (unit: pixel) is multiplied by the pixel resolution (unit: m/pixel) to obtain a pore diameter (unit: m). Although the pore diameter is given to all pixels, the maximum pore diameter among the pore diameters is defined as the maximum pore diameter $I_{max\,(n+2)}$ of the (n + 2)-th region.

The coefficient of variation of the pore diameter in the pore diameter distribution in the analysis target image is defined as a coefficient of variation $V_{n+2}$ of the (n + 2)-th region. Further, an average pore diameter $I_{n+2}$ of the (n + 2)-th region is given according to the following Expression (17) by using the total number $D_{n+2}$ of white pixels and the total $E_{n+2}$ of pore diameters given to all the pixels.

$$I_{n+2} = E_{n+2}/D_{n+2} \quad (17)$$

In addition, an opening ratio $C_{n+2}$ (%) is given according to the following Expression (18) by using the total number of white pixels $D_{n+2}$ and the total number of pixels $A_{n+2}$ in the (n + 2)-th region.

$$C_{n+2} = D_{n+2} \times 100/A_{n+2} \quad (18)$$

In a case where a coefficient of variation $V_{n+2}$ of pore diameter in the (n + 2)-th region is within a predetermined range of being more than 0.3 and less than 0.5, a width $W_{n+2}$ of the (n + 2)-th region in the vertical direction with respect to one side of the left end is maintained. In a case where the coefficient of variation $V_{n+2}$ of the pore diameter is larger than a value in a predetermined range, the width $V_{n+2}$ of the (n + 2)-th region is narrowed according to Expression (19) until the coefficient of variation $V_{n+2}$ of the diameter is within the above-described predetermined range while maintaining the center of the (n + 2)-th region, and then the calculations of the maximum pore diameter Imax $_{(n+2)}$, the coefficient of variation $V_{n+2}$, the average pore diameter $I_{n+2}$, and the opening ratio $C_{n+2}$ are carried out again. In Expression (19), $N_{n+2}$ indicates the number of times of the operation in which a region is re-taken until the region is within a predetermined range.

$$W_{n+2} = (10 - 0.1 \times N_{n+2}) \times I_{max\,(n+2)} \quad (19)$$

In a case where the coefficient of variation of the diameter is smaller than a value in a predetermined range, the width $W_{n+2}$ of the (n + 2)-th region is widened according to Expression (20) until the coefficient of variation of the diameter is within a predetermined range while maintaining the center of the (n + 2)-th region, and then the calculations of the maximum pore diameter $I_{max\,(n+2)}$, the coefficient of variation $V_{n+2}$, the average pore diameter $I_{n+2}$, and the opening ratio $C_{n+2}$ are carried out again. In Expression (20), $N_{n+2}$ indicates the number of times of the operation in which a region is re-taken until the region is within a predetermined range.

$$W_{n+2} = (10 + 0.1 \times N_{n+2}) \times I_{max\,(n+2)} \quad (20)$$

The coefficient of variation $V_{n+2}$ is allowed to satisfy a predetermined range, and the finally determined average pore diameter $I_{n+2}$ and the opening ratio $C_{n+2}$ are recorded in association with the center coordinates of the (n + 2)-th region.

Thereafter, the average pore diameter $I_{n+1}$ and the opening ratio $C_{n+1}$ of the (n + 1)-th region are calculated by adding up 1 to n, and this calculation is repeated until the center of the (n + 2)-th region exceeds one side of the right end of the first cross-sectional SEM image. Thereafter, a point where the corresponding opening ratio $C_{n+1}$ is 80% or more is excluded from the recorded average pore diameters $I_{n+1}$. In addition, in a case where a graph in which the average pore diameter $I_{n+1}$ is plotted with respect to the region center coordinates and the first cross-sectional SEM image are compared with each other and the values are clearly unnatural, such a point is deleted. R, which is a ratio (maximum value/minimum value) of the maximum value to the minimum value of the average pore diameter from the primary side surface to the secondary side surface of the porous membrane in the membrane thickness direction, is given according to the following Expression (21), where among the average pore diameters $I_{n+1}$ obtained by removing unnecessary points, the maximum value of the average pore diameter in the membrane thickness direction is defined as $I_{max}$ and the minimum value of the average pore diameter in the membrane thickness direction is defined as $I_{min}$.

$$R = I_{max}/I_{min} \quad (21)$$

R is calculated for three or more lots of one kind of hollow fiber membrane. The average value thereof is defined as the ratio R' of maximum value/minimum value of the average pore diameter of the porous membrane in the membrane thickness direction.

It indicates that as the ratio R' of maximum value/minimum value of the average pore diameter is to be larger than 1, the pore diameter of the porous membrane varies in the membrane thickness direction. It indicates that as the ratio R' of maximum value/minimum value of the average pore diameter is to be a value closer to 1, the pore diameter of the porous membrane does not vary in the membrane thickness direction.

The ratio R' of maximum value/minimum value of the average pore diameter of the porous membrane in the membrane thickness direction reflects the variation of the pore diameter of the porous membrane in the membrane thickness direction, and thus it has a strong correlation with a mechanism of progress of membrane clogging in the inside of the porous membrane. Therefore, while not being bound by theory, it is considered that there is a suitable combination for exhibiting excellent filtration performance, between the structural features of the liquid contact surface of the porous membrane and the filtration conditions.

[Blocking pore diameter of porous membrane]

The blocking pore diameter is exemplified as a particle diameter of a particle, at which a permeation blocking rate of particles is 90% in a case where a particle dispersion liquid in which particles having a constant particle diameter are dispersed is filtered using a porous hollow fiber membrane. The blocking pore diameter may also be referred to as a minimum pore diameter. In a case of determining the blocking pore diameter, polystyrene latex particles (manufactured by JSR Corporation, SIZE STANDARD PARTICLES) are dispersed in an aqueous solution of 0.5% by mass sodium dodecyl sulfate (manufactured by FUJIFILM Wako Pure Chemical Corporation) so that the particle concentration is 0.01% by mass, whereby a latex particle dispersion liquid is prepared. The latex particle dispersion liquid is filtered using a porous hollow fiber membrane, and a change in the concentration of the latex particles before and after the filtration is measured. This measurement is carried out while changing the latex particle diameter, whereby a blocking curve of the latex particles is created. From this blocking curve, a particle diameter at which 90% of permeation blocking can be achieved is read, and the diameter is defined as the blocking pore diameter.

[Membrane thickness of porous membrane]

The inner diameter ($\mu$m) and the outer diameter ($\mu$m) of the hollow fiber membrane can be measured by cutting the hollow fiber membrane into a thin tubular shape and observing the section with an optical microscope (manufactured by KEYENCE CORPORATION, VHX-7000). In addition, the membrane thickness $W_{TH}$ ($\mu$m) of the hollow fiber membrane can be calculated from an inner diameter $W_I$ and an outer diameter $W_O$ by using Expression (22).

$$W_{TH} = (W_O - W_I)/2 \qquad (22)$$

[Shear stress]

A shear stress SS due to a flow of the cell broth on the liquid contact surface of the porous membrane is given by a product by a viscosity VC (Pa·s) of a cell broth flowing through the hollow portion of the hollow fiber membrane and a shear rate SV (/s), as shown in the following Expression (23).

$$SS = VC \times SV \qquad (23)$$

The viscosity of the cell broth may be affected by, for example, the temperature of the cell broth, the composition of the cell broth, and the cell density in the cell broth. In particular, a polymer for anti-foaming which may be contained in the cell broth may affect the viscosity of the cell broth.

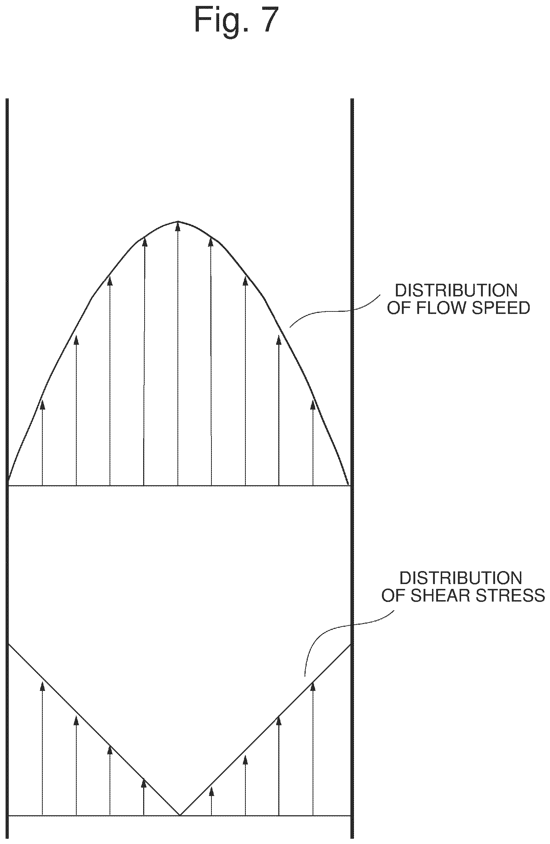

While not being bound by theory, under the laminar flow conditions of the Newtonian fluid, the flow of the cell broth in the hollow portion of the hollow fiber membrane is a Hagen-Poiseuille flow, and as illustrated in Figure 7, the distribution of the flow speed of the cell broth in the hollow portion of the hollow fiber membrane is a secondary curve with the center of the hollow portion of the hollow fiber membrane as an axis. The shear rate of the cell broth is the slope of the secondary curve. The shear rate SV of the cell broth has a maximum SVmax on the liquid contact surface of the hollow fiber membrane, and it is given according to the following Expression (24), where LV indicates the linear velocity (m/s) of the cell broth, and $W_I$ indicates the inner diameter (m) of the hollow portion of the hollow fiber membrane. As illustrated in Figure 7, The shear stress also has a maximum SSmax on the liquid contact surface of the hollow fiber membrane.

$$SVmax = 8LV/W_I \qquad (24)$$

It is noted that, in the present embodiment, the shear stress is calculated from Expression (23) described above even in a case where the flow of the cell broth is a turbulent flow under conditions other than the laminar flow conditions of the Newtonian fluid.

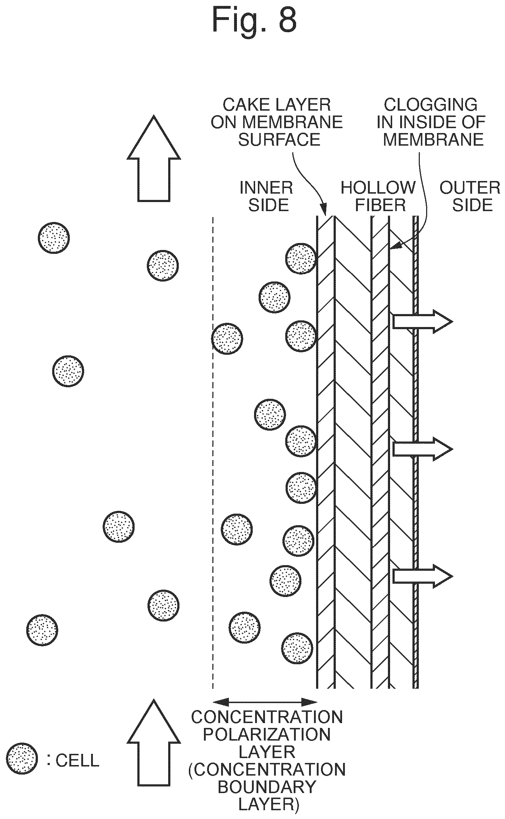

In a case where a cell broth containing cells flows through the hollow portion of the hollow fiber membrane, a concentration polarization layer having a high cell density is formed on the inner surface (primary side surface) of the hollow fiber membrane, as illustrated in Figure **8. In** addition, cells are deposited on the inner surface (primary side surface) of the hollow fiber membrane, and a cake layer is formed. The concentration polarization layer and the cake layer cause a decrease in the permeation rate of the product by cells, which is not associated with a decrease in the flow rate of the permeated solution of the hollow fiber membrane. In addition, in a case where the inside of the hollow fiber membrane is clogged by a substance contained in the cell broth, the flow rate of the permeated solution of the hollow fiber membrane is decreased, and the permeation rate of the product by cells is decreased. Since the product by cells such as an antibody is extremely expensive, even a slight decrease in permeation rate has a significant effect on the cost of the biopharmaceutical product.

[Permeation rate in porous membrane]

The permeation rate P (%) of the product by cells is given according to the following Expression (25), where the concentration of the product by cells in the cell broth before being filtered through the porous membrane is denoted as Q1, and the concentration of the product by cells in the cell broth after being filtered through the porous membrane is denoted as Q2.

$$P = Q2 \times 100/Q1 \qquad (25)$$

[Transmembrane pressure in porous membrane]

The clogging in the inside of the hollow fiber membrane can be evaluated with a transmembrane pressure. A transmembrane pressure Om in the hollow fiber membrane is given according to the following Expression (26), where a pressure of a cell broth supplied to the primary side of the hollow fiber membrane is denoted as O1i, a pressure of a cell broth that has passed through the primary side of the hollow fiber membrane without being filtered through the hollow fiber membrane is denoted as O1o, and a pressure of a cell broth that is filtered through the hollow fiber membrane and discharged from the secondary side is denoted as O2.

$$Om = (O1i + O1o)/2 - O2 \qquad (26)$$

The higher the degree of clogging in the inside of the hollow fiber membrane, the higher the transmembrane pressure.

[First example of porous membrane]

In the first example of the porous membrane, the opening ratio on the liquid contact surface is, for example, 57.5% or less, 55% or less, or 52.5% or less. In addition, the opening ratio is, for example, 1% or more, 5% or more, or 10% or more. In addition, the opening ratio is, for example, 1% or more and 57.5% or less, 5% or more and 57.5% or less, 10% or more and 57.5% or less, 1% or more and 55% or less, 5% or more and 55% or less, 10% or more and 55% or less, 1% or more and 52.5% or less, 5% or more and 52.5% or less, or 10% or more and 52.5% or less. In the first example of the porous membrane, there is a tendency that the smaller the opening ratio is, the less likely to occur the clogging of the membrane pores due to the deposition of the removed substances on the membrane surface.
In the first example of the porous membrane, the structural anisotropy of the liquid contact surface is, for example, 0.97 or more, 0.975 or more, 0.98 or more, 0.985 or more, or 0.99 or more. In addition, the structural anisotropy of the liquid contact surface is, for example, 1.03 or less, 1.025 or less, 1.02 or less, 1.015 or less, or 1.01 or less. In addition, the structural anisotropy of the liquid contact surface is, for example, 0.97 or more and 1.03 or less, 0.975 or more and 1.025 or less, 0.98 or more and 1.02 or less, 0.985 or more and 1.015 or less, or 0.99 or more and 1.01 or less. In the first example of the porous membrane, there is a tendency that the smaller the anisotropy of the liquid contact surface is, the less likely to occur the clogging of the membrane pores due to the deposition of the removed substances on the membrane surface.
In the first example of the porous membrane, the ratio of long pore diameter/short pore diameter on the liquid contact surface is, for example, 1 or more or 1.05 or more. In addition, the ratio of long pore diameter/short pore diameter is, for example, 1.8 or less, 1.75 or less, 1.7 or less, or 1.65 or less. In addition, the ratio of long pore diameter/short pore diameter is, for example, 1 or more and 1.8 or less, 1 or more and 1.75 or less, 1 or more and 1.7 or less, 1 or more and 1.65 or less, or 1.05 or more and 1.65 or less. In the first example of the porous membrane, there is a tendency that the smaller the anisotropy of pores on the liquid contact surface is, the less likely to occur the clogging of the membrane pores due to the deposition of the removed substances on the membrane surface.
In the first example of the porous membrane, the ratio of pore diameter/fiber diameter on the liquid contact surface is, for example, 0.05 or more, 0.1 or more, 0.2 or more, 0.3 or more, 0.4 or more, or 0.5 or more. The ratio of pore diameter/fiber diameter on the liquid contact surface is, for example, 1.3 or less, 1.25 or less, 1.2 or less, 1.15 or less, or 1.1 or less. The ratio of pore diameter/fiber diameter on the liquid contact surface for example, 0.05 or more and 1.3 or less, 0.1 or more and 1.3 or less, 0.2 or more and 1.3 or less, 0.3 or more and 1.3 or less, 0.4 or more and 1.25 or less, 0.4 or more and 1.2 or less, or 0.5 or more and 1.1 or less. In the first example of the porous membrane, there is a tendency that the smaller the ratio of pore diameter/fiber diameter is, the less likely to occur the clogging of the membrane pores due to the deposition of the removed substances on the membrane surface.
In the first example of the porous membrane, the coefficient of variation of the pore diameter on the liquid contact surface is 0 or more, 0.01 or more, 0.03 or more, or 0.05 or more. In addition, the coefficient of variation of the pore diameter is 0.5 or less, 0.49 or less, 0.48 or less, or 0.47 or less. In addition, the coefficient of variation of the pore diameter is 0 or more and 0.5 or less, 0.01 or more and 0.5 or less, 0.03 or more and 0.5 or less, 0.05 or more and 0.5 or less, 0.01 or more and 0.49 or less, 0.03 or more and 0.48 or less, or 0.05 or more and 0.47 or less. In the first example of the porous membrane, there is a tendency that the smaller the variation of the pore diameter on the liquid contact surface is, the less likely to occur the clogging of the membrane pores due to the deposition of the removed substances on the membrane surface.

In the first example of the porous membrane, a ratio (maximum value/minimum value) of a maximum value to a minimum value in a case where an average pore diameter of portions, which have the same distance from the membrane surface of the porous membrane in a membrane thickness direction, has been calculated, is 3 or more, 5 or more, or 10 or more. In addition, the ratio of maximum value/minimum value of the average pore diameter is 1,000 or less, 500 or less, or 100 or less. In addition, the ratio of maximum value/minimum value of the average pore diameter is 3 or more and 1,000 or less, 5 or more and 1,000 or less, 10 or more and 1,000 or less, 3 or more and 500 or less, 5 or more and 500 or less, 10 or more and 500 or less, 3 or more and 100 or less, 5 or more and 100 or less, or 10 or more and 100 or less. It indicates that as the ratio of maximum value/minimum value of the average pore diameter is to be larger than 1, the pore diameter of the porous membrane varies in the membrane thickness direction.

In the first example of the porous membrane, the porous membrane may be a hollow fiber membrane. The porous structure of the hollow fiber membrane may have an inclined structure in which the average pore diameter decreases from the primary side toward the secondary side in the membrane thickness direction. In the hollow fiber membrane having an inclined structure, the average pore diameter of the primary side surface is larger than the average pore diameter of the secondary side surface, and the pore diameter decreases from the primary side surface toward a minimum pore diameter layer. It is noted that there may be a portion in which the pore diameter does not change between the primary side surface and the secondary side surface. In the hollow fiber membrane having an inclined structure, the distribution of the pore diameter is asymmetric in the membrane thickness direction. A layer having a relatively large pore diameter in the vicinity of the primary side surface of the hollow fiber membrane is referred to as a coarse layer. A layer having a relatively small pore diameter in the vicinity of the secondary side surface of the hollow fiber membrane is referred to as a dense layer. A minimum pore diameter layer, in which the pore diameter is minimum, is included in the dense layer.

In the first example of the porous membrane, the average pore diameter of the primary side surface in the hollow fiber membrane having an inclined structure is, for example, 1 $\mu$m or more, 5 $\mu$m or more, 10 $\mu$m or more, or 15 $\mu$m or more. In addition, the average pore diameter of the primary side surface is, for example, 140 $\mu$m or less, 120 $\mu$m or less, 100 $\mu$m or less, or 80 $\mu$m or less. The average pore diameter of the primary side surface is, for example, 1 $\mu$m or more and 140 $\mu$m or less, 5 $\mu$m or more and 120 $\mu$m or less, 5 $\mu$m or more and 100 $\mu$m or less, 10 $\mu$m or more and 100 $\mu$m or less, 15 $\mu$m or more and 100 $\mu$m or less, or 15 $\mu$m or more and 80 $\mu$m or less. In a case where the pore diameter of the primary side surface is 1 $\mu$m or more, there is a tendency that the effect of depth filtration in which the removed substances are retained inside the membrane is easily obtained, and there is a tendency that the clogging of the membrane pores due to the deposition of the removed substances on the membrane surface is less likely to occur. In addition, in a case where the pore diameter of the primary side surface is 140 $\mu$m or less, the strength of the hollow fiber membrane tends to be easily maintained.

In the first example of the porous membrane, the average pore diameter of the secondary side surface in the hollow fiber membrane having an inclined structure is, for example, 0.1 $\mu$m or more, 0.2 $\mu$m or more, or 0.3 $\mu$m or more. In addition, the average pore diameter is, for example, 20 $\mu$m or less, 15 $\mu$m or less, 10 $\mu$m or less, 5 $\mu$m or less, or 2 $\mu$m or less. The average pore diameter of the secondary side surface is, for example, 0.1 $\mu$m or more and 20 $\mu$m or less, 0.1 $\mu$m or more and 15 $\mu$m or less, 0.1 $\mu$m or more and 10 $\mu$m or less, 0.1 $\mu$m or more and 5 $\mu$m or less, 0.1 $\mu$m or more and 2 $\mu$m or less, 0.2 $\mu$m or more and 15 $\mu$m or less, 0.3 $\mu$m or more and 10 $\mu$m or less, 0.3 $\mu$m or more and 5 $\mu$m or less, or 0.3 $\mu$m or more and 2 $\mu$m or less.

In the first example of the porous membrane, the blocking pore diameter of the hollow fiber membrane having an inclined structure is, for example, 0.05 $\mu$m or more, 0.1 $\mu$m or more, 0.2 $\mu$m or more, or 0.3 $\mu$m or more. In addition, the blocking pore diameter of the hollow fiber membrane having an inclined structure is, for example, 20 $\mu$m or less, 10 $\mu$m or less, 5 $\mu$m or less, 3 $\mu$m or less, 1 $\mu$m or less, 0.8 $\mu$m or less, or 0.5 $\mu$m or less. In addition, the blocking pore diameter of the hollow fiber membrane is, for example, 0.05 $\mu$m or more and 20 $\mu$m or less, 0.1 $\mu$m or more and 10 $\mu$m or less, 0.2 $\mu$m or more and 5 $\mu$m or less, 0.3 $\mu$m or more and 3 $\mu$m or less, 0.3 $\mu$m or more and 1 $\mu$m or less, 0.3 $\mu$m or more and 0.8 $\mu$m or less, or 0.3 $\mu$m or more and 0.5 $\mu$m or less. In a case where the blocking pore diameter is 0.05 $\mu$m or more, there is a tendency that the permeation resistance can be suppressed, the pressure required for filtration can be suppressed, and the membrane surface clogging due to the destruction and deformation of the microorganism particles, the decrease in the filtration efficiency, and the like can be suppressed. In addition, in a case where the blocking pore diameter is 20 $\mu$m or less, there is a tendency that sufficient fractionation properties are obtained.

In the first example of the porous membrane, the hollow fiber membrane having an inclined structure may include a minimum pore diameter layer having a minimum pore diameter, in the vicinity of the secondary side surface. The minimum pore diameter in the hollow fiber membrane is substantially the same as the blocking pore diameter.

In the first example of the porous membrane, the inner diameter of the hollow portion of the hollow fiber membrane is, for example, 1,000 μm or more, 1,100 μm or more, 1,200 μm or more, or 1,300 μm or more. In addition, the inner diameter of the hollow portion of the hollow fiber membrane is, for example, 3,000 μm or less, 2,500 μm or less, 2,000 μm or less, 1,900 μm or less, 1,800 μm or less, 1,700 μm or less, 1,600 μm or less, or 1,500 μm or less. In addition, the inner diameter of the hollow portion of the hollow fiber membrane is, for example, 1,000 μm or more and 3,000 μm or less, 1,000 μm or more and 2,500 μm or less, 1,000 μm or more and 2,000 μm or less, 1,100 μm or more and 1,900 μm or less, or 1,200 μm or more and 1,800 μm or less. In a case where the inner diameter of the hollow portion is 1,000 μm or more, the entrance of the hollow portion tends to be less likely to be clogged by the cells. In a case where the inner diameter of the hollow portion is 3,000 μm or less, the number of hollow fiber membranes constituting the filtration module is increased, the effective membrane area per filtration module is increased, and thus the filtration performance tends to be excellent.

In the first example of the porous membrane, the membrane thickness of the hollow fiber membrane is, for example, 100 μm or more, 200 μm or more, 300 μm or more, or 400 μm or more. In addition, the membrane thickness of the hollow fiber membrane is, for example, 1,400 μm or less, 1,300 μm or less, 1,200 μm or less, 1,100 μm or less, 1,000 μm or less, 900 μm or less, 800 μm or less, 700 μm or less, 600 μm or less, or 500 μm or less. In addition, the membrane thickness of the hollow fiber membrane is, for example, 100 μm or more and 1,400 μm or less, 200 μm or more and 1,200 μm or less, or 300 μm or more and 1,000 μm or less. In a case where the membrane thickness is 100 μm or more, the strength of the hollow fiber membrane tends to be easily maintained. Further, it tends to be easy to maintain a suitable filtration speed. In addition, in a case where the membrane thickness is 1,400 μm or less, the number of hollow fiber membranes constituting the filtration module is increased, the effective membrane area per filtration module is increased, and thus the filtration performance tends to be excellent.

In the first example of the porous membrane, the hollow fiber membrane consists of, for example, a synthetic polymer membrane. The synthetic polymer is, for example, hydrophobic. Examples of the synthetic polymer include polysulfone; however, examples thereof are not limited thereto. In general, impurities such as cells or debris (cell fragments) contained in a cell broth have a hydrophobic surface. The impurities are captured by the hollow fiber membrane by hydrophobic interaction. As a result, it is possible to purify a cell broth containing a product produced by cells. The hollow fiber membrane according to the embodiment can be manufactured, for example, with reference to the method described in Pamphlet of International Publication No. 2010/035793.

The present inventors have found that a plurality of structures of liquid contact surfaces of the first example of these porous membranes affects the filtration performance. In a case where the liquid contact surface of the porous membrane that is used for filtration has any of the structural feature quantities in the suitable range, the effect can be sufficiently exhibited even in a case where each of the plurality of structures of liquid contact surfaces is used alone. In addition, it is obvious that a synergistic effect is exhibited in a case where a plurality of structures of liquid contact surfaces is combined. For example, it is considered that the clogging of the membrane pores due to the deposition of the removed substances on the membrane surface proceeds at an accelerating rate with the deposits as a scaffold, in a case where the clogging once starts to proceed. Therefore, it is effective to use a porous membrane that has a liquid contact surface having a plurality of structural feature quantities in an appropriate range.

(Second example of porous membrane)

In the second example of the porous membrane, the opening ratio on the liquid contact surface of the porous membrane, the coefficient of variation of the pore diameter on the liquid contact surface, the ratio of pore diameter/fiber diameter on the liquid contact surface, the structural anisotropy of the liquid contact surface, and the ratio of long pore diameter/short pore diameter on the liquid contact surface are the same as those in the first example of the porous membrane. In the second example of the porous membrane, the porous membrane may be a hollow fiber membrane. The porous structure of the hollow fiber membrane may have a homogeneous structure that is substantially homogeneous from a primary side toward a secondary side in the membrane thickness direction The homogeneous structure that is substantially homogeneous refers to that the average pore diameter does not change significantly, and it refers to a structure of a membrane in which a ratio (maximum value/minimum value) of a maximum value to a minimum value is 10 or less, 5 or less, or 3 or less in a case where an average pore diameter of portions, which have the same distance from the membrane surface in a membrane thickness direction, has been calculated. The ratio of maximum value/minimum value of the average pore diameter may be 1 or more, 1.01 or more, or 1.05 or more. In addition, the ratio of maximum value/minimum value of the average pore diameter may be 1 or more and 10 or less, 1 or more and 5 or less, 1 or more and 3 or less, 1.01 or more and 10 or less, 1.01 or more and 5 or less, 1.01 or more and 3 or less, 1.05 or more and 10 or less, 1.05 or more and 5 or less, or 1.05 or more and 3 or less.

In the second example of the porous membrane, the average pore diameter of the primary side surface in the hollow fiber membrane having a homogeneous structure is, for example, 0.05 μm or more, 0.1 μm or more, or 0.15 μm or more. In addition, the average pore diameter of the primary side surface is, for example, 10 μm or less, 5 μm or less, 1 μm or less, 0.9 μm or less, 0.8 μm or less, 0.7 μm or less, 0.6 μm or less, 0.5 μm or less, or 0.4 μm or less. The average pore diameter of the primary side surface is, for example, 0.05 μm or more and 10 μm or less, 0.1 μm or more and 5 μm or less, 0.15 μm or more and 1 μm or less, 0.15 μm or more and 0.9 μm or less, 0.15 μm or more and 0.8 μm or less, 0.15 μm or more and 0.7 μm or less, 0.15 μm or more and 0.6 μm or less, 0.15 μm or more and 0.5 μm or less, or 0.15 μm or more and 0.4 μm or less. In a case where the pore diameter of the primary side surface is 0.05 μm or more and 10 μm or less, there is a tendency that an effect of screen filtration, in which the removed substances are not allowed to enter the inside of the membrane while maintaining the strength of the hollow fiber membrane, is easily obtained.

In the second example of the porous membrane, the blocking pore diameter of the hollow fiber membrane having a homogeneous structure is, for example, 0.05 μm or more, 0.1 μm or more, or 0.15 μm or more. In addition, the blocking pore diameter of the hollow fiber membrane having a homogeneous structure is, for example, 10 μm or less, 5 μm or less, 1 μm or less, 0.8 μm or less, 0.6 μm or less, 0.4 μm or less, 0.35 μm or less, 0.3 μm or less, or 0.25 μm or less. The blocking pore diameter of the hollow fiber membrane is, for example, 0.05 μm or more and 10 μm or less, 0.05 μm or more and 5 μm or less, 0.05 μm or more and 1 μm or less, 0.1 μm or more and 0.8 μm or less, 0.1 μm or more and 0.6 μm or less, 0.1 μm or more and 0.4 μm or less, 0.1 μm or more and 0.35 μm or less, 0.1 μm or more and 0.3 μm or less, 0.15 μm or more and 0.3 μm or less, or 0.15 μm or more and 0.25 μm or less. In a case where the blocking pore diameter is 0.05 μm or more, there is a tendency that the permeation resistance can be suppressed, the pressure required for filtration can be suppressed, and the membrane surface clogging due to the destruction and deformation of the microorganism particles, the decrease in the filtration efficiency, and the like can be suppressed. In addition, in a case where the blocking pore diameter is 10 μm or less, there is a tendency that sufficient fractionation properties are obtained.

In the second example of the porous membrane, the inner diameter of the hollow portion of the hollow fiber membrane is, for example, 1,000 μm or more, 1,100 μm or more, 1,200 μm or more, or 1,300 μm or more. In addition, the inner diameter of the hollow portion of the hollow fiber membrane is, for example, 3,000 μm or less, 2,500 μm or less, 2,000 μm or less, 1,900 μm or less, 1,800 μm or less, 1,700 μm or less, 1,600 μm or less, or 1,500 μm or less. In addition, the inner diameter of the hollow portion of the hollow fiber membrane is, for example, 1,000 μm or more and 3,000 μm or less, 1,000 μm or more and 2,500 μm or less, 1,000 μm or more and 2,000 μm or less, 1,100 μm or more and 1,900 μm or less, or 1,200 μm or more and 1,800 μm or less. In a case where the inner diameter of the hollow portion is 1,000 μm or more, the entrance of the hollow portion tends to be less likely to be clogged by the cells. In a case where the inner diameter of the hollow portion is 3,000 μm or less, the number of hollow fiber membranes constituting the filtration module is increased, the effective membrane area per filtration module is increased, and thus the filtration performance tends to be excellent.

In the second example of the porous membrane, the membrane thickness of the hollow fiber membrane is, for example, 100 μm or more, 200 μm or more, 300 μm or more, or 400 μm or more. In addition, the membrane thickness of the hollow fiber membrane is, for example, 1,400 μm or less, 1,300 μm or less, 1,200 μm or less, 1,100 μm or less, 1,000 μm or less, 900 μm or less, 800 μm or less, 700 μm or less, 600 μm or less, or 500 μm or less. In addition, the membrane thickness of the hollow fiber membrane is, for example, 100 μm or more and 1,400 μm or less, 200 μm or more and 1,200 μm or less, or 300 μm or more and 1,000 μm or less. In a case where the membrane thickness is 100 μm or more, the strength of the hollow fiber membrane tends to be easily maintained. Further, it tends to be easy to maintain a suitable filtration speed. In addition, in a case where the membrane thickness is 1,400 μm or less, the number of hollow fiber membranes constituting the filtration module is increased, the effective cross-sectional area per filtration module is increased, and thus the filtration performance tends to be excellent.

In the second example of the porous membrane, the hollow fiber membrane consists of, for example, a synthetic polymer membrane. The synthetic polymer is, for example, hydrophobic. Examples of the synthetic polymer include polyvinylidene fluoride; however, examples thereof are not limited thereto.

The present inventors have found that a plurality of structures of liquid contact surfaces of the second example of these porous membranes affects the filtration performance. In a case where the liquid contact surface of the porous membrane that is used for filtration has any of the structural feature quantities in the suitable range, the effect can be sufficiently exhibited even in a case where each of the plurality of structures of liquid contact surfaces is used alone. In addition, it is obvious that a synergistic effect is exhibited in a case where a plurality of structures of liquid contact surfaces is combined. For example, it is considered that the clogging of the membrane pores due to the deposition of the removed substances on the membrane surface proceeds at an accelerating rate with the deposits as a scaffold, in a case where the clogging once starts to proceed. Therefore, it is effective to use a porous membrane that has a liquid contact surface having a plurality of structural feature quantities in an appropriate range.

[Shear stress in case where porous membrane is first and second examples]

In both the first example and the second example of the porous membrane, a shear stress due to a flow of the cell broth on the liquid contact surface of the porous membrane 112 is set to 4.0 N/m² or less. The shear stress may be 3.75 N/m² or less, 3.5 N/m² or less, 3.25 N/m² or less, or 3.0 N/m² or less. The shear stress may be 0 N/m² or more, 0.01 N/m² or more, 0.05 N/m² or more, or 0.1 N/m² or more. The shear stress may be 0 N/m² or more and 4.0 N/m² or less, 0.01 N/m² or more and 4.0 N/m² or less, 0.05 N/m² or more and 4.0 N/m² or less, 0.1 N/m² or more and 4.0 N/m² or less, 0 N/m² or more and 3.75 N/m$^2$ or less, 0.01 N/m$^2$ or more and 3.75 N/m$^2$ or less, 0.05 N/m$^2$ or more and 3.75 N/m$^2$ or less, 0.1 N/m$^2$ or more and N/m$^2$ or less, 0 N/m$^2$ or more and 3.5 N/m$^2$ or less, 0.01 N/m$^2$ or more and 3.5 N/m$^2$ or less, 0.05 N/m$^2$ or more and N/m$^2$ or less, 0.1 N/m$^2$ or more and 3.5 N/m$^2$ or less, 0 N/m$^2$ or more and 3.25 N/m$^2$ or less, 0.01 N/m$^2$ or more and 3.25 N/m$^2$ or less, 0.05 N/m$^2$ or more and 3.25 N/m$^2$ or less, 0.1 N/m$^2$ or more and 3.25 N/m$^2$ or less, 0 N/m$^2$ or more and 3.0 N/m$^2$ or less, 0.01 N/m$^2$ or more and 3.0 N/m$^2$ or less, 0.05 N/m$^2$ or more and 3.0 N/m$^2$ or less, or 0.1 N/m$^2$ or more and 3.0 N/m$^2$ or less.

In a case where the porous membrane according to the first example and the second example is used, it is possible to suppress a decrease in the permeation rate of the product produced by cells in the porous membrane 112 by setting a shear stress due to the cell broth on the liquid contact surface of the porous membrane 112 to 4.0 N/m$^2$ or less.

In a case where the porous membrane according to the first example and the second example is used with reference to Figure 1, a flow speed of the cell broth to be filtered through the porous membrane 112, in a direction parallel to a membrane surface, may be controlled such that in a case of measuring the shear stress based on a viscosity of the cell broth, a measured shear stress is 4.0 N/m$^2$ or less. As described above, the shear stress SS, the viscosity VC of the cell broth, and the shear rate SV have a relationship of Expression (3) described above. For example, the viscosity VC of the cell broth can be acquired in advance. It is noted that the measurement may be carried out with a viscometer provided in the flow channel 113. The shear rate SV can be calculated from the linear velocity LV, and the linear velocity LV can be acquired, for example, from the output of the pump 123. It is noted that the linear velocity LV may be measured with a flowmeter provided in the flow channel 113. The shear stress SS of the cell broth can be measured based on the known viscosity VC of the cell broth and the acquired linear velocity LV or shear rate SV of the cell broth.

For example, the controller 301 is connected to a pump 123. In a case where the porous membrane according to the first example and the second example is used, the controller 301 may control the pump 123 to control the linear velocity of the cell broth flowing through the porous membrane 112 in order to control, in a direction parallel to a membrane surface, a flow speed of the cell broth to be filtered through the porous membrane 112 such that the shear stress is 4.0 N/m$^2$ or less.

In the embodiment in which the porous membrane according to the first example and the second example is used, the shear stress in the porous membrane 112 is set to 4.0 N/m$^2$ or less. However, the occurrence of a period in which the shear stress in the porous membrane 112 is 4.0 N/m$^2$ or more is not excluded. For example, due to a delay in the feedback control of the pump, a time in which the shear stress in the porous membrane 112 is 4.0 N/m$^2$ or more may occur. In a case where the porous membrane according to the first example and the second example is used, the shear stress in the porous membrane 112 may be set to 4.0 N/m$^2$ or less in a period of at least 80% of the entire period of culturing the cells.

[Cell density in case where porous membrane is first example]

In the first example of the porous membrane, the cell density contained in the cell broth that is filtered through the porous membrane 112 is not particularly limited; however, it may be $5 \times 10^7$ cells/mL or less. The cell density may be $4.5 \times 10^7$ cells/mL or less, $4 \times 10^7$ cells/mL or less, $3.5 \times 10^7$ cells/mL or less, $3 \times 10^7$ cells/mL or less, $2.5 \times 10^7$ cells/mL or less, $2 \times 10^7$ cells/mL or less, or $1.5 \times 10^7$ cells/mL or less.

In the first example of the porous membrane, the cell density contained in the cell broth that is filtered through the porous membrane 112 is not particularly limited; however, it may be $0.5 \times 10^6$ cells/mL or more. The cell density may be $1 \times 10^6$ cells/mL or more, $0.5 \times 10^7$ cells/mL or more, or $1 \times 10^7$ cells/mL or more.

In the first example of the porous membrane, the cell density contained in the cell broth that is filtered through the porous membrane 112 is not particularly limited; however, it may be $0.5 \times 10^6$ cells/mL or more and $5 \times 10^7$ cells/mL or less. The cell density may be $1 \times 10^6$ cells/mL or more and $5 \times 10^7$ cells/mL or less, $0.5 \times 10^7$ cells/mL or more and $5 \times 10^7$ cells/mL or less, $1 \times 10^7$ cells/mL or more and $5 \times 10^7$ cells/mL or less, $0.5 \times 10^6$ cells/mL or more and $4.5 \times 10^7$ cells/mL or less, $0.5 \times 10^6$ cells/mL or more and $4 \times 10^7$ cells/mL or less, $0.5 \times 10^6$ cells/mL or more and $3.5 \times 10^7$ cells/mL or less, $0.5 \times 10^6$ cells/mL or more and $3 \times 10^7$ cells/mL or less, $0.5 \times 10^6$ cells/mL or more and $2.5 \times 10^7$ cells/mL or less, $0.5 \times 10^6$ cells/mL or more and $2 \times 10^7$ cells/mL or less, $0.5 \times 10^6$ cells/mL or more and $1.5 \times 10^7$ cells/mL or less, $1 \times 10^7$ cells/mL or more and $3 \times 10^7$ cells/mL or less, $0.5 \times 10^7$ cells/mL or more and $3 \times 10^7$ cells/mL or less, or $1 \times 10^6$ cells/mL or more and $3 \times 10^7$ cells/mL or less.

[Cell density in case where porous membrane is second example]

In the second example of the porous membrane, the cell density contained in the cell broth that is filtered through the porous membrane 112 is not particularly limited; however, a cell broth having the following density is filtered: for example, $8 \times 10^7$ cells/mL or more, $8.5 \times 10^7$ cells/mL or more, $9 \times 10^7$ cells/mL or more, $9.5 \times 10^7$ cells/mL or more, $1 \times 10^8$ cells/mL or more, $1.1 \times 10^8$ cells/mL or more, $1.2 \times 10^8$ cells/mL or more, $1.3 \times 10^8$ cells/mL or more, $1.4 \times 10^8$ cells/mL or more, $1.5 \times 10^8$ cells/mL or more, or $2 \times 10^8$ cells/mL or more.

In a case where the porous membrane according to the second example is used, the degree of suppression of the decrease in the permeation rate of the product produced by cells in the porous membrane 112 is increased under the conditions in which the cell density contained in the cell broth filtered through the porous membrane 112 is $8 \times 10^7$ cells/mL

or more.

In general, as the number of particles contained in a liquid to be filtered increases, the change in the performance of the filtration membrane is likely to proceed more quickly. This is because the amounts of particles that serve as foulants for the filtration membrane increases as the number of particles contained in the liquid increases. However, as a result of intensive studies, the present inventors have confirmed that in the porous membrane according to the second example, the filtration performance is excellent in a case where the cell density contained in the cell broth is high. While not being bound by theory, it is considered that this is because, in the porous membrane according to the second example, the clogging of the membrane pores due to the deposition of the removed substances on the membrane surface is less likely to occur, whereas an effect of hindering foulants from entering the inside of the membrane, which causes clogging in the inside of the membrane due to a large amount of cell particles present in the solution, is provided. Therefore, in the porous membrane according to the second example, which has a structure in which the clogging of the membrane pores due to the deposition of the removed substances on the membrane surface is less likely to occur, there is a tendency that the higher the cell density contained in the cell broth is, the less likely to proceed the clogging in the inside of the membrane. However, in a case where a certain upper limit is exceeded, such a tendency may be weakened. Therefore, the cell density is $2 \times 10^9$ cells/mL or less. The cell density may be $1.5 \times 10^9$ cells/mL or less, $1.4 \times 10^9$ cells/mL or less, $1.3 \times 10^9$ cells/mL or less, $1.2 \times 10^9$ cells/mL or less, $1.1 \times 10^9$ cells/mL or less, $1 \times 10^9$ cells/mL or less, $9 \times 10^8$ cells/mL or less, $8 \times 10^8$ cells/mL or less, $7 \times 10^8$ cells/mL or less, $6 \times 10^8$ cells/mL or less, $5 \times 10^8$ cells/mL or less, $4 \times 10^8$ cells/mL or less, or $3 \times 10^8$ cells/mL or less.

In the second example of the porous membrane, the cell density contained in the cell broth that is filtered through the porous membrane 112 is not particularly limited; however, it may be $8 \times 10^7$ cells/mL or more and $2 \times 10^9$ cells/mL or less. The cell density may be $8.5 \times 10^7$ cells/mL or more and $2 \times 10^9$ cells/mL or less, $9 \times 10^7$ cells/mL or more and $2 \times 10^9$ cells/mL or less, $9.5 \times 10^7$ cells/mL or more and $2 \times 10^9$ cells/mL or less, $1 \times 10^8$ cells/mL or more and $2 \times 10^9$ cells/mL or less, $1.1 \times 10^8$ cells/mL or more and $2 \times 10^9$ cells/mL or less, $1.2 \times 10^8$ cells/mL or more and $2 \times 10^9$ cells/mL or less, $1.3 \times 10^8$ cells/mL or more and $2 \times 10^9$ cells/mL or less, $1.4 \times 10^8$ cells/mL or more and $2 \times 10^9$ cells/mL or less, $1.5 \times 10^8$ cells/mL or more and $2 \times 10^9$ cells/mL or less, $2 \times 10^8$ cells/mL or more and $2 \times 10^9$ cells/mL or less, $8 \times 10^7$ cells/mL or more and $1.5 \times 10^9$ cells/mL or less, $8.5 \times 10^7$ cells/mL or more and $1.5 \times 10^9$ cells/mL or less, $9 \times 10^7$ cells/mL or more and $1.5 \times 10^9$ cells/mL or less, $9.5 \times 10^7$ cells/mL or more and $1.5 \times 10^9$ cells/mL or less, $1 \times 10^8$ cells/mL or more and $1.5 \times 10^9$ cells/mL or less, $1.1 \times 10^8$ cells/mL or more and $1.5 \times 10^9$ cells/mL or less, $1.2 \times 10^8$ cells/mL or more and $1.5 \times 10^9$ cells/mL or less, $1.3 \times 10^8$ cells/mL or more and $1.5 \times 10^9$ cells/mL or less, $1.4 \times 10^8$ cells/mL or more and $1.5 \times 10^9$ cells/mL or less, $1.5 \times 10^8$ cells/mL or more and $1.5 \times 10^9$ cells/mL or less, $2 \times 10^8$ cells/mL or more and $1.5 \times 10^9$ cells/mL or less, $8 \times 10^7$ cells/mL or more and $1 \times 10^9$ cells/mL or less, $8 \times 10^7$ cells/mL or more and $5 \times 10^8$ cells/mL or less, $8 \times 10^7$ cells/mL or more and $3 \times 10^8$ cells/mL or less, $9 \times 10^7$ cells/mL or more and $3 \times 10^8$ cells/mL or less, $1 \times 10^8$ cells/mL or more and $1 \times 10^9$ cells/mL or less, $1 \times 10^8$ cells/mL or more and $5 \times 10^8$ cells/mL or less, or $1 \times 10^8$ cells/mL or more and $3 \times 10^8$ cells/mL or less.

[Third example of porous membrane]

In the third example of the porous membrane, the opening ratio on the liquid contact surface of the porous membrane, the coefficient of variation of the pore diameter on the liquid contact surface, and the ratio of pore diameter/fiber diameter on the liquid contact surface are the same as those in the first example of the porous membrane.

In the third example of the porous membrane, the structural anisotropy of the liquid contact surface is 0.97 or less, 0.965 or less, 0.96 or less, or 0.955 or less. The structural anisotropy of the liquid contact surface may be 0.3 or more, 0.5 or more, 0.7 or more, 0.8 or more, or 0.9 or more. The structural anisotropy of the liquid contact surface may be 0.3 or more and 0.97 or less, 0.5 or more and 0.97 or less, 0.7 or more and 0.97 or less, 0.8 or more and 0.97 or less, 0.9 or more and 0.97 or less, 0.5 or more and 0.965 or less, 0.7 or more and 0.96 or less, or 0.8 or more and 0.955 or less. In the third example of the porous membrane, there is a tendency that the larger the anisotropy of the liquid contact surface is, the less likely to occur the clogging in the inside of the membrane due to the entering of the removed substances into the membrane.

In the third example of the porous membrane, the ratio of long pore diameter/short pore diameter on the liquid contact surface is, for example, 1.8 or more, 1.82 or more, 1.84 or more, 1.85 or more, 1.9 or more, or 1.95 or more. In addition, the ratio of long pore diameter/short pore diameter is, for example, 10 or less, 5 or less, 3 or less, or 2.5 or less. In addition, the ratio of long pore diameter/short pore diameter is, for example, 1.8 or more and 10 or less, 1.8 or more and 5 or less, 1.8 or more and 3 or less, 1.8 or more and 2.5 or less, 1.85 or more and 3 or less, 1.85 or more and 2.5 or less, 1.9 or more and 3 or less, or 1.9 or more and 2.5 or less. In the third example of the porous membrane, there is a tendency that the larger the anisotropy of the pore on the liquid contact surface is, the less likely to occur the clogging in the inside of the membrane due to the entering of the removed substances into the membrane.

In the third example of the porous membrane, the porous membrane may be a hollow fiber membrane. The porous structure of the hollow fiber membrane may have a homogeneous structure that is substantially homogeneous from a primary side toward a secondary side in the membrane thickness direction. The homogeneous structure that is substantially homogeneous refers to that the average pore diameter does not change significantly, and it refers to a structure of a membrane in which a ratio (maximum value/minimum value) of a maximum value to a minimum value is 10 or less, 5 or less, or 3 or less in a case where an average pore diameter of portions, which have the same distance from the membrane surface in a membrane thickness direction, has been calculated. The ratio of maximum value/minimum value of the average pore diameter may be 1 or more, 1.01 or more, or 1.05 or more. In addition, the ratio of maximum value/minimum value of the average pore diameter may be 1 or more and 10 or less, 1 or more and 5 or less, 1 or more and 3 or less, 1.01 or more and 10 or less, 1.01 or more and 5 or less, 1.01 or more and 3 or less, 1.05 or more and 10 or less, 1.05 or more and 5 or less, or 1.05 or more and 3 or less.

In the third example of the porous membrane, the average pore diameter of the primary side surface in the hollow fiber membrane having a homogeneous structure is, for example, 0.05 µm or more, 0.1 µm or more, 0.2 µm or more, 0.3 µm or more, 0.4 µm or more, 0.5 µm or more, or 0.6 µm or more. In addition, the average pore diameter is, for example, 10 µm or less, 5 µm or less, 1 µm or less, 0.95 µm or less, 0.9 µm or less, 0.85 µm or less, or 0.8 µm or less. In addition, the average pore diameter of the primary side surface is, for example, 0.05 µm or more and 10 µm or less, 0.1 µm or more and 5 µm or less, 0.2 µm or more and 1 µm or less, 0.3 µm or more and 0.95 µm or less, 0.4 µm or more and 0.9 µm or less, 0.5 µm or more and 0.85 µm or less, or 0.6 µm or more and 0.8 µm or less. In a case where the pore diameter of the primary side surface is 0.05 µm or more and 10 µm or less, there is a tendency that an effect of screen filtration, in which the removed substances are not allowed to enter the inside of the membrane while maintaining the strength of the hollow fiber membrane, is easily obtained.

In the third example of the porous membrane, the blocking pore diameter of the hollow fiber membrane having a homogeneous structure is, for example, 0.05 µm or more, 0.1 µm or more, 0.2 µm or more, 0.3 µm or more, 0.4 µm or more, or 0.5 µm or more. In addition, the blocking pore diameter of the hollow fiber membrane having a homogeneous structure is, for example, 10 µm or less, 5 µm or less, 1 µm or less, 0.9 µm or less, 0.8 µm or less, or 0.7 µm or less. In addition, the blocking pore diameter of the hollow fiber membrane is, for example, 0.05 µm or more and 10 µm or less, 0.05 µm or more and 5 µm or less, 0.05 µm or more and 1 µm or less, 0.1 µm or more and 0.9 µm or less, 0.2 µm or more and 0.8 µm or less, 0.3 µm or more and 0.7 µm or less, 0.4 µm or more and 0.7 µm or less, or 0.5 µm or more and 0.7 µm or less. In a case where the blocking pore diameter is 0.05 µm or more, there is a tendency that the permeation resistance can be suppressed, the pressure required for filtration can be suppressed, and the membrane surface clogging due to the destruction and deformation of the microorganism particles, the decrease in the filtration efficiency, and the like can be suppressed. In addition, in a case where the blocking pore diameter is 10 µm or less, there is a tendency that sufficient fractionation properties are obtained.

In the third example of the porous membrane, the inner diameter of the hollow portion of the hollow fiber membrane is, for example, 1,000 µm or more or 1,050 µm or more. In addition, the inner diameter of the hollow portion of the hollow fiber membrane is, for example, 3,000 µm or less, 2,500 µm or less, 2,000 µm or less, 1,900 µm or less, 1,800 µm or less, 1,700 µm or less, 1,600 µm or less, or 1,500 µm or less. In addition, the inner diameter of the hollow portion of the hollow fiber membrane is, for example, 1,000 µm or more and 3,000 µm or less, 1,000 µm or more and 2,500 µm or less, 1,000 µm or more and 2,000 µm or less, 1,100 µm or more and 1,900 µm or less, or 1,200 µm or more and 1,800 µm or less. In a case where the inner diameter of the hollow portion is 1,000 µm or more, the entrance of the hollow portion tends to be less likely to be clogged by the cells. In a case where the inner diameter of the hollow portion is 3,000 µm or less, the number of hollow fiber membranes constituting the filtration module is increased, the effective membrane area per filtration module is increased, and thus the filtration performance tends to be excellent.

In the third example of the porous membrane, the membrane thickness of the hollow fiber membrane is, for example, 100 µm or more, 200 µm or more, 300 µm or more, or 400 µm or more. In addition, the membrane thickness of the hollow fiber membrane is, for example, 1,400 µm or less, 1,300 µm or less, 1,200 µm or less, 1,100 µm or less, 1,000 µm or less, 900 µm or less, 800 µm or less, 700 µm or less, 600 µm or less, or 500 µm or less. In addition, the membrane thickness of the hollow fiber membrane is, for example, 100 µm or more and 1,400 µm or less, 200 µm or more and 1,200 µm or less, or 300 µm or more and 1,000 µm or less. In a case where the membrane thickness is 100 µm or more, the strength of the hollow fiber membrane tends to be easily maintained. Further, it tends to be easy to maintain a suitable filtration speed. In addition, in a case where the membrane thickness is 1,400 µm or less, the number of hollow fiber membranes constituting the filtration module is increased, the effective cross-sectional area per filtration module is increased, and thus the filtration performance tends to be excellent.

In the third example of the porous membrane, the hollow fiber membrane consists of, for example, a synthetic polymer membrane. The synthetic polymer is, for example, hydrophobic. Examples of the synthetic polymer include polyvinylidene fluoride; however, examples thereof are not limited thereto.

The present inventors have found that a plurality of structures of liquid contact surfaces of the third example of these porous membranes affects the filtration performance. In a case where the liquid contact surface of the porous membrane that is used for filtration has any of the structural feature quantities in the suitable range, the effect can be sufficiently exhibited even in a case where each of the plurality of structures of liquid contact surfaces is used alone. In addition, it is obvious that a synergistic effect is exhibited in a case where a plurality of structures of liquid contact surfaces is combined. For example, it is considered that the clogging of the membrane pores due to the deposition of the removed substances on the membrane surface proceeds at an accelerating rate with the deposits as a scaffold, in a case where the clogging once starts to proceed. Therefore, it is effective to use a porous membrane that has a liquid contact surface having a plurality of structural feature quantities in an appropriate range.

[Shear stress in case where porous membrane is third example]

In a case where the porous membrane is the third example, a shear stress due to a flow of the cell broth on the liquid contact surface of the porous membrane 112 is set to 4.0 N/m$^2$ or more. The shear stress may be 4.1 N/m$^2$ or more, 4.2 N/m$^2$ or more, 4.3 N/m$^2$ or more, 4.4 N/m$^2$ or more, or 4.5 N/m$^2$ or more. In addition, for example, the shear stress may be 50 N/m$^2$ or less, 30 N/m$^2$ or less, 20 N/m$^2$ or less, 15 N/m$^2$ or less, or 10 N/m$^2$ or less. In addition, for example, the shear stress may be 4.0 N/m$^2$ or more and 50 N/m$^2$ or less, 4.0 N/m$^2$ or more and 30 N/m$^2$ or less, 4.0 N/m$^2$ or more and 20 N/m$^2$ or less, 4.0 N/m$^2$ or more and 15 N/m$^2$ or less, 4.0 N/m$^2$ or more and 10 N/m$^2$ or less, 4.1 N/m$^2$ or more and 50 N/m$^2$ or less, 4.2 N/m$^2$ or more and 30 N/m$^2$ or less, 4.3 N/m$^2$ or more and 20 N/m$^2$ or less, 4.4 N/m$^2$ or more and 15 N/m$^2$ or less, 4.5 N/m$^2$ or more and 50 N/m$^2$ or less, 4.5 N/m$^2$ or more and 30 N/m$^2$ or less, 4.5 N/m$^2$ or more and 20 N/m$^2$ or less, 4.5 N/m$^2$ or more and 15 N/m$^2$ or less, or 4.5 N/m$^2$ or more and 10 N/m$^2$ or less. In a case where the porous membrane according to the third example is used, it is possible to suppress a decrease in the permeation rate of the product produced by cells in the porous membrane 112 by setting a shear stress due to the cell broth on the liquid contact surface of the porous membrane 112 to 4.0 N/m$^2$ or more.

In a case where the porous membrane according to the third example is used with reference to Figure 1, the shear stress based on a viscosity of the cell broth may be measured and a flow speed of the cell broth to be filtered through the porous membrane 112, in a direction parallel to a membrane surface, may be controlled such that a measured shear stress is 4.0 N/m$^2$ or more. In a case where the porous membrane according to the third example is used, the controller 301 may control the pump 123 to control the linear velocity of the cell broth flowing through the porous membrane 112 in order to control, in a direction parallel to a membrane surface, a flow speed of the cell broth to be filtered through the porous membrane 112 such that the shear stress is 4.0 N/m$^2$ or more. As described above, the shear stress SS, the viscosity VC of the cell broth, and the shear rate SV have a relationship of Expression (3) described above. For example, the viscosity VC of the cell broth can be acquired in advance. It is noted that the measurement may be carried out with a viscometer provided in the flow channel 113. The shear rate SV can be calculated from the linear velocity LV, and the linear velocity LV can be acquired, for example, from the output of the pump 123. It is noted that the linear velocity LV may be measured with a flowmeter provided in the flow channel 113. The shear stress SS of the cell broth can be measured based on the known viscosity VC of the cell broth and the acquired linear velocity LV or shear rate SV of the cell broth.

For example, the controller 301 is connected to a pump 123. In a case where the porous membrane according to the third example is used, the controller 301 may control the pump 123 to control the linear velocity of the cell broth flowing through the porous membrane 112 in order to control, in a direction parallel to a membrane surface, a flow speed of the cell broth to be filtered through the porous membrane 112 such that the shear stress is 4.0 N/m$^2$ or more.

In the embodiment in which the porous membrane according to the third example is used, the shear stress in the porous membrane 112 is set to 4.0 N/m$^2$ or more. However, the occurrence of a period in which the shear stress in the porous membrane 112 is 4.0 N/m$^2$ or less is not excluded. For example, due to a delay in the feedback control of the pump, a time in which the shear stress in the porous membrane 112 is 4.0 N/m$^2$ or less may occur. In a case where the porous membrane according to the third example is used, the shear stress in the porous membrane 112 may be set to 4.0 N/m$^2$ or more in a period of at least 50% of the entire period of culturing the cells.

[Examples]

According to the method for producing a product by cells according to the present embodiment, it is possible to suppress a decrease in the permeation rate of the product produced by cells in the porous membrane by setting a shear stress suitable for the structure of the porous membrane.

(Example 1)

A frozen Chinese hamster ovary (CHO) cell line producing a monoclonal antibody (ATCC CRL-12445), which had been obtained by selecting cells adapted to and suspended in a serum-free culture medium, was thawed. Then, the cells were put into a 125 mL Erlenmeyer flask in which 10 mL of a serum-free culture medium having the composition shown in Table 1 had been dispensed in advance, and the cells and a culture medium were mixed in the Erlenmeyer flask. The number of cells was measured with a live and dead cell autoanalyzer (Vi-CELL XR, Beckman Coulter, Inc.), and the cells were diluted with a culture medium so that the cell density was $3.5 \times 10^5$ cells/mL. Thereafter, the cells were shaken and cultured in an incubator at 37°C and in an atmosphere of 5% $CO_2$ for 4 days.

[Table 1]

| Raw material of culture medium composition | Maker | Amount contained in 1 L of culture medium |
|---|---|---|
| EX-CELL® ACF CHO Medium | Sigma-Aldrich | 10.8 g |
| GIBCO™ CD Opti CHO™ AGT™ | Thermo Fisher Scientific | 9.7 g |
| GIBCO™ L-Glutamine 200 mM | Thermo Fisher Scientific | 40 mL |
| GIBCO™ MEM Amino Acids [-]L-Glutamine | Thermo Fisher Scientific | 10 mL |
| MEM Vitamin Solution | FUJIFILM Wako Pure Chemical Corporation | 5 mL |
| GIBCO™ HT Supplement | Thermo Fisher Scientific | 5 mL |
| Insulin, Human, recombinant | FUJIFILM Wako Pure Chemical Corporation | 4 mg |
| Kanamycin Sulfate | FUJIFILM Wako Pure Chemical Corporation | 0.05 g |
| Amphotericin B 250 μg/mL | Thermo Fisher Scientific | 1 mL |
| Methotrexate for Biochemistry *used only in cell passage | FUJIFILM Wako Pure Chemical Corporation | 0.2 μmol |

On the 4th day after thawing the cells, 50 mL of a cell broth containing cells at a density of $3.5 \times 10^5$ cells/mL was put into two new 125 mL Erlenmeyer flasks, and the cells were shaken and cultured in an incubator at 37°C and in an atmosphere of 5% $CO_2$ for 3 days. After three days, 120 to 130 mL of a cell broth containing cells at a density of $3.5 \times 10^5$ cells/mL was put into two new 250 mL Erlenmeyer flasks, and the cells were shaken and cultured in an incubator at 37°C and in an atmosphere of 5% $CO_2$ for 3 days. After three days, 500 mL of a cell broth containing cells at a density of $3.5 \times 10^5$ cells/mL was put into a 1 L three new Erlenmeyer flasks, and the cells were shaken and cultured in an incubator at 37°C and in an atmosphere of 5% $CO_2$ for 3 days.

6 L of a cell broth containing cells at a density of $5.5 \times 10^5$ cells/mL was put aseptically into a culture vessel having a volume of 12 L, which had been sterilized by autoclaving in advance. The cells were cultured with stirring for 3 days while blowing oxygen so that the DO did not fall below 70% at 37°C in an atmosphere of 5% $CO_2$. After three days, a serum-free culture medium having the composition shown in Table 2 was added to the culture vessel, where this addition was carried out at 0.28 mL/min for 3 days consecutively.

[Table 2]

| Raw material of culture medium composition to be added | Maker | Amount contained in 1 L of culture medium |
|---|---|---|
| GIBCO™ CHO CD EfficientFeed™ A chemically Defined Feed Supplement | Thermo Fisher Scientific | 450 mL |
| GIBCO™ CHO CD EfficientFeed™ B chemically Defined Feed Supplement | Thermo Fisher Scientific | 450 mL |
| GIBCO™ L-Glutamine 200 mM | Thermo Fisher Scientific | 100 mL |

Next, a cell broth containing cells at a density of $1.5 \times 10^7$ cells/mL was aseptically extracted from the culture vessel, and 600 mL thereof was transferred into a spinner flask as a culture vessel which had been sterilized by autoclaving in advance. A mini-module of a porous hollow fiber membrane, in which the effective length was adjusted such that the membrane area of the liquid contact portion was 3 cm$^2$, was prepared. A porous hollow fiber membrane (manufactured by Asahi Kasei Medical Co., Ltd., BioOptimal (registered trademark) MF-SL), which has a blocking pore diameter of 0.4 μm, consists of polysulfone (PSf), and has an inclined structure, was used. As a result of measuring the pore diameter, the inner diameter, the outer diameter, and the membrane thickness of the hollow fiber membrane of BioOptimal MF-SL on the primary side, the average value of the pore diameters on the primary side surface was 19.671 $\mu$m (3 × standard deviation ($\sigma$) = 4.714), the average value of the coefficient of variation of the pore diameters was 0.433 (3$\sigma$ = 0.107), the inner diameter was 1.4 mm, the outer diameter was 2.3 mm, and the membrane thickness was 0.45 **mm.** In addition, the average value of the opening ratio of the primary side surface of the hollow fiber membrane of BioOptimal MF-SL was 42.1% (3$\sigma$ = 1.3), the average value of the ratio of pore diameter/fiber diameter of the primary side surface of the hollow fiber membrane was 0.707 (3$\sigma$ = 0.126), the average value of the ratio of long pore diameter/short pore diameter of the primary side surface of the hollow fiber membrane was 1.558 (3$\sigma$ = 0.068), the ratio of maximum value/minimum value of the average pore diameter of the hollow fiber membrane in the membrane thickness direction was 44.3, and the average value of the structural anisotropy calculated from the power spectrum obtained by subjecting an image of the primary side surface of the hollow fiber membrane to two-dimensional Fourier transform was 1.002 (3$\sigma$ = 0.029).

The spinner flask and a first opening of the hollow portion, which is the primary side of the porous hollow fiber membrane, were connected to each other by a first flow channel, and a second opening of the hollow portion of the porous hollow fiber membrane and the spinner flask were connected to each other by a second flow channel. In addition, the secondary side of the porous hollow fiber membrane and the spinner flask were connected to each other by a third flow channel, and a cell broth filtered through the porous hollow fiber membrane was returned to the spinner flask.

The atmospheric temperature of the spinner flask and the porous hollow fiber membrane was set to 4°C, and the cell broth was stirred in the spinner flask. The cell broth was fed from the spinner flask to the porous hollow fiber membrane through the first flow channel by a magnetic levitation centrifugal pump (Pura Lev i30SU, manufactured by Levitronix), and tangential flow filtration was carried out. A cell broth that had not been filtered through the porous hollow fiber membrane and had passed through the hollow portion of the porous hollow fiber membrane was returned to the spinner flask through the second flow channel. The cell broth filtered through the porous hollow fiber membrane was returned to the spinner flask through the third flow channel. The first and third flow channels had a structure that made it possible to sample the cell broth inside the flow channels.

A single-use pressure gauge (manufactured by PendoTECH, PREPS-N-000 or PREPS-N-012) was installed on the primary side and the secondary side of the porous hollow fiber membrane, and the transmembrane pressure (TMP) was measured over time. The viscosity of the cell broth was measured with an EMS viscometer (EMS-1000S), and the flow rate of the cell broth fed from the spinner flask to the porous hollow fiber membrane was set such that the shear stress on the inner surface of the porous hollow fiber membrane was 1.15 N/m$^2$. The filtration flow rate in the porous hollow fiber membrane was set to be constant (1 LMH) by a pump.

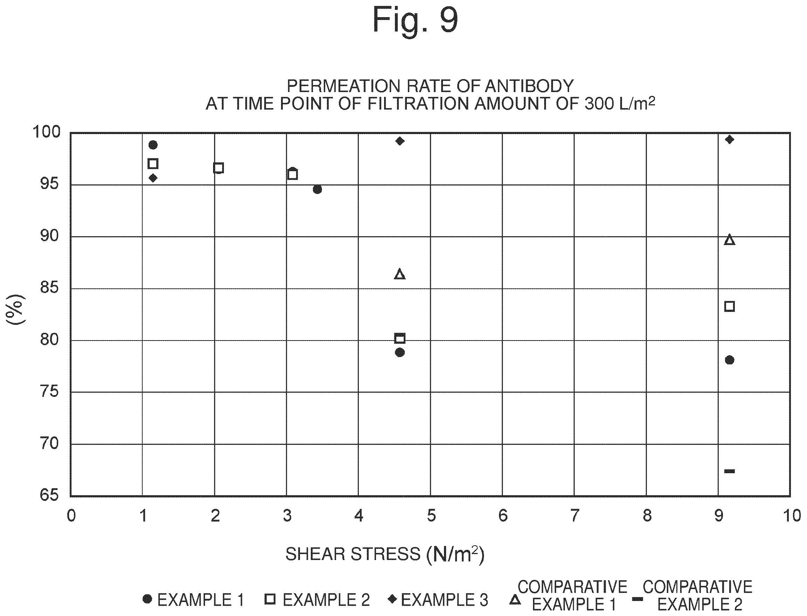

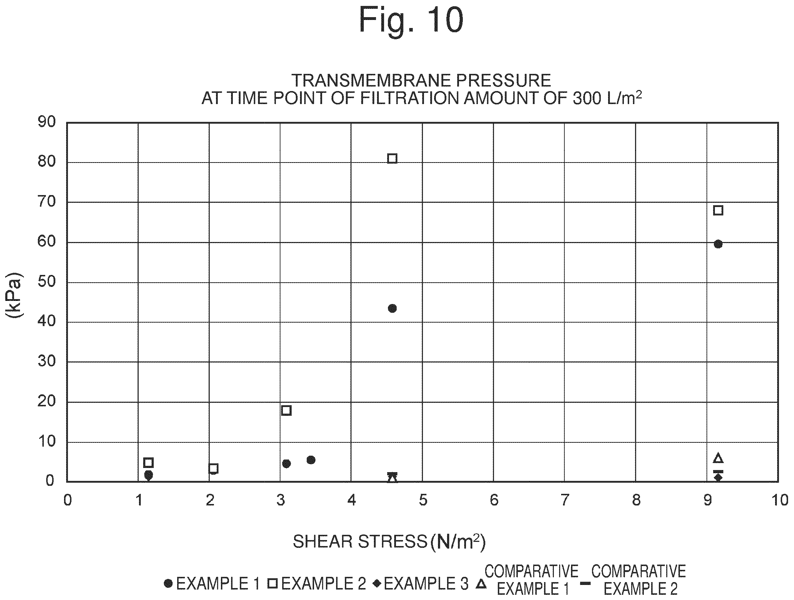

As shown in Figure 9 and Figure 10, at a time point at which the cell broth was filtered at 300 L/m$^2$ through the BioOptimal MF-SL, the permeation rate of the monoclonal antibody was 98.8%, and the transmembrane pressure was 1.8 kPa in the BioOptimal MF-SL.

Next, a cell broth was filtered through BioOptimal MF-SL by the same method, except that the shear stress was set to 2.06 N/m$^2$. As shown in Figure 9 and Figure 10, at a time point at which the shear stress was set to 2.06 N/m$^2$, and the cell broth was filtered at 300 L/m$^2$ through the BioOptimal MF-SL, the permeation rate of the monoclonal antibody was 96.5%, and the transmembrane pressure was 2.8 kPa in the BioOptimal MF-SL.

Next, a cell broth was filtered through BioOptimal MF-SL by the same method, except that the shear stress was set to 3.09 N/m$^2$. As shown in Figure 9 and Figure 10, at a time point at which the shear stress was set to 3.09 N/m$^2$, and the cell broth was filtered at 300 L/m$^2$ through the BioOptimal MF-SL, the permeation rate of the monoclonal antibody was 96.3%, and the transmembrane pressure was 4.5 kPa in the BioOptimal MF-SL.

Next, a cell broth was filtered through BioOptimal MF-SL by the same method, except that the shear stress was set to 3.435 N/m$^2$. As shown in Figure 9 and Figure 10, at a time point at which the shear stress was set to 3.435 N/m$^2$, and the cell broth was filtered at 300 L/m$^2$ through the BioOptimal MF-SL, the permeation rate of the monoclonal antibody was 94.5%, and the transmembrane pressure was 5.4 kPa in the BioOptimal MF-SL.

Next, a cell broth was filtered through BioOptimal MF-SL by the same method, except that the shear stress was set to 4.58 N/m$^2$. As shown in Figure 9 and Figure 10, at a time point at which the shear stress was set to 4.58 N/m$^2$, and the cell broth was filtered at 300 L/m$^2$ through the BioOptimal MF-SL, the permeation rate of the monoclonal antibody was decreased to 78.8%, and the transmembrane pressure was increased to 43.4 kPa in the BioOptimal MF-SL.

Next, a cell broth was filtered through BioOptimal MF-SL by the same method, except that the shear stress was set to 9.16 N/m$^2$. As shown in Figure 9 and Figure 10, at a time point at which the shear stress was set to 9.16 N/m$^2$, and the cell broth was filtered at 300 L/m$^2$ through the BioOptimal MF-SL, the permeation rate of the monoclonal antibody was 78.1%, and the transmembrane pressure was 59.6 kPa in the BioOptimal MF-SL.

As a result, it was shown that in a case where BioOptimal MF-SL is used as the porous hollow fiber membrane, the permeation rate of the product by cells is maintained at a high level, and the transmembrane pressure is also suppressed to be low in a case where the shear stress is set to 4.0 N/m$^2$ or less.

It is noted that in Example 1 carried out in an atmosphere of 4°C, the activity of cells present in the system was suppressed, and thus the total amount of the product by cells was kept constant. On the other hand, under the conditions in which cells present in the system produced products over time, the products accumulate in the system over time when the products were not permeated through the porous membrane. Therefore, in a case where the membrane permeation of the product by cells is inhibited, the value of the denominator in the calculation of the permeation rate increases, and thus the permeation rate of the product by cells becomes smaller as compared with in a case where the atmospheric temperature is 4°C. Therefore, the difference in the permeation rate between before and after the membrane permeation of the product by cells is inhibited becomes larger. As a result, the difference in permeation rate between smaller and larger value than the shear stress of 4.0 N/m$^2$ shown in Example 1 is further increased as the atmospheric temperature is higher. The same applies to other examples carried out in an atmosphere of 4°C.

(Example 2)

A porous hollow fiber membrane (manufactured by Asahi Kasei Corporation, MICROZA (registered trademark) UMP), which has a blocking pore diameter of 0.2 $\mu$m, consists of polyvinylidene fluoride (PVDF), and has a homogeneous structure, was prepared. As a result of measuring the pore diameter, the inner diameter, the outer diameter, and the membrane thickness of the hollow fiber membrane of the MICROZA UMP on the primary side, the average value of the pore diameters on the primary side surface was 0.364 $\mu$m (3$\sigma$ = 0.054), the average value of the coefficient of variation of the pore diameters was 0.335 (3$\sigma$ = 0.033), the inner diameter was 1.4 mm, the outer diameter was 2.2 mm, and the membrane thickness was 0.4 **mm.** In addition, the average value of the opening ratio of the primary side surface of the hollow fiber membrane of the MICROZA UMP was 49.8% (3$\sigma$ = 7.7), the average value of the ratio of pore diameter/fiber diameter of the primary side surface of the hollow fiber membrane was 1.080 (3$\sigma$ = 0.242), the average value of the ratio of long pore diameter/short pore diameter of the primary side surface of the hollow fiber membrane was 1.625 (3$\sigma$ = 0.071), the ratio of maximum value/minimum value of the average pore diameter of the hollow fiber membrane in the membrane thickness direction was 1.5, and the structural anisotropy calculated from the power spectrum obtained by subjecting an image of the primary side surface of the hollow fiber membrane to two-dimensional Fourier transform was 1.005 (3$\sigma$ = 0.017).

A cell broth was filtered through a porous hollow fiber membrane by the same method as in Example 1, except that MICROZA UMP was used as the porous hollow fiber membrane. As shown in Figure 9 and Figure 10, at a time point at which the shear stress was set to be 1.15 N/m$^2$, and then the cell broth was filtered at 300 L/m$^2$ through the MICROZA UMP, the permeation rate of the monoclonal antibody was 97.0%, and the transmembrane pressure was 4.7 kPa in the MICROZA UMP.

At a time point at which the shear stress was set to be 2.06 N/m$^2$, and then the cell broth was filtered at 300 L/m$^2$ through the MICROZA UMP, the permeation rate of the monoclonal antibody was 96.6%, and the transmembrane pressure was 3.3 kPa in the MICROZA UMP.

At a time point at which the shear stress was set to be 3.09 N/m$^2$, and then the cell broth was filtered at 300 L/m$^2$ through the MICROZA UMP, the permeation rate of the monoclonal antibody in the MICROZA UMP was 96.0%, and the transmembrane pressure was 17.9 kPa, and at a time point at which the cell broth was filtered at 340 L/m$^2$, the permeation rate of the monoclonal antibody was 90.2%, and the transmembrane pressure was 42.3 kPa in the MICROZA UMP.

At a time point at which the shear stress was set to be 4.58 N/m$^2$, and then the cell broth was filtered at 300 L/m$^2$ through the MICROZA UMP, the permeation rate of the monoclonal antibody was decreased to 80.2%, and the transmembrane pressure was increased to 81.0 kPa in the MICROZA UMP.

At a time point at which the shear stress was set to be 9.16 N/m$^2$, and then the cell broth was filtered at 300 L/m$^2$ through the MICROZA UMP, the permeation rate of the monoclonal antibody was 83.3%, and the transmembrane pressure was 68.0 kPa in the MICROZA UMP.

As a result, it was shown that in a case where MICROZA UMP is used as the porous hollow fiber membrane, the permeation rate of the product by cells is maintained at a high level, and the transmembrane pressure is also suppressed to be low in a case where the shear stress is set to 4.0 N/m$^2$ or less.

(Example 3)

A porous hollow fiber membrane (manufactured by Asahi Kasei Corporation, MICROZA (registered trademark) UJP), which has a blocking pore diameter of 0.65 $\mu$m, consists of polyvinylidene fluoride (PVDF), and has a homogeneous structure, was prepared. As a result of measuring the pore diameter, the inner diameter, the outer diameter, and the membrane thickness of the hollow fiber membrane of the MICROZA UJP on the primary side, the average value of the pore diameters on the primary side surface was 0.797 $\mu$m (3$\sigma$ = 0.197), the average value of the coefficient of variation of the pore diameters was 0.467 (3$\sigma$ = 0.133), the inner diameter was 1.1 mm, the outer diameter was 2 mm, and the membrane thickness was 0.45 **mm.** In addition, the average value of the opening ratio of the primary side surface of the hollow fiber membrane of the MICROZA UJP was 45.2% (3$\sigma$ = 6.3), the average value of the ratio of pore diameter/fiber diameter of the primary side surface of the hollow fiber membrane was 0.901 (3$\sigma$ = 0.130), the average value of the ratio of long pore diameter/short pore diameter of the primary side surface of the hollow fiber membrane was 1.957 (3$\sigma$ = 0.118), the ratio of maximum value/minimum value of the average pore diameter of the hollow fiber membrane in the membrane thickness direction was 2.0, and the average value of the structural anisotropy calculated from the power spectrum obtained by subjecting an image of the primary side surface of the hollow fiber membrane to two-dimensional Fourier transform was 0.954 ($3\sigma$ = 0.023).

A cell broth was filtered through a porous hollow fiber membrane by the same method as in Example 1, except that MICROZA UJP was used as the porous hollow fiber membrane. As shown in Figure 9 and Figure 10, at a time point at which the shear stress was set to be 1.15 N/m$^2$, and then the cell broth was filtered at 300 L/m$^2$ through the MICROZA UJP, the permeation rate of the monoclonal antibody was 95.7%, and the transmembrane pressure was 1.2 kPa in the MICROZA UJP.

At a time point at which the shear stress was set to be 4.58 N/m$^2$, and then the cell broth was filtered at 300 L/m$^2$ through the MICROZA UJP, the permeation rate of the monoclonal antibody was increased to 99.2%, and the transmembrane pressure was 1.3 kPa and did not change substantially in the MICROZA UJP.

At a time point at which the shear stress was set to be 9.16 N/m$^2$, and then the cell broth was filtered at 300 L/m$^2$ through the MICROZA UJP, the permeation rate of the monoclonal antibody was 99.4%, and the transmembrane pressure was 1.0 kPa in the MICROZA UJP.

As a result, it was shown that in a case where MICROZA UJP is used as the porous hollow fiber membrane, the permeation rate of the product by cells is maintained at a high level, and the transmembrane pressure is also suppressed to be low in a case where the shear stress is set to 4.0 N/m$^2$ or more.

(Comparative Example 1)

A porous hollow fiber membrane (manufactured by Repligen Corporation, used by being taken out from F2: RF02PES), which has a blocking pore diameter of 0.2 $\mu$m, consists of polyether sulfone (PES), and has a symmetric inclined structure, was prepared. The symmetric inclined structure is a structure in which the average pore diameter of the porous structure of the hollow fiber membrane increases from the primary side toward the secondary side in the membrane thickness direction and then decreases again to the same size as the primary side. As a result of measuring the pore diameter, the inner diameter, the outer diameter, and the membrane thickness of the hollow fiber membrane manufactured by Repligen Corporation on the primary side, the average value of the pore diameters on the primary side surface was 1.525 $\mu$m ($3\sigma$ = 0.052), the average value of the coefficient of variation of the pore diameters was 0.526 ($3\sigma$ = 0.071), the inner diameter was 1 mm, the outer diameter was 1.3 mm, and the membrane thickness was 0.15 mm. In addition, the average value of the opening ratio of the primary side surface of the hollow fiber membrane manufactured by Repligen Corporation was 61.7% ($3\sigma$ = 3.7), the average value of the ratio of pore diameter/fiber diameter of the primary side surface of the hollow fiber membrane was 1.455 ($3\sigma$ = 0.145), the average value of the ratio of long pore diameter/short pore diameter of the primary side surface of the hollow fiber membrane was 1.713 ($3\sigma$ = 0.063), the ratio of maximum value/minimum value of the average pore diameter of the hollow fiber membrane in the membrane thickness direction was 13.3, and the average value of the structural anisotropy calculated from the power spectrum obtained by subjecting an image of the primary side surface of the hollow fiber membrane to two-dimensional Fourier transform was 1.021 ($3\sigma$ = 0.012).

A cell broth was filtered through a porous hollow fiber membrane by the same method as in Example 1, except that a hollow fiber membrane manufactured by Repligen Corporation was used as the porous hollow fiber membrane. As shown in Figure 9 and Figure 10, at a time point at which the shear stress was set to be 4.58 N/m$^2$, and then the cell broth was filtered at 300 L/m$^2$ through a hollow fiber membrane manufactured by Repligen Corporation, the permeation rate of the monoclonal antibody was 86.4%, and the transmembrane pressure was 1.0 kPa in the hollow fiber membrane manufactured by Repligen Corporation.

At a time point at which the shear stress was set to be 9.16 N/m$^2$, and then the cell broth was filtered at 300 L/m$^2$ through a hollow fiber membrane manufactured by Repligen Corporation, the permeation rate of the monoclonal antibody was 89.7%, and the transmembrane pressure was 5.9 kPa in the hollow fiber membrane manufactured by Repligen Corporation.

(Comparative Example 2)

A porous hollow fiber membrane (manufactured by Cytiva, used by being taken out from CFP-4-E-5A), which has a blocking pore diameter of 0.45 $\mu$m, consists of polysulfone (PSf), and has a symmetric inclined structure, was prepared. As a result of measuring the pore diameter, the inner diameter, the outer diameter, and the membrane thickness of the hollow fiber membrane manufactured by Cytiva on the primary side, the average value of the pore diameters on the primary side surface was 1.092 $\mu$m ($3\sigma$ = 0.483), the average value of the coefficient of variation of the pore diameters was 0.745 ($3\sigma$ = 0.098), the inner diameter was 1 mm, the outer diameter was 1.5 mm, and the membrane thickness was 0.25 mm. In addition, the average value of the opening ratio of the primary side surface of the hollow fiber membrane manufactured by Cytiva was 62.9% ($3\sigma$ = 4.2), the average value of the ratio of pore diameter/fiber diameter of the primary side surface of the hollow fiber membrane was 2.325 ($3\sigma$ = 0.768), the average value of the ratio of long pore diameter/short pore diameter of the primary side surface of the hollow fiber membrane was 1.714 ($3\sigma$ = 0.097), the ratio of maximum value/minimum value of the average pore diameter of the hollow fiber membrane in the membrane thickness direction was 11.1, and the average value of the structural anisotropy calculated from the power spectrum obtained by subjecting an image of the primary side surface of the hollow fiber membrane to two-dimensional Fourier transform was 0.990 ($3\sigma$ = 0.022).

A cell broth was filtered through a porous hollow fiber membrane by the same method as in Example 1, except that a hollow fiber manufactured by Cytiva was used as the porous hollow fiber membrane. As shown in Figure 9 and Figure 10, at a time point at which the shear stress was set to be 4.58 N/m$^2$, and then the cell broth was filtered at 300 L/m$^2$ through a hollow fiber manufactured by Cytiva, the permeation rate of the monoclonal antibody was 80.6%, and the transmembrane pressure was 2.0 kPa in the hollow fiber manufactured by Cytiva.

At a time point at which the shear stress was set to be 9.16 N/m$^2$, and then the cell broth was filtered at 300 L/m$^2$ through a hollow fiber manufactured by Cytiva, the permeation rate of the monoclonal antibody was 67.4%, and the transmembrane pressure was 2.5 kPa in the hollow fiber manufactured by Cytiva.

(Example 4)

A frozen Chinese hamster ovary (CHO) cell line producing a monoclonal antibody (ATCC CRL-12445), which had been obtained by selecting cells adapted to and suspended in a serum-free culture medium, was thawed. Then, the cells were put into a 125 mL Erlenmeyer flask in which 10 mL of a serum-free culture medium having the composition shown in Table 1 had been dispensed in advance, and the cells and a culture medium were mixed in the Erlenmeyer flask. The number of cells was checked with a live and dead cell autoanalyzer (Vi-CELL XR, Beckman Coulter, Inc.), and the cells were diluted with a culture medium so that the cell density was $3.5 \times 10^5$ cells/mL. Thereafter, the cells were shaken and cultured in an incubator at 37°C and in an atmosphere of 5% $CO_2$ for 4 days.

On the 4th day after thawing the cells, 50 mL of a cell broth containing cells at a density of $3.5 \times 10^5$ cells/mL was put into two new 125 mL Erlenmeyer flasks, and the cells were shaken and cultured in an incubator at 37°C and in an atmosphere of 5% $CO_2$ for 3 days. Thereafter, 120 to 130 mL of a cell broth containing cells at a density of $3.5 \times 10^5$ cells/mL was put into two new 250 mL Erlenmeyer flasks, and the cells were shaken and cultured in an incubator at 37°C and in an atmosphere of 5% $CO_2$ for 3 days. Thereafter, 500 mL of a cell broth containing cells at a density of $3.5 \times 10^5$ cells/mL was put into three new 1 L Erlenmeyer flasks, and the cells were shaken and cultured in an incubator at 37°C and in an atmosphere of 5% $CO_2$ for 3 days.

5 L of a cell broth containing cells at a density of $5.5 \times 10^5$ cells/mL was put aseptically into a culture vessel having a volume of 12 L, which had been sterilized by autoclaving in advance. The cells were cultured with stirring for 3 days while blowing oxygen so that the DO did not fall below 70% at 37°C in an atmosphere of 5% $CO_2$. Thereafter, continuous culture, which was accompanied by filtration with a porous hollow fiber membrane (manufactured by Asahi Kasei Medical Co., Ltd., BioOptimal MF-SL0190) and addition of a culture medium to the culture vessel, was started.

A fresh culture medium of the same amount as the amount of the cell broth extracted from the culture vessel by filtration was aseptically fed from a 50 L bag (manufactured by Thermo Fisher Scientific, Inc., Productainer Bioprocess Container (BPC), 50 L) to the culture vessel, and the amount of the cell broth in the culture vessel was controlled to be constant. The culture medium replacement rate of the cell broth was set to 1 vessel volumes per day (vvd$^{-1}$), and the culture medium replacement rate of the cell broth was increased by 0.5 vvd$^{-1}$ at a time point at which the glucose concentration was 1 g/L or less or the glutamine concentration was 1 mmol/L or less.

After 7 days from the start of the continuous culture, a cell broth containing cells at a density of $1 \times 10^8$ cells/mL was aseptically recovered, and 600 mL of the cell broth was transferred into a spinner flask as a culture vessel which had been sterilized by autoclaving in advance. A mini-module of a porous hollow fiber membrane (manufactured by Asahi Kasei Corporation, MICROZA (registered trademark) UMP), in which the effective length was adjusted such that the membrane area of the liquid contact portion was 3 cm$^2$, was prepared. The spinner flask and a first opening of the hollow portion, which is the primary side of the porous hollow fiber membrane, were connected to each other by a first flow channel, a second opening of the hollow portion of the porous hollow fiber membrane and the spinner flask were connected to each other by a second flow channel, and the secondary side of the porous hollow fiber membrane and the spinner flask were connected to each other by a third flow channel.

The atmospheric temperature of the spinner flask and the porous hollow fiber membrane was set to 4°C, and the cell broth was stirred in the spinner flask. The cell broth was fed from the spinner flask to the porous hollow fiber membrane through the first flow channel by a magnetic levitation centrifugal pump (Pura Lev i30SU, manufactured by Levitronix), and tangential flow filtration was carried out. A cell broth that had not been filtered through the porous hollow fiber membrane and had passed through the hollow portion of the porous hollow fiber membrane was returned to the spinner flask through the second flow channel. The cell broth filtered through the porous hollow fiber membrane was returned to the spinner flask through the third flow channel. The first and third flow channels had a structure that made it possible to sample the cell broth inside the flow channels.

A single-use pressure gauge (manufactured by PendoTECH, PREPS-N-000 or PREPS-N-012) was installed on the primary side and the secondary side of the porous hollow fiber membrane, and the transmembrane pressure (TMP) was measured over time. The flow rate of the cell broth fed from the spinner flask to the porous hollow fiber membrane was set such that the shear stress on the inner surface of the porous hollow fiber membrane was 1.69 N/m$^2$. The filtration flow rate in the porous hollow fiber membrane was set to be constant (1 LMH) by a pump.

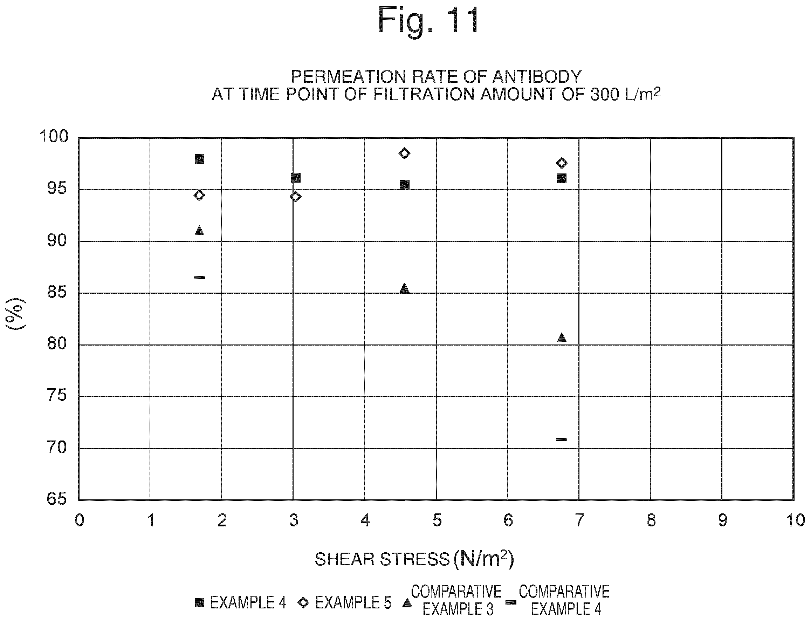

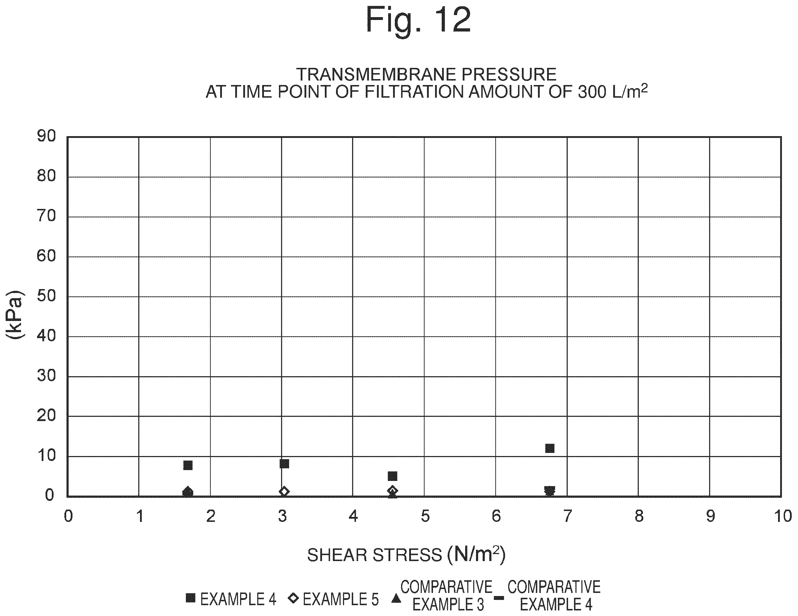

As shown in Figure 11 and Figure 12, at a time point at which the shear stress was set to be 1.69 N/m$^2$, and then the cell broth was filtered at 300 L/m$^2$ through the MICROZA UMP, the permeation rate of the monoclonal antibody was 98.0%, and the transmembrane pressure was 7.8 kPa in the MICROZA UMP.

Next, a cell broth was filtered with MICROZA UMP by the same method, except that the shear stress was set to be 3.04 N/m$^2$. As shown in Figure 11 and Figure 12, at a time point at which the shear stress was set to be 3.04 N/m$^2$, and then the cell broth was filtered at 300 L/m$^2$ through the MICROZA UMP, the permeation rate of the monoclonal antibody was 96.1%, and the transmembrane pressure was 8.2 kPa in the MICROZA UMP, and at a time point when the cell broth was filtered at 340L/m$^2$, the permeation rate of the monoclonal antibody was 94.2%, and the transmembrane pressure was 21.5 kPa in the MICROZA UMP.

Next, a cell broth was filtered with MICROZA UMP by the same method, except that the shear stress was set to be 4.56 N/m$^2$. As shown in Figure 11 and Figure 12, at a time point at which the shear stress was set to be 4.56 N/m$^2$, and then the cell broth was filtered at 300 L/m$^2$ through the MICROZA UMP, the permeation rate of the monoclonal antibody was decreased to 95.5%, and the transmembrane pressure was 5.1 kPa in the MICROZA UMP.

Next, a cell broth was filtered with MICROZA UMP by the same method, except that the shear stress was set to be 6.76 N/m$^2$. As shown in Figure 11 and Figure 12, at a time point at which the shear stress was set to be 6.76 N/m$^2$, and then the cell broth was filtered at 300 L/m$^2$ through the MICROZA UMP, the permeation rate of the monoclonal antibody was 96.1%, and the transmembrane pressure was 12.0 kPa in the MICROZA UMP.

As a result, it was shown that in a case where MICROZA UMP is used as the porous hollow fiber membrane, the permeation rate of the product by cells is maintained at a high level, and the transmembrane pressure is also suppressed to be low in a case where the shear stress is set to 4.0 N/m$^2$ or less.

(Example 5)

A cell broth was filtered through a porous hollow fiber membrane by the same method as in Example 4, except that MICROZA UJP was used as the porous hollow fiber membrane. As shown in Figure 11 and Figure 12, at a time point at which the shear stress was set to be 1.69 N/m$^2$, and then the cell broth was filtered at 300 L/m$^2$ through the MICROZA UJP, the permeation rate of the monoclonal antibody was 94.4%, and the transmembrane pressure was 1.2 kPa in the MICROZA UJP.

At a time point at which the shear stress was set to be 3.04 N/m$^2$, and then the cell broth was filtered at 300 L/m$^2$ through the MICROZA UJP, the permeation rate of the monoclonal antibody was 94.3%, and the transmembrane pressure was 1.2 kPa in the MICROZA UJP.

At a time point at which the shear stress was set to be 4.56 N/m$^2$, and then the cell broth was filtered at 300 L/m$^2$ through the MICROZA UJP, the permeation rate of the monoclonal antibody was increased to 98.5%, and the transmembrane pressure was 1.4 kPa in the MICROZA UJP.

At a time point at which the shear stress was set to be 6.76 N/m$^2$, and then the cell broth was filtered at 300 L/m$^2$ through the MICROZA UJP, the permeation rate of the monoclonal antibody was 97.5%, and the transmembrane pressure was 1.2 kPa in the MICROZA UJP.

As a result, it was shown that in a case where MICROZA UJP is used as the porous hollow fiber membrane, the permeation rate of the product by cells is maintained at a high level, and the transmembrane pressure is also suppressed to be low in a case where the shear stress is set to 4.0 N/m$^2$ or more.

(Comparative Example 3)

A cell broth was filtered through a porous hollow fiber membrane by the same method as in Example 4, except that the same hollow fiber membrane as the hollow fiber membrane in Comparative Example 1, manufactured by Repligen Corporation, was used as the porous hollow fiber membrane. As shown in Figure 11 and Figure 12, at a time point at which the shear stress was set to be 1.69 N/m$^2$, and then the cell broth was filtered at 300 L/m$^2$ through a hollow fiber membrane manufactured by Repligen Corporation, the permeation rate of the monoclonal antibody was 91.1%, and the transmembrane pressure was 1.2 kPa in the hollow fiber membrane manufactured by Repligen Corporation.

At a time point at which the shear stress was set to be 4.56 N/m$^2$, and then the cell broth was filtered at 300 L/m$^2$ through a hollow fiber membrane manufactured by Repligen Corporation, the permeation rate of the monoclonal antibody was decreased to 85.5%, and the transmembrane pressure was 0.7 kPa and did not change substantially in the hollow fiber membrane manufactured by Repligen Corporation.

At a time point at which the shear stress was set to be 6.76 N/m$^2$, and then the cell broth was filtered at 300 L/m$^2$ through a hollow fiber membrane manufactured by Repligen Corporation, the permeation rate of the monoclonal antibody was decreased to 80.7%, and the transmembrane pressure was 1.4 kPa in the hollow fiber membrane manufactured by Repligen Corporation.

(Comparative Example 4)

A cell broth was filtered through a porous hollow fiber membrane by the same method as in Example 4, except that the same hollow fiber membrane as the hollow fiber membrane in Comparative Example 2, manufactured by Cytiva, was used as the porous hollow fiber membrane. As shown in Figure 11 and Figure 12, at a time point at which the shear stress was set to be 1.69 N/m$^2$, and then the cell broth was filtered at 300 L/m$^2$ through a hollow fiber membrane manufactured by Cytiva, the permeation rate of the monoclonal antibody was 86.5%, and the transmembrane pressure was 0.7 kPa in the hollow fiber membrane manufactured by Cytiva.

At a time point at which the shear stress was set to be 6.76 N/m$^2$, and then the cell broth was filtered at 300 L/m$^2$ through a hollow fiber membrane manufactured by Cytiva, the permeation rate of the monoclonal antibody was decreased to 70.9%, and the transmembrane pressure was 2.1 kPa in the hollow fiber membrane manufactured by Cytiva.

(Example 6)

A frozen Chinese hamster ovary (CHO) cell line producing a monoclonal antibody (ATCC CRL-12445), which had been obtained by selecting cells adapted to and suspended in a serum-free culture medium, was thawed. Then, the cells were put into a 125 mL Erlenmeyer flask in which 10 mL of a serum-free culture medium having the composition shown in Table 1 had been dispensed in advance, and the cells and a culture medium were mixed in the Erlenmeyer flask. The number of cells was checked with a live and dead cell autoanalyzer (Vi-CELL XR, Beckman Coulter, Inc.), and the cells were diluted with a culture medium so that the cell density was $3.5 \times 10^5$ cells/mL. Thereafter, the cells were shaken and cultured in an incubator at 37°C and in an atmosphere of 5% CO$_2$ for 4 days.

On the 4th day after thawing the cells, 50 mL of a cell broth containing cells at a density of $3.5 \times 10^5$ cells/mL was put into two new 125 mL Erlenmeyer flasks, and the cells were shaken and cultured in an incubator at 37°C and in an atmosphere of 5% CO$_2$ for 3 days. Thereafter, 120 to 130 mL of a cell broth containing cells at a density of $3.5 \times 10^5$ cells/mL was put into two new 250 mL Erlenmeyer flasks, and the cells were shaken and cultured in an incubator at 37°C and in an atmosphere of 5% CO$_2$ for 3 days.

1.28 L of a cell broth containing cells at a density of $5.5 \times 10^5$ cells/mL was put aseptically into a culture vessel having a volume of 3 L, which had been sterilized by autoclaving in advance. The cells were cultured with stirring for 3 days while blowing oxygen so that the DO did not fall below 70% at 37°C in an atmosphere of 5% CO$_2$. Thereafter, a module of a porous hollow fiber membrane (manufactured by Asahi Kasei Corporation, Microza UMP), in which the number of sheets and the effective length were adjusted such that the membrane area of the liquid contact portion was 200 cm$^2$, was prepared. The culture vessel and a first opening of the hollow portion, which is the primary side of the porous hollow fiber membrane, were connected to each other by a first flow channel, and a second opening of the hollow portion of the porous hollow fiber membrane and the culture vessel were connected to each other by a second flow channel. The cell broth filtered through the porous hollow fiber membrane was not allowed to return to the culture vessel. In addition, the culture vessel was equipped with an outflow port for sampling an internal cell broth and a supply port for supplying a fresh culture medium.

The atmospheric temperature of the culture vessel and the porous hollow fiber membrane was set to 37°C, and the cell broth was stirred in the culture vessel. The cell broth was fed from the culture vessel to the porous hollow fiber membrane through the first flow channel by a magnetic levitation centrifugal pump (Pura Lev 200MU, manufactured by Levitronix), and tangential flow filtration was carried out. A cell broth that had not been filtered through the porous hollow fiber membrane and had passed through the hollow portion of the porous hollow fiber membrane was returned to the culture vessel through the second flow channel. In a case where the DO of the cell broth was less than 70%, oxygen was introduced into the culture vessel using a sparger. In addition, air containing carbon dioxide at a concentration of 5% was at all times introduced into the culture vessel.

A fresh culture medium of the same amount as the amount of the cell broth extracted from the culture vessel by filtration was aseptically fed from a 20 L bag (manufactured by Thermo Fisher Scientific, Inc., Productainer Bioprocess Container (BPC), 20 L) to the culture vessel, and the amount of the cell broth in the culture vessel was controlled to be constant. The culture medium replacement rate of the cell broth was set to 1 vessel volumes per day (vvd$^{-1}$), and the culture medium replacement rate of the cell broth was increased by 0.375 vvd$^{-1}$ at a time point at which the glucose concentration was 1 g/L or less or the glutamine concentration was 1 mmol/L or less.

A single-use pressure gauge (manufactured by PendoTECH, PREPS-N-038) was installed on the primary side and the secondary side of the porous hollow fiber membrane, and the transmembrane pressure (TMP) was measured over time. The flow rate of the cell broth fed from the culture vessel to the porous hollow fiber membrane was set such that the shear stress on the inner surface of the porous hollow fiber membrane was 2.10 N/m$^2$. The filtration flow rate in the porous hollow fiber membrane was increased stepwise to 1.0 LMH, 1.5 LMH, 2.0 LMH, 2.5 LMH, and 3.0 LMH by a pump, and the introduction amount of the fresh culture medium was allowed to be increased for adjusting the glucose concentration and the glutamine concentration in accordance with the increase in the number of cells. In addition, bleeding was carried out so that the cell density in the cell broth in the culture vessel was $7.5 \times 10^7$ cells/mL. After the start of bleeding, the actual cell density fluctuated in a range of $6.5 \times 10^7$ cells/mL or more and $9.3 \times 10^7$ cells/mL or less. Further, a liquid level sensor was disposed in the culture vessel, and the same amount of fresh culture medium as the bled cell broth was introduced into the culture vessel so that the liquid level was constant.

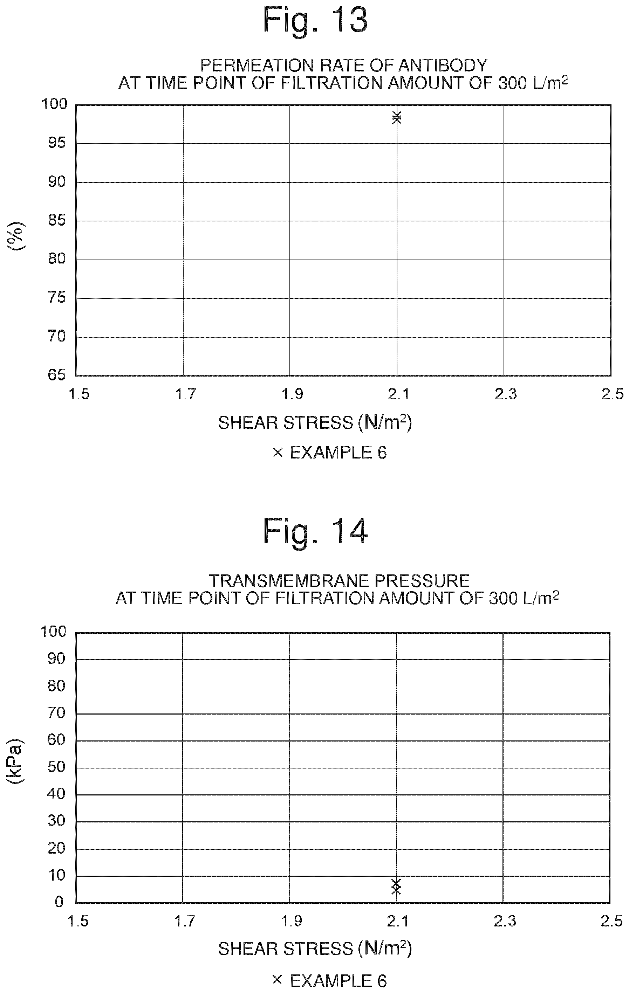

The cell broth before and after filtration was sampled once or twice a day, and the concentration of the antibody in the cell broth was measured. The same experiment was repeated twice, and the results were such that as shown in Figure 13 and Figure 14, at a time point at which the shear stress was set to be 2.10 N/m$^2$, and then the cell broth was filtered at 300 L/m$^2$ through the MICROZA UMP, the permeation rate of the monoclonal antibody was 98.7% and 98.1%, respectively, and the transmembrane pressure was 7.3 kPa and 4.8 kPa, respectively, in the MICROZA UMP.

(Analysis method)

The analysis methods used in Examples, Comparative Examples, and Reference Examples will be described below. The density and viability of cells contained in the cell broth were measured by a live and dead cell autoanalyzer (Vi-CELL XR, manufactured by Beckman Coulter, Inc.). The sample was diluted using PBS (-) (FUJIFILM Wako Pure Chemical Corporation), and 600 $\mu$L thereof was used for analysis. A method, in which "CHO" in Vi-CELL XR was used and the set values of a Minimum diameter ($\mu$m), a Cell Brightness (%), and a Viable cell spot Brightness (%) were changed as appropriate according to the actual situation, was used as an image analysis method.

The HPLC measurement of the antibody concentration was carried out by the following method.

(1) Detector: Ultraviolet absorption spectrophotometer (measurement wavelength: 280 nm)
(2) Column: POROS G 20 $\mu$m Column, 4.6 $\times$ 50 mm, 0.8 mL (Thermo Fisher Scientific, Inc.)
(3) Column temperature: room temperature
(4) Mobile phase

Mobile phase A: 7.098 g of disodium hydrogen phosphate (anhydrous) and 8.766 g of sodium chloride were dissolved in 800 mL of water, 1 mol/L hydrochloric acid was added thereto to adjust the pH to 7.0, and then water was added thereto to adjust the total volume to 1,000 mL.

Mobile phase B: 12 mL of 1 mol/L hydrochloric acid and 8.766 g of sodium chloride were dissolved in water, and the total volume was adjusted to 1,000 mL.

(5) Feeding of mobile phase

The proportion of the mobile phase A and the mobile phase B was changed as shown in the table below, and then feeding was carried out at a flow rate of 2 mL/min.

[Table 3]

| Time after specimen injection (minutes) | Mobile phase A (vol%) | Mobile phase B (vol%) |
| --- | --- | --- |
| 0 to 4 | 100 | 0 |
| 4 to 11 | 0 | 100 |
| 11 to 16 | 100 | 0 |

The cell broth was centrifuged at 300 $\times$ g for 2 minutes to 5 minutes, and then the supernatant was sampled. According to the above-described method, a series of nine stepwise diluted solutions of a commercially available human immunoglobulin G (blood donation Venoglobulin IH 5% for intravenous injection, manufactured by the Japan Blood Products Organization, 2.5 g/50 mL) and the supernatant of the cell broth are fed according to the above procedure. A standard curve was created using 9 peak areas of human immunoglobulin G, and then the antibody concentration in each solution was calculated from the standard curve and the peak area of the specimen.

The viscosity of the cell broth was measured with an EMS viscometer (EMS-1000S, manufactured by KYOTO ELECTRONICS MANUFACTURING Co., Ltd.). 300 $\mu$L of the sample was placed in a dedicated standard sample container together with a spherical probe of $\varphi$1.5 mm and then analyzed. The motor rotation speed was set to 400 rpm, and the measurement was carried out by repeating 10 times of measurements with a measurement time of 1 second and a holding time of 30 seconds. The average value from the 10 times of the measurements was used as the viscosity of the cell broth.

Reference Signs List

111: culture vessel
112: porous membrane
113, 114, 115, 116, 117, 118, 119: flow channel
123, 125, 126, 133, 134, 135: pump
201, 217, 218: container
212: stirring device
216: culture medium tank
301: controller

**Claims**

1. A method for producing a product by cells, the method comprising:

   filtering a cell broth through a porous membrane to obtain a product by the cells,
   wherein the porous membrane has a liquid contact surface having at least any one selected from the group consisting of an opening ratio of 57.5% or less, a pore diameter having a coefficient of variation of 0.5 or less, and a ratio of pore diameter/fiber diameter of 1.3 or less, and
   the liquid contact surface has at least any one selected from the group consisting of a structural anisotropy of 0.97 or more and 1.03 or less and a ratio of long pore diameter/short pore diameter of 1.8 or less, and
   a shear stress due to a flow of the cell broth on the liquid contact surface is set to 4.0 N/m$^2$ or less.

2. The method for producing a product by cells according to Claim 1,
   wherein the porous membrane has at least any one selected from the group consisting of an average pore diameter of 1 $\mu$m or more on the liquid contact surface and a ratio of maximum value/minimum value of an average pore diameter in a membrane thickness direction of 3 or more.

3. The method for producing a product by cells according to Claim 1,
   wherein the porous membrane has at least any one selected from the group consisting of an average pore diameter of 10 $\mu$m or less on the liquid contact surface and a ratio of maximum value/minimum value of an average pore diameter in a membrane thickness direction of 1 or more and 10 or less.

4. The method for producing a product by cells according to Claim 2,
   wherein the porous membrane is a hollow fiber membrane.

5. The method for producing a product by cells according to Claim 4,
   wherein the hollow fiber membrane has an inclined structure in which the average pore diameter decreases from a primary side toward a secondary side in the membrane thickness direction.

6. The method for producing a product by cells according to Claim 4,
   wherein a pore of the hollow fiber membrane has a blocking pore diameter of 0.05 $\mu$m or more and 20 $\mu$m or less.

7. The method for producing a product by cells according to Claim 4,
   wherein the hollow fiber membrane consists of a synthetic polymer membrane.

8. The method for producing a product by cells according to Claim 7,
   wherein the synthetic polymer is polysulfone.

9. The method for producing a product by cells according to Claim 4,

wherein the hollow fiber membrane has a coarse layer and a dense layer.

10. The method for producing a product by cells according to Claim 3,
wherein the porous membrane is a hollow fiber membrane.

11. The method for producing a product by cells according to Claim 10,
wherein the hollow fiber membrane has a homogeneous structure that is substantially homogeneous from a primary side toward a secondary side in a membrane thickness direction.

12. The method for producing a product by cells according to Claim 10,
wherein a pore of the hollow fiber membrane has a blocking pore diameter of 0.05 $\mu$m or more and 10 $\mu$m or less.

13. The method for producing a product by cells according to Claim 10,
wherein the hollow fiber membrane consists of a synthetic polymer membrane.

14. The method for producing a product by cells according to Claim 13,
wherein the synthetic polymer is polyvinylidene fluoride.

15. The method for producing a product by cells according to Claim 1, further comprising:
measuring the shear stress based on a viscosity of the cell broth, and controlling, in a direction parallel to a membrane surface, a flow speed of the cell broth to be filtered through the porous membrane such that the measured shear stress is 4.0 N/m$^2$ or less.

16. The method for producing a product by cells according to Claim 1,
wherein the cell broth that is not filtered through the hollow fiber membrane is returned to a culture vessel of the cells.

17. The method for producing a product by cells according to Claim 1,
wherein the product is an antibody.

18. The method for producing a product by cells according to Claim 1,
wherein the cells are subjected to perfusion culture.

19. The method for producing a product by cells according to Claim 3,
wherein a cell density contained in the cell broth is $8 \times 10^7$ cells/mL or more.

20. A method for producing a product by cells, the method comprising:

filtering a cell broth through a porous membrane to obtain a product by the cells,
wherein the porous membrane has a liquid contact surface having at least any one selected from the group consisting of an opening ratio of 57.5% or less, a pore diameter having a coefficient of variation of 0.5 or less, and a ratio of pore diameter/fiber diameter of 1.3 or less, and
the liquid contact surface has at least any one selected from the group consisting of a structural anisotropy of 0.97 or less and a ratio of long pore diameter/short pore diameter of 1.8 or more, and
a shear stress due to a flow of the cell broth on the liquid contact surface is set to 4.0 N/m$^2$ or more.

21. The method for producing a product by cells according to Claim 20,
wherein the porous membrane is a hollow fiber membrane.

22. The method for producing a product by cells according to Claim 21,
wherein the hollow fiber membrane has a homogeneous structure that is substantially homogeneous from a primary side toward a secondary side in a membrane thickness direction.

23. The method for producing a product by cells according to Claim 21,
wherein a pore of the hollow fiber membrane has a blocking pore diameter of 0.05 $\mu$m or more and 10 $\mu$m or less.

24. The method for producing a product by cells according to Claim 21,
wherein the hollow fiber membrane consists of a synthetic polymer membrane.

**25.** The method for producing a product by cells according to Claim 24,
wherein the synthetic polymer is polyvinylidene fluoride.

**26.** The method for producing a product by cells according to Claim 20, further comprising:
measuring the shear stress based on a viscosity of the cell broth, and controlling, in a direction parallel to a membrane surface, a flow speed of the cell broth to be filtered through the porous membrane such that the measured shear stress is 4.0 N/m$^2$ or more.

**27.** The method for producing a product by cells according to Claim 20,
wherein the cell broth that is not filtered through the hollow fiber membrane is returned to a culture vessel of the cells.

**28.** The method for producing a product by cells according to Claim 20,
wherein the product is an antibody.

**29.** The method for producing a product by cells according to Claim 20,
wherein the cells are subjected to perfusion culture.

**30.** A method for suppressing a decrease in a membrane permeability of a product produced by cells, the method comprising:

filtering a cell broth through a porous membrane to obtain a product by the cells,
wherein the decrease in the membrane permeability of the product by cells is suppressed by;
the porous membrane having a liquid contact surface having at least any one selected from the group consisting of an opening ratio of 57.5% or less, a pore diameter having a coefficient of variation of 0.5 or less, and a ratio of pore diameter/fiber diameter of 1.3 or less, and
the liquid contact surface having at least any one selected from the group consisting of a structural anisotropy of 0.97 or more and 1.03 or less and a ratio of long pore diameter/short pore diameter of 1.8 or less, and
a shear stress due to a flow of the cell broth on the liquid contact surface being set to 4.0 N/m$^2$ or less.

**31.** The method for suppressing a decrease in a membrane permeability of a product produced by cells according to Claim 30,
wherein the porous membrane has at least any one selected from the group consisting of an average pore diameter of 1 μm or more on the liquid contact surface and a ratio of maximum value/minimum value of an average pore diameter in a membrane thickness direction of 3 or more.

**32.** The method for suppressing a decrease in a membrane permeability of a product produced by cells according to Claim 30,
wherein the porous membrane has at least any one selected from the group consisting of an average pore diameter of 10 μm or less on the liquid contact surface and a ratio of maximum value/minimum value of an average pore diameter in a membrane thickness direction of 1 or more and 10 or less.

**33.** The method for suppressing a decrease in a membrane permeability of a product produced by cells according to Claim 31,
wherein the porous membrane is a hollow fiber membrane.

**34.** The method for suppressing a decrease in a membrane permeability of a product produced by cells according to Claim 33,
wherein the hollow fiber membrane has an inclined structure in which the average pore diameter decreases from a primary side toward a secondary side in the membrane thickness direction.

**35.** The method for suppressing a decrease in a membrane permeability of a product produced by cells according to Claim 33,
wherein a pore of the hollow fiber membrane has a blocking pore diameter of 0.05 μm or more and 20 μm or less.

**36.** The method for suppressing a decrease in a membrane permeability of a product produced by cells according to Claim 33,
wherein the hollow fiber membrane consists of a synthetic polymer membrane.

37. The method for suppressing a decrease in a membrane permeability of a product produced by cells according to Claim 36,
wherein the synthetic polymer is polysulfone.

38. The method for suppressing a decrease in a membrane permeability of a product produced by cells according to Claim 33,
wherein the hollow fiber membrane has a coarse layer and a dense layer.

39. The method for suppressing a decrease in a membrane permeability of a product produced by cells according to Claim 32,
wherein the porous membrane is a hollow fiber membrane.

40. The method for suppressing a decrease in a membrane permeability of a product produced by cells according to Claim 39,
wherein the hollow fiber membrane has a homogeneous structure that is substantially homogeneous from a primary side toward a secondary side in a membrane thickness direction.

41. The method for suppressing a decrease in a membrane permeability of a product produced by cells according to Claim 39,
wherein a pore of the hollow fiber membrane has a blocking pore diameter of 0.05 $\mu$m or more and 10 $\mu$m or less.

42. The method for suppressing a decrease in a membrane permeability of a product produced by cells according to Claim 39,
wherein the hollow fiber membrane consists of a synthetic polymer membrane.

43. The method for suppressing a decrease in a membrane permeability of a product produced by cells according to Claim 42,
wherein the synthetic polymer is polyvinylidene fluoride.

44. The method for suppressing a decrease in a membrane permeability of a product produced by cells according to Claim 30, further comprising:
measuring the shear stress based on a viscosity of the cell broth, and controlling, in a direction parallel to a membrane surface, a flow speed of the cell broth to be filtered through the porous membrane such that the measured shear stress is 4.0 N/m$^2$ or less.

45. The method for suppressing a decrease in a membrane permeability of a product produced by cells according to Claim 30,
wherein the cell broth that is not filtered through the hollow fiber membrane is returned to a culture vessel of the cells.

46. The method for suppressing a decrease in a membrane permeability of a product produced by cells according to Claim 30,
wherein the product is an antibody.

47. The method for suppressing a decrease in a membrane permeability of a product produced by cells according to Claim 30,
wherein the cells are subjected to perfusion culture.

48. The method for suppressing a decrease in a membrane permeability of a product produced by cells according to Claim 32,
wherein a cell density contained in the cell broth is $8 \times 10^7$ cells/mL or more.

49. A method for suppressing a decrease in a membrane permeability of a product produced by cells, the method comprising:

filtering a cell broth through a porous membrane to obtain a product by the cells,
wherein the decrease in the membrane permeability of the product by cells is suppressed by;
the porous membrane having a liquid contact surface having at least any one selected from the group consisting of an opening ratio of 57.5% or less, a pore diameter having a coefficient of variation of 0.5 or less, and a ratio of pore diameter/fiber diameter of 1.3 or less, and

the liquid contact surface having at least any one selected from the group consisting of a structural anisotropy of 0.97 or less and a ratio of long pore diameter/short pore diameter of 1.8 or more, and

a shear stress due to a flow of the cell broth on the liquid contact surface being set to 4.0 N/m² or more.

50. The method for suppressing a decrease in a membrane permeability of a product produced by cells according to Claim 49,

wherein the porous membrane is a hollow fiber membrane.

51. The method for suppressing a decrease in a membrane permeability of a product produced by cells according to Claim 50,

wherein the hollow fiber membrane has a homogeneous structure that is substantially homogeneous from a primary side toward a secondary side in a membrane thickness direction.

52. The method for suppressing a decrease in a membrane permeability of a product produced by cells according to Claim 50,

wherein a pore of the hollow fiber membrane has a blocking pore diameter of 0.05 $\mu$m or more and 10 $\mu$m or less.

53. The method for suppressing a decrease in a membrane permeability of a product produced by cells according to Claim 50,

wherein the hollow fiber membrane consists of a synthetic polymer membrane.

54. The method for suppressing a decrease in a membrane permeability of a product produced by cells according to Claim 53,

wherein the synthetic polymer is polyvinylidene fluoride.

55. The method for suppressing a decrease in a membrane permeability of a product produced by cells according to Claim 49, further comprising:

measuring the shear stress based on a viscosity of the cell broth, and controlling, in a direction parallel to a membrane surface, a flow speed of the cell broth to be filtered through the porous membrane such that the measured shear stress is 4.0 N/m² or more.

56. The method for suppressing a decrease in a membrane permeability of a product produced by cells according to Claim 49,

wherein the cell broth that is not filtered through the hollow fiber membrane is returned to a culture vessel of the cells.

57. The method for suppressing a decrease in a membrane permeability of a product produced by cells according to Claim 49,

wherein the product is an antibody.

58. The method for suppressing a decrease in a membrane permeability of a product produced by cells according to Claim 49,

wherein the cells are subjected to perfusion culture.

59. A system for producing a product by cells, the system comprising:

a culture vessel for culturing cells;

a porous membrane for filtering a cell broth to obtain a product by the cells;

a pump for feeding the cell broth from the culture vessel to the porous membrane; and

a controller configured to control the pump such that a shear stress due to a flow of the cell broth on a liquid contact surface of the porous membrane is set to 4.0 N/m² or less,

wherein the liquid contact surface has at least any one selected from the group consisting of an opening ratio of 57.5% or less, a pore diameter having a coefficient of variation of 0.5 or less, and a ratio of pore diameter/fiber diameter of 1.3 or less, and

the liquid contact surface has at least any one selected from the group consisting of a structural anisotropy of 0.97 or more and 1.03 or less and a ratio of long pore diameter/short pore diameter of 1.8 or less.

60. The system for producing a product by cells according to Claim 59,

wherein the porous membrane has at least any one selected from the group consisting of an average pore diameter of 1 μm or more on the liquid contact surface and a ratio of maximum value/minimum value of an average pore diameter in a membrane thickness direction of 3 or more.

61. The system for producing a product by cells according to Claim 59,
wherein the porous membrane has at least any one selected from the group consisting of an average pore diameter of 10 μm or less on the liquid contact surface and a ratio of maximum value/minimum value of an average pore diameter in a membrane thickness direction of 1 or more and 10 or less.

62. The system for producing a product by cells according to Claim 60,
wherein the porous membrane is a hollow fiber membrane.

63. The system for producing a product by cells according to Claim 62,
wherein the hollow fiber membrane has an inclined structure in which the average pore diameter decreases from a primary side toward a secondary side in the membrane thickness direction.

64. The system for producing a product by cells according to Claim 62,
wherein a pore of the hollow fiber membrane has a blocking pore diameter of 0.05 μm or more and 20 μm or less.

65. The system for producing a product by cells according to Claim 62,
wherein the hollow fiber membrane consists of a synthetic polymer membrane.

66. The system for producing a product by cells according to Claim 65,
wherein the synthetic polymer is polysulfone.

67. The system for producing a product by cells according to Claim 62,
wherein the hollow fiber membrane has a coarse layer and a dense layer.

68. The system for producing a product by cells according to Claim 61,
wherein the porous membrane is a hollow fiber membrane.

69. The system for producing a product by cells according to Claim 68,
wherein the hollow fiber membrane has a homogeneous structure that is substantially homogeneous from a primary side toward a secondary side in a membrane thickness direction.

70. The system for producing a product by cells according to Claim 68,
wherein a pore of the hollow fiber membrane has a blocking pore diameter of 0.05 μm or more and 10 μm or less.

71. The system for producing a product by cells according to Claim 68,
wherein the hollow fiber membrane consists of a synthetic polymer membrane.

72. The system for producing a product by cells according to Claim 71,
wherein the synthetic polymer is polyvinylidene fluoride.

73. The system for producing a product by cells according to Claim 59,
wherein the controller measures the shear stress based on a viscosity of the cell broth, and controls, in a direction parallel to a membrane surface, a flow speed of the cell broth to be filtered through the porous membrane such that the measured shear stress is 4.0 N/m$^2$ or less.

74. The system for producing a product by cells according to Claim 59,
wherein the controller receives an input of data of a viscosity of the cell broth.

75. The system for producing a product by cells according to Claim 59, further comprising:
a flow channel for returning the cell broth that is not filtered through the hollow fiber membrane, to the culture vessel.

76. The system for producing a product by cells according to Claim 59,
wherein the product is an antibody.

**77.** The system for producing a product by cells according to Claim 59,
wherein the cells are subjected to perfusion culture.

**78.** The system for producing a product by cells according to Claim 61,
wherein a cell density contained in the cell broth is $8 \times 10^7$ cells/mL or more.

**79.** A system for producing a product by cells, the system comprising:

a culture vessel for culturing cells;
a porous membrane for filtering a cell broth of the cells to obtain a product by the cells;
a pump for feeding the cell broth from the culture vessel to the porous membrane; and
a controller configured to control the pump such that a shear stress due to a flow of the cell broth on a liquid contact surface of the porous membrane is set to 4.0 N/m$^2$ or more,
wherein the liquid contact surface has at least any one selected from the group consisting of an opening ratio of 57.5% or less, a pore diameter having a coefficient of variation of 0.5 or less, and a ratio of pore diameter/fiber diameter of 1.3 or less, and
the liquid contact surface has at least any one selected from the group consisting of a structural anisotropy of 0.97 or less and a ratio of long pore diameter/short pore diameter of 1.8 or more.

**80.** The system for producing a product by cells according to Claim 79,
wherein the porous membrane is a hollow fiber membrane.

**81.** The system for producing a product by cells according to Claim 80,
wherein the hollow fiber membrane has a homogeneous structure that is substantially homogeneous from a primary side toward a secondary side in a membrane thickness direction.

**82.** The system for producing a product by cells according to Claim 80,
wherein a pore of the hollow fiber membrane has a blocking pore diameter of 0.05 μm or more and 10 μm or less.

**83.** The system for producing a product by cells according to Claim 80,
wherein the hollow fiber membrane consists of a synthetic polymer membrane.

**84.** The system for producing a product by cells according to Claim 83,
wherein the synthetic polymer is polyvinylidene fluoride.

**85.** The system for producing a product by cells according to Claim 79,
wherein the controller measures the shear stress based on a viscosity of the cell broth, and controls, in a direction parallel to a membrane surface, a flow speed of the cell broth to be filtered through the porous membrane such that the measured shear stress is 4.0 N/m$^2$ or more.

**86.** The system for producing a product by cells according to Claim 79,
wherein the controller receives an input of data of a viscosity of the cell broth.

**87.** The system for producing a product by cells according to Claim 79, further comprising:
a flow channel for returning the cell broth that is not filtered through the hollow fiber membrane, to the culture vessel.

**88.** The system for producing a product by cells according to Claim 79,
wherein the product is an antibody.

**89.** The system for producing a product by cells according to Claim 79,
wherein the cells are subjected to perfusion culture.

**90.** A method for producing a product by cells, the method comprising:

filtering a cell broth through a porous membrane to obtain a product by the cells,
wherein the porous membrane has a liquid contact surface having at least any one selected from the group consisting of an opening ratio of 57.5% or less, a pore diameter having a coefficient of variation of 0.5 or less, and a ratio of pore diameter/fiber diameter of 1.3 or less, and

the liquid contact surface has at least any one selected from the group consisting of a structural anisotropy of 0.97 or more and 1.03 or less and a ratio of long pore diameter/short pore diameter of 1.8 or less, and the porous membrane has at least any one selected from the group consisting of an average pore diameter of 10 $\mu$m or less on the liquid contact surface and a ratio of maximum value/minimum value of an average pore diameter in a membrane thickness direction of 1 or more and 10 or less, and a cell density contained in the cell broth to be filtered is $8 \times 10^7$ cells/mL or more.

91. The method for producing a product by cells according to Claim 90, wherein the porous membrane is a hollow fiber membrane.

92. The method for producing a product by cells according to Claim 90, wherein the cell broth that is not filtered through the hollow fiber membrane is returned to a culture vessel of the cells.

93. The method for producing a product by cells according to Claim 90, wherein the product is an antibody.

94. The method for producing a product by cells according to Claim 90, wherein the cells are subjected to perfusion culture.

95. A method for suppressing a decrease in a membrane permeability of a product produced by cells, the method comprising:

filtering a cell broth through a porous membrane to obtain a product by the cells, wherein the decrease in the membrane permeability of the product by cells is suppressed by:

the porous membrane having a liquid contact surface having at least any one selected from the group consisting of an opening ratio of 57.5% or less, a pore diameter having a coefficient of variation of 0.5 or less, and a ratio of pore diameter/fiber diameter of 1.3 or less, and the liquid contact surface having at least any one selected from the group consisting of a structural anisotropy of 0.97 or more and 1.03 or less and a ratio of long pore diameter/short pore diameter of 1.8 or less, and the porous membrane having at least any one selected from the group consisting of an average pore diameter of 10 $\mu$m or less on the liquid contact surface and a ratio of maximum value/minimum value of an average pore diameter in a membrane thickness direction of 1 or more and 10 or less, and a cell density contained in the cell broth to be filtered being $8 \times 10^7$ cells/mL or more.

96. The method for suppressing a decrease in a membrane permeability of a product produced by cells according to Claim 95, wherein the porous membrane is a hollow fiber membrane.

97. The method for suppressing a decrease in a membrane permeability of a product produced by cells according to Claim 95, wherein the cell broth that is not filtered through the hollow fiber membrane is returned to a culture vessel of the cells.

98. The method for suppressing a decrease in a membrane permeability of a product produced by cells according to Claim 95, wherein the product is an antibody.

99. The method for suppressing a decrease in a membrane permeability of a product produced by cells according to Claim 95, wherein the cells are subjected to perfusion culture.

100.
A system for producing a product by cells, the system comprising:

a culture vessel for culturing cells;
a porous membrane for filtering a cell broth to obtain a product by the cells; and
a pump for feeding the cell broth from the culture vessel to the porous membrane,
wherein a liquid contact surface of the porous membrane has at least any one selected from the group consisting of an opening ratio of 57.5% or less, a pore diameter having a coefficient of variation of 0.5 or less, and a ratio of pore diameter/fiber diameter of 1.3 or less, and

the liquid contact surface has at least any one selected from the group consisting of a structural anisotropy of 0.97 or more and 1.03 or less and a ratio of long pore diameter/short pore diameter of 1.8 or less, and

the porous membrane has at least any one selected from the group consisting of an average pore diameter of 10 μm or less on the liquid contact surface and a ratio of maximum value/minimum value of an average pore diameter in a membrane thickness direction of 1 or more and 10 or less, and

a cell density contained in the cell broth to be filtered is $8 \times 10^7$ cells/mL or more.

**101.**
The system for producing a product by cells according to Claim 100,
wherein the porous membrane is a hollow fiber membrane.

**102.**
The system for producing a product by cells according to Claim 100,
wherein the cell broth that is not filtered through the hollow fiber membrane is returned to a culture vessel of the cells.

**103.**
The system for producing a product by cells according to Claim 100,
wherein the product is an antibody.

**104.**
The system for producing a product by cells according to Claim 100,
wherein the cells are subjected to perfusion culture.

# Fig. 1

EP 4 621 062 A1

# Fig. 2

SMALL ▬▬▭ LARGE

EP 4 621 062 A1

# Fig. 3

501

500

# Fig. 4

501

502

# Fig. 5

505

CIRCUMSCRIBED
RECTANGLE

Fig. 6

# Fig. 7

DISTRIBUTION
OF FLOW SPEED

DISTRIBUTION
OF SHEAR STRESS

# Fig. 8

CAKE LAYER
ON MEMBRANE
SURFACE

CLOGGING
IN INSIDE OF
MEMBRANE

INNER
SIDE

HOLLOW
FIBER

OUTER
SIDE

CONCENTRATION
POLARIZATION
LAYER
(CONCENTRATION
BOUNDARY
LAYER)

: CELL

# Fig. 9

PERMEATION RATE OF ANTIBODY
AT TIME POINT OF FILTRATION AMOUNT OF 300 L/m²

● EXAMPLE 1　□ EXAMPLE 2　◆ EXAMPLE 3　△ COMPARATIVE EXAMPLE 1　▬ COMPARATIVE EXAMPLE 2

EP 4 621 062 A1

EP 4 621 062 A1

## Fig. 10

TRANSMEMBRANE PRESSURE
AT TIME POINT OF FILTRATION AMOUNT OF 300 L/m²

(kPa)

SHEAR STRESS (N/m²)

● EXAMPLE 1  □ EXAMPLE 2  ◆ EXAMPLE 3  △ COMPARATIVE EXAMPLE 1  ─ COMPARATIVE EXAMPLE 2

## Fig. 11

PERMEATION RATE OF ANTIBODY
AT TIME POINT OF FILTRATION AMOUNT OF 300 L/m²

SHEAR STRESS (N/m²)

■ EXAMPLE 4    ◇ EXAMPLE 5    ▲ COMPARATIVE EXAMPLE 3    ▬ COMPARATIVE EXAMPLE 4

# Fig. 12

TRANSMEMBRANE PRESSURE
AT TIME POINT OF FILTRATION AMOUNT OF 300 L/m²

■ EXAMPLE 4   ◇ EXAMPLE 5   ▲ COMPARATIVE   ▬ COMPARATIVE
                                EXAMPLE 3         EXAMPLE 4

EP 4 621 062 A1

# Fig. 13

PERMEATION RATE OF ANTIBODY
AT TIME POINT OF FILTRATION AMOUNT OF 300 L/m$^2$

× EXAMPLE 6

# Fig. 14

TRANSMEMBRANE PRESSURE
AT TIME POINT OF FILTRATION AMOUNT OF 300 L/m$^2$

× EXAMPLE 6

## INTERNATIONAL SEARCH REPORT

| International application No. |
| --- |
| **PCT/JP2023/040994** |

**A. CLASSIFICATION OF SUBJECT MATTER**

*C12P 21/08*(2006.01)i; *B01D 61/14*(2006.01)i; *B01D 63/02*(2006.01)i; *B01D 69/00*(2006.01)i; *B01D 69/02*(2006.01)i; *B01D 71/06*(2006.01)i; *B01D 71/34*(2006.01)i; *B01D 71/68*(2006.01)i; *C12M 1/00*(2006.01)i; *C12M 1/12*(2006.01)i

FI: C12P21/08; B01D61/14 500; B01D63/02; B01D69/00; B01D69/02; B01D71/06; B01D71/34; B01D71/68; C12M1/00 A; C12M1/12

According to International Patent Classification (IPC) or to both national classification and IPC

**B. FIELDS SEARCHED**

Minimum documentation searched (classification system followed by classification symbols)

C12P21/08; B01D61/14; B01D63/02; B01D69/00; B01D69/02; B01D71/06; B01D71/34; B01D71/68; C12M1/00; C12M1/12

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2020/209267 A1 (ASAHI KASEI MEDICAL CO., LTD.) 15 October 2020 (2020-10-15) paragraph [0066] | 1-19, 30-48, 59-78, 90-104 |
| X | PINTO, N. D. S., Wide-surface pore microfiltration membrane drastically improves sieving decay in TFF-based perfusion cell culture and streamline chromatography integration for continuous bioprocessing, Biotechnology and Bioengineering, 2020, vol. 117, pp. 3336-3344, DOI:10.1002/bit.27504 2.1 Perfusion cell culture | 1-104 |

☐ Further documents are listed in the continuation of Box C.   ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **14 December 2023** | **26 December 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2023/040994**

| Patent document cited in search report | Publication date (day/month/year) | Patent family member(s) | Publication date (day/month/year) |
|---|---|---|---|
| WO 2020/209267 A1 | 15 October 2020 | US 2022/0212167 A1 paragraph [0207] | |

Form PCT/ISA/210 (patent family annex) (January 2015)

**REFERENCES CITED IN THE DESCRIPTION**

**Patent documents cited in the description**

- JP 2018076291 A
- JP 2009045019 A
- JP 2021048776 A
- JP 5696479 B

- JP 2022516516 W
- WO 2001053213 A
- WO 2010035793 A

**Non-patent literature cited in the description**

- **GOSTICK et al.** PoreSpy: A Python Toolkit for Quantitative Analysis of Porous Media Images. *Journal of Open Source Software*, 2019, vol. 4 (37), 1296